# EUROPEAN PATENT APPLICATION

(11) **EP 1 666 468 A1**
(43) Date of publication of application: **07.06.2006**
(21) Application number: 04773059.3
(22) Date of filing: 08.09.2004
(51) Int. Cl.: C07D 221/04, C07D 239/16, C07D 239/70, C07D 491/048, C07D 487/04, C07D 513/04, A61K 31/435, A61K 31/505, A61K 31/517, A61K 31/519, A61K 31/53

(54) **CRF ANTAGONISTS AND HETEROBICYCLIC COMPOUNDS**

(30) Priority: 09.09.2003 JP 2003316662; 19.04.2004 JP 2004122409
(71) Applicant: ONO PHARMACEUTICAL CO., LTD., Osaka-shi, Osaka 541-8526 (JP)
(72) Inventor: NAKAI, Hisao, c/o Ono Pharmaceutical Co., Ltd., Mishima-gun, Osaka 618-8585 (JP); SAITO, Tetsuji, c/o Ono Pharmaceutical Co., Ltd., Mishima-gun, Osaka 618-8585 (JP); OBITSU, Tetsuo, c/o Ono Pharmaceutical Co., Ltd., Mishima-gun, Osaka 618-8585 (JP); MINAMOTO, Chiaki, c/o Ono Pharmaceutical Co., Ltd., Mishima-gun, Osaka 618-8585 (JP); YOSHIDA, Mayuki, Kawaguchi-shi, Saitama 332-0011 (JP); KISHI, Akihiro, c/o Ono Pharmaceutical Co., Ltd., Mishima-gun, Osaka 618-8585 (JP); KATSUMATA, Seishi, Ono Pharmaceutical Co., Ltd., Sakakai-gun, Fukui 913-0032 (JP); KATAYAMA, Hideo, c/o Ono Pharmaceutical Co., Ltd., Mishima-gun, Osaka 618-8585 (JP)
(74) Representative: Henkel, Feiler & Hänzel
(86) International application number: PCT/JP2004/013386
(87) International publication number: WO 2005/026126

(57) **Abstract**

CRF antagonists comprising as an active ingredient, the compound of formula (I) 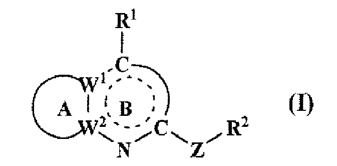 wherein A ring is 5-6 membered mono-cyclic ring which may be substituted; B ring is 5-7 membered unsaturated mono-heterocyclic ring which may be contained another 1-2 of hetero atom(s) and substituted by another substituents; W¹ and W² is carbon atom or nitrogen atom; Z is NR³, oxygen atom, sulfur which may be oxidized or CR⁴R⁵; R' is alkyl, alkenyl or alkynyl that may be substituted, amino which may be protected, hydroxyl which may be protected, S(O)ₙR⁶, COR⁷, or cyclic group which may be substituted; R² is unsaturated cyclic group which may be substituted.

## Description

### Technical Field

The present invention relates to a Corticotropin Releasing Factor antagonist, a novel bi-heterocyclic ring compound, a salt thereof, an N-oxide thereof, a solvate thereof or a prodrug thereof, and a pharmaceutical comprising them as an active ingredient. For more detail, the present invention relates to a Corticotropin Releasing Factor antagonist comprising a compound of formula (I): 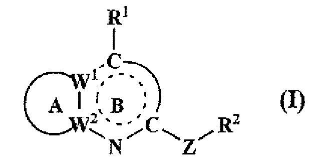 wherein all symbols are as hereinafter defined;
as an active ingredient and a novel bi-heterocyclic ring compound of formula (I-A): 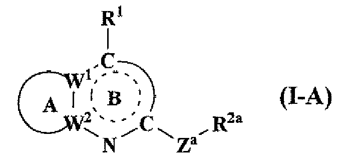 wherein all symbols are as hereinafter defined;
a salt thereof, an N-oxide thereof, a solvate thereof or a prodrug thereof, and a pharmaceutical comprising them as an active ingredient.

### Background Art

Corticotropin Releasing Factor (CRF) was a peptide comprising 41 amino acid residues and isolated from ovine hypothalamic in 1981. It was suggested that CRF was released from hypothalamic and controlled a secretion of adrenocorticotropic hormone (ACTH) from hypophysis *[Science,* 218, 377-379(1982)].

ACTH, which is released by a stimulation of CRF, stimulates a secretion of cortisol from adrenal cortex, and relates to a systemic action for reproduction, growth, gastrointestinal function, inflammation, immune system, nervous system *etc.* Consequently, CRF is believed to plays a role as a regulator of these functions. In view of these, a relationship of CRF and a central nervous system disease or a neuropsychiatric disorder has gotten a lot of attention.

On the other hand, the depression patients and the anxiety disorder patients increase, and the number also of depression patients with the slight illness increases recently. Moreover, an aged patient is commanding a majority in the depression patient. Under these circumstances, from the earliness of the appearance of the effect and respect of the side effect, psychiatric and neurologic disorders treatment used easily is requested more and more.

Currently, for the treatment of psychiatric and neurologic disorders, for example, tricyclic antidepressants, tetracyclic antidepressants, monoamine oxidase inhibitors, serotonin and noradrenaline reuptake inhibitors (SNRI), selective serotonin reuptake inhibitors (SSRI), *etc.* as antidepressant are used. However, the therapeutic gain is not enough; it will take a long time by the time the effect appears; drowsiness, a dryness of the mouth and constipation and difficulty feelings in micturition *etc.* are seen as a side effect. As an antianxiety agent, such as benzodiazepine anxiolytic, thienodiazepine anxiolytic, non-benzodiazepine anxiolytic *etc.* are used. However, the therapeutic gain is not also enough; decrease in mental movement function and decrease in concentration and attention power, drowsiness, stagger, dizziness, headache, amnesia, *etc.* are seen as a side effect.

It is expected to use a compound having an activity of CRF antagonist for the treatment of depression and anxiety disorders. For example, in pamphlet of WO 02/53565, a compound of formula (A): 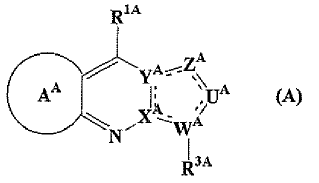 wherein X^{A} and Y^{A} each independently, is carbon or nitrogen and both are not nitrogens at the same time; W^{A} is carbon or nitrogen; U^{A} and Z^{A} each independently, is CR^{2A}, NR^{13A}, nitrogen, oxygen, sulfur, C=O or C=S;
---- is a single bond or a double bond; 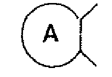 is C4-6 carbocyclic ring or 4-6 membered heterocyclic ring containing at least one of nitrogen, oxygen and sulfur and these rings are unsubstituted or substituted by 1-3 of substitutes selected from C1-4 alkyl, C1-4 alkoxy, a halogen atom and CF₃;
R^{1A} is (i) C1-8 alkyl which is unsubstituted or substituted by 1-5 of R^{14A}, (ii) C2-8 alkenyl which is unsubstituted or substituted by 1-5 of R^{14A}, (iii) C2-8 alkynyl which is unsubstituted or substituted by 1-5 of R^{14A}, (iv) NR^{4A}R^{5A}, (v) OR^{6A}, (vi) SH, (vii) S(O)nR^{7A}, (viii) COR^{6A}, (ix) COOR^{6A}, (x) CONR^{4A}R^{5A}, (xi) NR^{8A}COR^{6aA}, (xii) NR^{8A}COOR^{6A}, (xiii) NR^{8A}CONR^{4A}R^{5A}, (xiv) C3-15 mono- or bi-carbocyclic ring which is unsubstituted or substituted by 1-5 of R^{15A}, (xv) 3-15 membered mono- or bi-heterocyclic ring containing 1-4 of nitrogen(s), 1-2 of oxygen(s) and / or 1-2 of sulfur(s) which is unsubstituted or substituted by 1-5 of R^{15A}_{;}
R^{3A} is (i) C5-10 mono- or bi-carbocyclic ring substituted by 1-5 of R^{16A} or (ii) 5-10 membered mono- or bi-heterocyclic ring containing 1-4 of nitrogen(s), 1-2 of oxygen(s) and / or 1-2 of sulfur(s) substituted by 1-5 of R^{16A}; was described.

On the other hand, as bi-heterocyclic ring compound, for example, in pamphlet of WO 97/11946, a compound of formula (B): 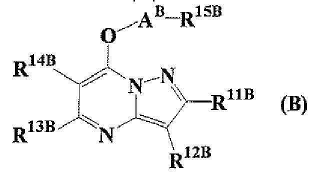 wherein R^{11B} is a hydrogen, lower alkyl, pyridyl, furyl, thienyl, phenyl which may be substituted by lower alkyl or phenylthio, N-lower alkyl pyrrolyl or pyrazinyl; R^{12B} is a hydrogen, a halogen atom, phenyl, phenyl which may be substituted by substituents selected by a halogen atom, phenylthio and trifluoromethyl and nitro, or phenyl substituted by lower alkoxy and phenylthio; R^{13B} is a hydrogen, lower alkyl which may be substituted by oxo, ethylenedioxy, lower alkanoyloxy, lower alkoxy, lower alkylthio, carboxyl, halogen or thienyl, lower alkenyl, cycloalkyl, phenyl, furyl or thienyl which may be substituted by 1-3 of lower alkyl, halogen and lower alkoxy; R^{14B} is a hydrogen, carboxyl, lower alkoxycarbonyl, nitro, a halogen atom or lower alkyl substituted by lower alkoxy carbonyl or alkali metal salt residue of carboxylic acid; or R^{13B} and R^{14B} were taken together, may be formed lower alkylene; R^{15B} is a hydrogen, alkali metal atom, lower alkyl, phenyl which may be substituted by 1-3 of lower alkyl and lower alkoxy, pyridyl, quinolyl or isoquinolyl which may substituted by lower alkyl or halogen; A^{B} is bond or lower alkylene;
was described as analgesic drug,
in pamphlet of WO 01/32632, a compound of formula (C): 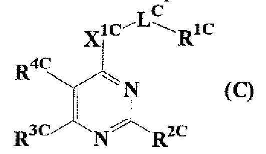 wherein X^{1C} is O or NH; L^{C} is bond or C1-6 alkylene chain optionally interrupted by O, S, SO, SO₂ or NH and optionally substituted on alkylene carbon by fluoro, hydroxyl, C1-4 alkoxy or oxo; R^{1C} is unsubstituted or substituted carbocyclic ring or heterocyclic ring; R^{2C} is a hydrogen, a halogen atom, carboxyl, cyano, SCH₂CH, or X^{2C}-R^{5C} in which X^{2C} is bond, O, S, SO, SO₂ or NH, R^{5C} is C1-8 alkyl, C3-10 cycloalkyl, halo(C1-6) alkyl, hydroxyl(C1-6)alkyl, dihydroxy(C1-6)alkyl, phenyl or phenyl(C1-4)alkyl in which phenyl is unsubstituted or substituted by one or two substituents selected independently from a halogen atom, C1-4 alkyl and C1-4 alkoxy, *etc.*; R^{3C} and R^{4C} each independently is C1-4 alkyl or together with the carbon atoms to which they are attached form unsubstituted or substituted carbocyclic ring or heterocyclic ring;
was described as mGluR1 antagonist.

### Disclosure of the Invention

An object of the present invention is to provide an agent which is easily handled and has potent prevention and/or treatment effects in the prevention and/or treatment of psychiatric and neurologic disorders, diseases of peripheral organs or the like.

The present inventors studied intensively in order to solve the above problems, and as a result, found that the object can be achieved by a bicyclic heterocyclic ring.

The present invention relates to the followings:
1. A CRF antagonist comprising, as an active ingredient, a compound represented by formula (I): 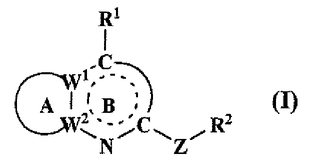 wherein ring A represents a 5- or 6-membered monocyclic ring which may be substituted with 1 to 3 substituents selected from a halogen atom, CF₃, OCF₃, hydroxyl, mercapto, carboxyl, (C1-6 alkoxy)carbonyl, carbamoyl, nitro, cyano, oxo, and C1-6 alkyl, C2-6 alkenyl, C2-6 alkynyl, C1-6 alkoxy or C1-6 alkylthio which each may be substituted with 1 to 3 substituents selected from a halogen atom, CF₃ and hydroxyl;
   ring B represents a 5- to 7-membered monocyclic unsaturated heterocyclic ring which may contain 1 or 2 hetero atoms selected from a nitrogen atom, an oxygen atom and/or a sulfur atom which may be oxidized, other than the nitrogen atom, W¹ and W² and which may be further substituted;
   W¹ and W² each independently, represents a carbon atom or a nitrogen atom;
   Z represents -NR³-, in which R³ represents a hydrogen atom, C1-6 alkyl, C2-6 alkenyl or C2-6 alkynyl which each may be substituted, -CO-(C1-6 alkyl which may be substituted), -SO₂-(C1-6 alkyl which may be substituted), an oxygen atom, a sulfur atom which may be oxidized, or -CR⁴R⁵-, in which R⁴ and R⁵ each independently represents a hydrogen atom, C1-6 alkyl, C2-6 alkenyl or C2-6 alkynyl which each may be substituted, or R⁴ and R⁵ may be taken together to represent (i) oxo, (ii) C2-5 alkylene in which one carbon atom may be substituted with one oxygen atom, nitrogen atom or sulfur atom which may be oxidized, wherein the C2-5 alkylene may be substituted with a substituent(s), or (iii) C1-6 alkylidene which may be substituted;
   R¹ represents:
   (i) C1-15 alkyl, C2-15 alkenyl or C2-15 alkynyl which each may be substituted,
   (ii) amino which may be protected,
   (iii) hydroxyl which may be protected,
   (iv) mercapto which may be protected,
   (v) -S(O)ₙR⁶, in which n represents 1 or 2, and R⁶ represents (a) C1-15 alkyl, C2-15 alkenyl or C2-15 alkynyl which each may be substituted or (b) a cyclic group which may be substituted,
   (vi) -COR⁷, in which R⁷ represents (a) a hydrogen atom, (b) C1-15 alkyl, C2-15 alkenyl or C2-15 alkynyl which each may be substituted, (c) hydroxyl which may be protected, (d) amino which may be protected, or (e) a cyclic group which may be substituted, or
   (vii) a cyclic group which may be substituted;
   R² represents an unsaturated cyclic group which may be substituted, in which the substituent may be taken together with R³ to form C2-5 alkylene which may be substituted,
   a salt thereof, an N-oxide thereof, a solvate thereof or a prodrug thereof
2. A compound represented by formula (I-A): 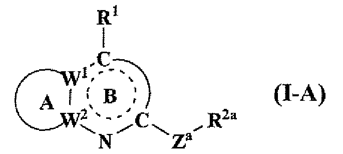 wherein 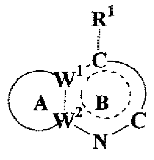 represents a ring selected from
   (1) cyclic group 1: 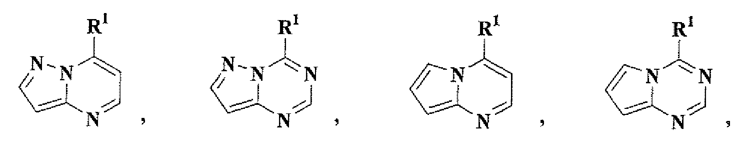 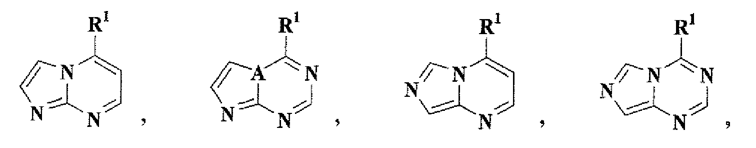 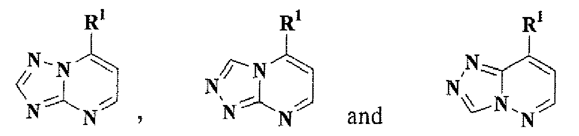 and
   (2) cyclic group 2: 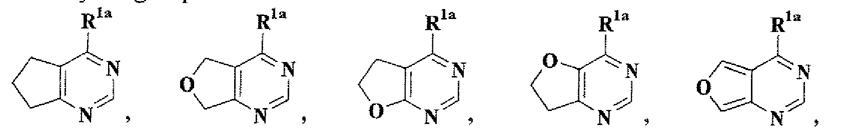 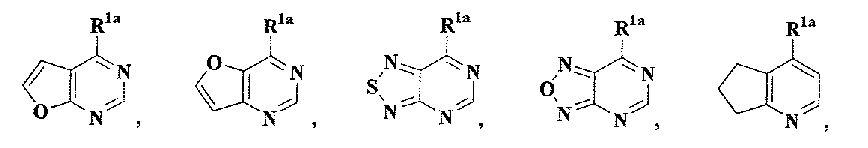 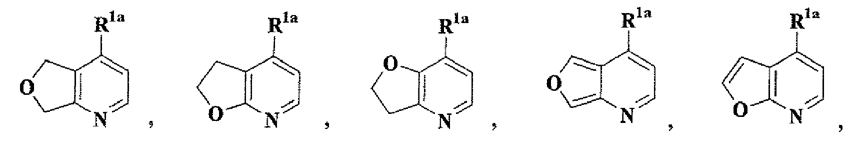 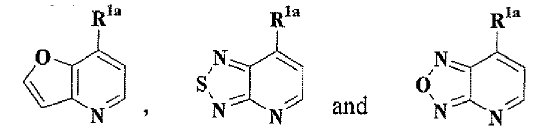 and ring A may be substituted with 1 to 3 substituents selected from a halogen atom, CF₃, OCF₃, hydroxyl, mercapto, carboxyl, (C1-6 alkoxy)carbonyl, carbamoyl, nitro, cyano, oxo, and C1-6 alkyl, C2-6 alkenyl, C2-6 alkynyl, C1-6 alkoxy or C1-6 alkylthio which each may be substituted with 1 to 3 substituents selected from a halogen atom, CF₃ and hydroxyl, and ring B may be further substituted;
      R¹ represents:
      (i) C1-15 alkyl, C2-15 alkenyl or C2-15 alkynyl which each may be substituted,
      (ii) amino which may be protected,
      (iii) hydroxyl which may be protected,
      (iv) mercapto which may be protected,
      (v) -S(O)ₙR⁶, in which n represents 1 or 2, and R⁶ represents (a) C1-15 alkyl, C2-15 alkenyl or C2-15 alkynyl which each may be substituted, or (b) a cyclic ring which may be substituted,
      (vi) -COR⁷, in which R⁷ represents (a) a hydrogen atom, (b) C1-15 alkyl, C2-15 alkenyl or C2-15 alkynyl which each may be substituted, (c) hydroxyl which may be protected, (d) amino which may be protected, or (e) a cyclic group which may be substitute, or
      (vii) a cyclic group which may be substituted;
      R^{1a} represents:
      (i) C1-15 alkyl or C2-15 alkenyl which may be substituted with substituent group 1,
      (ii) NR⁸R⁹, in which R⁸ represents (a) a hydrogen atom or (b) C1-15 alkyl or C2-15 alkenyl which each may be substituted with substituent group 1, and R⁹ represents (a) a hydrogen atom, (b) C1-15 alkyl or C2-15 alkenyl substituted with substituent group 1, (c) -COR¹⁰, in which R¹⁰ represents (aa) a hydrogen atom or (bb) C1-15 alkyl or C2-15 alkenyl which each may be substituted with substituent group 1, (d) -COOR¹⁰, in which R¹⁰ has the same meaning as described above, or (e) -CON(R⁸)₂, in which R⁸s each independently has the same meaning as described above,
      (iii) OR¹⁰, in which R¹⁰ has the same meaning described above,
      (iv) SR¹⁰, in which R¹⁰ has the same meaning described above,
      (v) S(O)ₙR¹¹, in which n represents 1 or 2, and R¹¹ represents C1-15 alkyl or C2-15 alkenyl which each may be substituted with substituent group 1, or
      (vi) COR¹², in which R¹² represents (a) a hydrogen atom, (b) C1-15 alkyl or C2-15 alkenyl which each may be substituted with substituent group 1, (c) -OR¹⁰, in which R¹⁰ has the same meaning as described above, or (d) -NR⁸R⁹, in which R⁸ and R⁹ have the same meanings as described above;
         the substituent group 1 represents (1) a halogen atom, (2) CF₃, (3) OCF₃, (4) cyano, (5) nitro, (6) hydroxyl, (7) C1-6 alkoxy, (8) carboxyl, (9) (C1-6 alkoxy)carbonyl, (10) C1-5 acyl, (11) carbamoyl in which a nitrogen atom may be protected with 1 or 2 of C1-6 alkyl, (12) C1-6 alkylthio, (13) C1-6 alkylsulfonyl, or (14) NR¹³R¹⁴, in which R¹³ represents (a) a hydrogen atom, (b) C1-6 alkyl, or (c) C2-6 alkenyl, and R¹⁴ represents (a) a hydrogen atom, (b) C1-6 alkyl, (c) C2-6 alkenyl, (d) -COR¹⁵, in which R¹⁵ represents (aa) a hydrogen atom, (bb) C1-6 alkyl or (cc) C2-6 alkenyl, (e) -COOR¹⁵, in which R¹⁵ has the same meaning as described above, or (f) -CON(R¹⁶)₂, in which R¹⁶s each independently represents a hydrogen atom or C1-6 alkyl;
         Z^{a} represents -NR³-, in which R³ represents a hydrogen atom, C1-6 alkyl, C2-6 alkenyl or C2-6 alkynyl which each may be substituted, -CO-(C1-6 alkyl which may be substituted), -SO₂-(C1-6 alkyl which may be substituted), an oxygen atom, a sulfur atom which may be oxidized, or -CR⁴R⁵-, in which R⁴ and R⁵ each independently represents a hydrogen atom, or C1-6 alkyl, C2-6 alkenyl or C2-6 alkynyl which each may be substituted, or R⁴ and R⁵ may be taken together to represent (i) oxo, (ii) C2-5 alkylene in which one carbon atom may be substituted with one oxygen atom, nitrogen atom or sulfur atom which may be oxidized, wherein the C2-5 alkylene may be substituted with a substituent(s), or (iii) Cl-6 alkylidene which may be substituted;
         R^{2a} represents (1) a C5-12 monocyclic or bicyclic unsaturated carbocyclic ring which may be substituted, (2) pyridine which may be substituted, (3) a bicyclic heterocyclic ring which may be substituted, in which benzene and a 5- or 6-membered monocyclic heterocyclic ring are fused, (4) a bicyclic heterocyclic ring which may be substituted, in which a pyridine ring and a C5-6 monocyclic carbocyclic ring are fused, or (5) a bicyclic heterocyclic ring which may be substituted, in which a pyridine ring and a 5-or 6-membered monocyclic heterocyclic ring are fused,
         a salt thereof, an N-oxide thereof, a solvate thereof or a prodrug thereof
3. The compound according to the above 2,
   wherein the ring 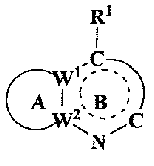 is 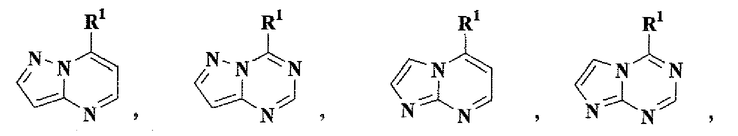 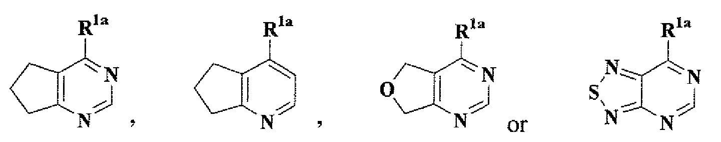 wherein all symbols have the same meanings as described in claim 2,
   a salt thereof, an N-oxide thereof, a solvate thereof or a prodrug thereof
4. The compound according to the above 2, wherein R¹ is amino which may be protected, or R^{1a} is NR⁸R⁹, in which R⁸ and R⁹ have the same meanings as described in the above 2, a salt thereof, an N-oxide thereof, a solvate thereof or a prodrug thereof
5. The compound according to the above 2, wherein Z^{a} is -NR³-, in which R³ has the same meaning as described in the above 2, a salt thereof, an N-oxide thereof, a solvate thereof or a prodrug thereof.
6. The compound according to the above 2, wherein Z^{a} is -CR^{4b}R^{5b}-, in which R^{4b} and R^{5b} are taken together to represent C2-5 alkylene in which one carbon atom may be substituted with one oxygen atom, nitrogen atom or sulfur atom which may be oxidized, wherein the C2-5 alkylene may be substituted with a substituent(s), a salt thereof, an N-oxide thereof, a solvate thereof or a prodrug thereof.
7. The compound according to the above 2, which is represented by formula (I-A-3): 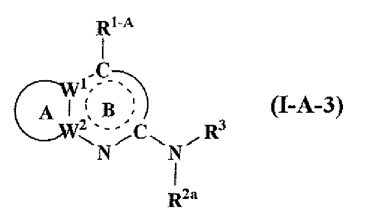 wherein 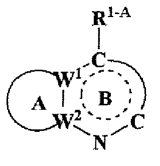 is 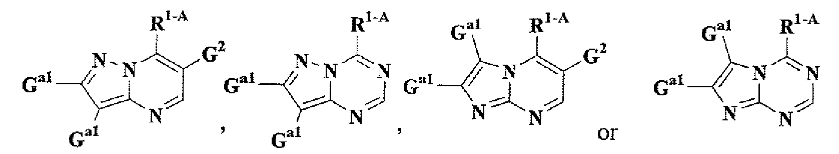 R^{1-A} represents amino which may be protected with 1 or 2 of C1-15 alkyl which may be substituted;
   G^{al}S each independently represents a hydrogen atom, a halogen atom, CF₃, OCF₃, hydroxyl, mercapto, carboxyl, (C1-6 alkoxy)carbonyl, carbamoyl, nitro, cyano, or C1-6 alkyl, C2-6 alkenyl, C2-6 alkynyl, C1-6 alkoxy or C1-6 alkylthio which each may be substituted with 1 or 2 substituents selected from a halogen atom, CF₃ and hydroxyl;
   G² represents a hydrogen atom, C1-15 alkyl, C2-15 alkenyl or C2-15 alkynyl which may be substituted, hydroxyl which may be protected, cyclopropane, cyclobutane, cyclopentane, cyclohexane, phenyl, a halogen atom, CF₃, or cyano; and other symbols have the same meanings as in the above 2,
   a salt thereof, an N-oxide thereof, a solvate thereof or a prodrug thereof.
8. The compound according to the above 2, which is represented by formula (I-A-4): 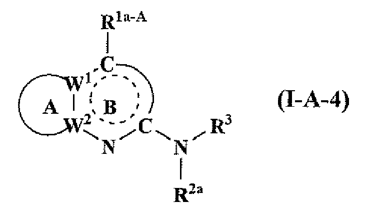 wherein 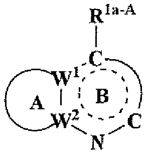 is 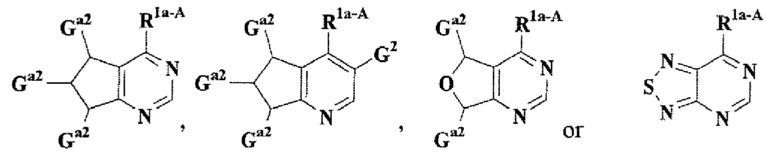 R^{1a-A} represents NR^{8A}R^{9A}, in which one of R^{8A} and R^{9A} represents C1-15 alkyl which may be substituted with the substituent group 1 and another represents a hydrogen atom or C1-15 alkyl which may be substituted with the substituent group 1, wherein the substituent group 1 has the same meaning as in the above 2;
   G^{a2}s each independently represents a hydrogen atom, a halogen atom, CF₃, OCF₃, hydroxyl, mercapto, carboxyl, (C1-6 alkoxy)carbonyl, carbamoyl, nitro, cyano, oxy, oxo, or C1-6 alkyl, C2-6 alkenyl, C2-6 alkynyl, C1-6 alkoxy or C1-6 alkylthio which each may be substituted with 1 or 2 substituents selected from a halogen atom, CF₃ and hydroxyl; and other symbols have the same meanings as described in the above 2 or 7,
   a salt thereof, an N-oxide thereof, a solvate thereof or a prodrug thereof
9. The compound according to the above 2, which is:
   (1) N⁵-(2-chloro-4-methoxyphenyl)-6-methyl-N⁷,N⁷-dipropylpyrazolo[1,5-a]pyrimidine-5,7-diamine,
   (2) N⁵-(2-chloro-4-methoxyphenyl)-N⁷-(1-ethylpropyl)-6-methylpyrazolo[1,5-a]pyrimidine-5,7-diamine,
   (3) N⁵-(2-chloro-4-methoxyphenyl)-6-ethyl-N⁷,N⁷-dipropylpyrazolo[1,5-a]pyrimidine-5,7-diamine,
   (4) N²-(2-chloro-4-methoxyphenyl)-N²-ethyl-N⁴,N⁴-dipropyl-6,7-dihydro-5H-cyclopcnta[d]pynmidine-2,4-diamine,
   (5) N⁵-(2-chloro-4-methoxyphenyl)-6-methoxy-N⁷,N⁷-dipropylpyrazolo[1,5-a]pyrimidine-5,7-diamine,
   (6) N²-allyl-N²-(2-chloro-4-methoxyphenyl)-N⁴,N⁴-dipropyl-6,7-dihydro-5H-cyclopenta[d]pyrimidine-2,4-diamine,
   (7) 6-methyl-N⁵-[2-methyl-4-(trifluoromethoxy)phenyl]-N⁷,N⁷⁻dipropylpyrazolo[1,5-a]pyrimidine-5,7-diamine,
   (8) N⁷-butyl-N⁵-(2-chloro-4-methoxyphenyl)-N⁷-ethyl-6-methylpyrazolo[1,5-a]pyrimidine-5,7-diamine,
   (9) N⁵-(2-ethyl-4-methylphenyl)-6-methyl-N⁷,N⁷-dipropylpyrazolo[1,5-a]pyrimidine-5,7-diamine,
   (10) 6-methoxy-N⁵-(4-methyl-2-vinylphenyl)-N⁷,N⁷-dipropylpyrazolo[1,5-a]pyrimidine-5,7-diamine, or
   (11) N⁵-(2-ethyl-4-methylphenyl)-6-methoxy-N⁷,N⁷-dipropylpyrazolo[1,5-a]pyrimidine-5,7-diamine,
      a salt thereof, an N-oxide thereof, a solvate thereof or a prodrug thereof
10. A pharmaceutical composition comprising, as an active ingredient, the compound represented by formula (I-A) according to the above 2, a salt thereof, an N-oxide thereof, a solvate thereof or a prodrug thereof
11. The pharmaceutical composition according to the above 10, which is a CRF antagonist.
12. The pharmaceutical composition according to the above 10, which is an agent for preventing and/or treating CRF mediated diseases.
13. The pharmaceutical composition according to the above 12, wherein the CRF mediated diseases are psychiatric and neurologic disorders or digestive diseases.
14. The pharmaceutical composition according to the above 13, wherein the psychiatric and neurologic disorders or the digestive diseases are mood disorders, anxiety disorders, stress-related disorders, eating disorders, symptom caused by psychotropic substance or dependency thereon, organic mental disorder, schizophrenic disorder, attention-deficit hyperactivity disorder or irritable bowel syndrome.
15. The pharmaceutical composition according to the above 14, wherein the psychiatric and neurologic disorders or the digestive diseases are depression, mood disorders, eating disorders, drug addiction, drug dependency or irritable bowel syndrome.
16. A medicament comprising a combination of the compound represented by formula (I-A) according to the above 2, a salt thereof, an N-oxide thereof, a solvate thereof or a prodrug thereof with at least one selected from a tricyclic antidepressant, a tetracyclic antidepressant, a monoamine oxidase inhibitor, a serotonin and noradrenaline reuptake inhibitor, a selective serotonin reuptake inhibitor, a serotonin reuptake inhibitor, a psychoanaleptic, an antianxiety agent, an antipsychotic agent, a mitochondrial benzodiazepine receptor ligand, an NK1 antagonist, a gastrointestinal promotility agent, a 5-HT₃ antagonist, a 5-HT₄ agonist, an anticholinergic agent, an antidiarrheal drug, a lapactic and an autonomic modulating agent.
17. A method for antagonizing CRF, which comprises administering to a mammal an effective amount of the compound represented by formula (I), a salt thereof, an N-oxide thereof, a solvate thereof or a prodrug thereof 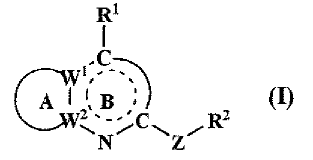 wherein all symbols have the same meanings as described in the above 1.
18. A method for preventing and/or treating a CRF mediated disease, which comprises administering to a mammal an effective amount of the compound represented by formula (I-A), a salt thereof, an N-oxide thereof, a solvate thereof or a prodrug thereof: 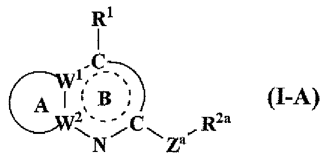 wherein all symbols have the same meanings as described in the above 2.
19. Use of the compound represented by formula (I), a salt thereof, an N-oxide thereof, a solvate thereof or a prodrug thereof for the manufacture of a CRF antagonist: 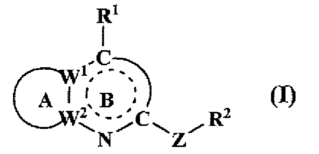 wherein all symbols have the same meanings as described in the above 1.
20. Use of the compound represented by formula (I), a salt thereof, an N-oxide thereof, a solvate thereof or a prodrug thereof for the manufacture of a CRF mediated disease: 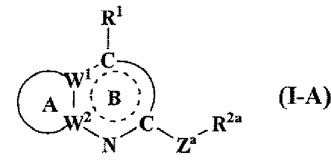
wherein all symbols have the same meanings as described in the above 2.
In the present invention, ring 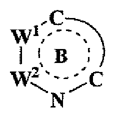 includes, for example,

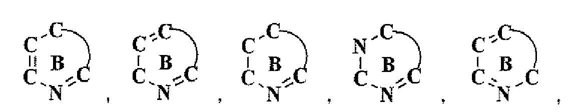

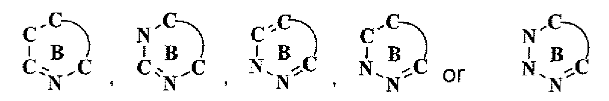

In the present invention, the 5- or 6-membered monocyclic ring includes a 5- or 6-membered monocyclic carbocyclic ring or monocyclic heterocyclic ring.

The 5- or 6-membered monocyclic carbocyclic ring includes, for example, cyclopentane, cyclohexane, cyclopentene, cyclohexene, cyclopentadiene, cyclohexadiene, and benzene rings.

The 5- or 6-membered monocyclic heterocyclic ring includes a 5- or 6-membered monocyclic heterocyclic ring containing 1 to 4 hetero atoms selected from a nitrogen atom, an oxygen atom and/or a sulfur atom which may be oxidized. Examples include pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydrofuran, tetrahydrofuran, dihydropyran, tetrahydropyran, dihydrothiophene, tetrahydrothiophene, dihydrothiopyran, tetrahydrothiopyran, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole (oxadiazolidine), dihydrooxazine, tetrahydrooxazine, dihydrooxadiazine, tetrahydrooxadiazine, dihydrothiadiazole, tetrahydrothiadiazole (thiadiazolidine), dihydrothiazine, tetrahydrothiazine; dihydrothiadiazine, tetrahydrothiadiazine, morpholine, thiomorpholine, oxathiane, pyrrole, imidazole, triazole, tetrazole, pyrazole, pyridine, pyrazine, pyrimidine, pyridazine, furan, thiophene, oxazole, isoxazole, thiazole, isothiazole, furazan, thiadiazole, pyran, thiopyran, oxazine, oxadiazine, thiazine and thiadiazine rings.

In the present invention, the 5- to 7-membered monocyclic unsaturated heterocyclic ring which may contain 1 or 2 hetero atoms selected from a nitrogen atom, an oxygen atom and/or a sulfur atom which may be oxidized, other than the nitrogen atom, W¹ and W² and which may be further substituted includes a 5- to 7-membered monocyclic unsaturated heterocyclic ring which may be substituted with 1 or 2 substituents selected from the substituent group 2, always contains one nitrogen atom and may contain 1 or 2 hetero atoms selected from a nitrogen atom, an oxygen atom and/or a sulfur atom which may be oxidized, other than the nitrogen atom, W¹ and W². Examples include pyrrole, imidazole, triazole, pyridine, pyrimidine, pyridazine, triazine, azepine, diazepine, oxazine, oxadiazine, oxazepine, oxadiazepine, thiazine, thiadiazine, thiazepine, and thiadiazepine rings.

In this connection, in ring A and ring B, the total number of the nitrogen atoms contained is 5 or less, and the total number of the oxygen atom and the sulfur atom which may be oxidized contained in ring A and ring B is 2 or less.

In the present invention, the substituent group 2 includes:
(i) C1-15 alkyl, C2-15 alkenyl or C2-15 alkynyl which each may be substituted,
(ii) amino which may be protected,
(iii) hydroxyl which may be protected,
(iv) mercapto which may be protected,
(v) -S(O)ₙR⁶, in which n and R⁶ have the same meanings as described above,
(vi) -COR⁷, in which R⁷ has the same meaning as described above,
(vii) a cyclic group which may be substituted, and
(viii) a halogen atom, CF₃, OCF₃, nitro, or cyano.

In the present invention, the sulfur atom which may be oxidized includes S, SO, and SO₂.

In the present invention, the C1-15 alkyl which may be substituted includes straight or branched C1-15 alkyl which may be substituted, and examples include methyl, ethyl, n-propyl, isopropyl, n-butyl, isobutyl, *sec*-butyl, *tert*-butyl, pentyl, hexyl, heptyl, octyl, nonyl, decyl, undecyl, dodecyl, tridecyl, tetradecyl and pentadecyl, which each may be substituted.

In the present invention, the C2-15 alkenyl which may be substituted includes straight or branched C2-15 alkenyl having 1 to 3 double bonds which may be substituted, and examples include vinyl, propenyl, butenyl, pentenyl, hexenyl, hexadienyl, heptenyl, heptadienyl, octenyl, octadienyl, nonenyl, nonadienyl, decenyl, decadienyl, undecenyl, dodecenyl, tridecenyl, tetradecenyl and pentadecenyl, which each may be substituted.

In the present invention, the C2-15 alkynyl which may be substituted includes straight or branched C2-15 alkynyl having 1 to 3 triple bonds which may be substituted, and examples include ethynyl, propynyl, butynyl, pentynyl, hexynyl, hexadiynyl, heptynyl, heptadiynyl, octynyl, octadiynyl, nonyl, decynyl, undecynyl, dodecynyl, tridecynyl, tetradecynyl and pentadecynyl, which each may be substituted.

In the present invention, the substituent group 1 in "C1-15 alkyl or C2-15 alkenyl which may be substituted with substituent group 1" may be substituted on 1 to 4 substitutable positions on the C1-15 alkyl or C2-15 alkenyl.

The substituent group 1 includes (1) a halogen atom, (2) CF₃, (3) OCF₃, (4) cyano, (5) nitro, (6) hydroxyl, (7) C1-6 alkoxy, (8) carboxyl, (9) (C1-6 alkoxy)carbonyl, (10) C1-5 acyl, (11) carbamoyl in which a nitrogen atom may be protected with 1 or 2 of C1-6 alkyl, (12) C1-6 alkylthio, (13) C1-6 alkylsulfonyl, and (14) NR¹³R¹⁴, in which R¹³ is (a) a hydrogen atom, (b) C1-6 alkyl, or (c) C2-6 alkenyl, and R¹⁴ represents (a) a hydrogen atom, (b) C1-6 alkyl, (c) C2-6 alkenyl, (d) -COR¹, in which R¹⁵ represents (aa) a hydrogen atom, (bb) C1-6 alkyl or (cc) C2-6 alkenyl, (e) -COOR¹⁵, in which R¹⁵ has the same meaning described above, or (f) -CON(R¹⁶)₂, in which R¹⁶s each independently represents a hydrogen atom or C1-6 alkyl.

In the present invention, the C1-6 alkyl which may be substituted includes straight or branched C1-6 alkyl which may be substituted, and examples include methyl, ethyl, n-propyl, isopropyl, n-butyl, isobutyl, *sec*-butyl, *tert*-butyl, pentyl and hexyl, which each may be substituted.

In the present invention, the C2-6 alkenyl which may be substituted includes straight or branched C2-6 alkenyl having one double bond which may be substituted, and examples include vinyl, propenyl, butenyl, pentenyl and hexenyl, which each may be substituted.

In the present invention, the C2-6 alkynyl which may be substituted includes straight or branched C2-6 alkynyl having one triple bond which may be substituted, and examples include ethynyl, propynyl, butynyl, pentynyl and hexynyl, which each may be substituted.

In the present invention, the C2-5 alkylene or the C2-5 alkylene which may be substituted includes methylene, methylene, ethylene, trimethylene, tetramethylene, pentamethylene and isomers thereof.

In the present invention, the C1-6 alkylidene which may be substituted includes methylidene, ethylidene, propylidene, pentylidene, hexylidene and isomers thereof

In the present invention, the "C1-6 alkyl which may be substituted", the "C2-6 alkenyl which may be substituted", the "C2-6 alkynyl which may be substituted", the "C2-5 alkylene which may be substituted", the "C1-6 alkylidene which may be substituted", the "C1-15 alkyl which may be substituted", the "C2-15 alkenyl which may be substituted", the "C2-15 alkynyl which may be substituted" and the "C1-15 alkoxy which may be substituted" include "substituted or unsubstituted C1-6 alkyl", "substituted or unsubstituted C2-6 alkenyl", "substituted or unsubstituted C2-6 alkynyl", "substituted or unsubstituted C2-5 alkylene", "substituted or unsubstituted C1-6 alkylidene", "substituted or unsubstituted C1-15 alkyl", "substituted or unsubstituted C2-15 alkenyl", "substituted or unsubstituted C2-15 alkynyl" and "substituted or unsubstituted C1-15 alkoxy", and the "substituent(s)" include the following substituent group 3.

The substituent group 3 includes (1) a halogen atom, (2) CF₃, (3) OCF₃, (4) cyano, (5) nitro, (6) hydroxyl which may be protected with C1-6 alkyl, C2-6 alkenyl, C2-6 alkynyl, a cyclic group or a protective group having leaving ability, (7) C1-7 acyl, (8) carbonyl which may be protected with C1-6 alkyl, C2-6 alkenyl, C2-6 alkynyl or a cyclic group, (9) carbamoyl which may be protected with C1-6 alkyl, C2-6 alkenyl, C2-6 alkynyl or a cyclic group, (10) thiol which may be protected with C1-6 alkyl, C2-6 alkenyl, C2-6 alkynyl or a cyclic group, (11) NR¹⁷R¹⁸, in which R¹⁷ represents (a) a hydrogen atom, (b) C1-6 alkyl, (c) C2-6 alkenyl, (d) C2-6 alkynyl, or (e) a cyclic group; and R¹⁸ represents (a) a hydrogen atom, (b) C1-6 alkyl, (c) C2-6 alkenyl, (d) C2-6 alkynyl, (e) -COR²⁰, in which R²⁰ represents (aa) a hydrogen atom, (bb) C1-6 alkyl, C2-6 alkenyl or C2-6 alkynyl or (cc) a cyclic group, (f) -COOR²⁰, in which R²⁰ has the same meaning as described above, or (g) -CON(R¹⁷)₂ in which R¹⁷s each independently has the same meaning as described above, (12) -S(O)ₙR¹⁹, in which n has the same meaning as described above, and R¹⁹ represents (a) a hydrogen atom, (b) C1-6 alkyl, C2-6 alkenyl or C2-6 alkynyl or (c) a cyclic group, (13) -COR²⁰, in which R²⁰ has the same meaning as described above, and (14) a cyclic group which may be substituted. These substituents may be substituted on 1 to 4 substitutable positions. Furthermore, the C1-6 alkyl, C2-6 alkenyl and C2-6 alkynyl in the substituent group 3 may be substituted with a substituent(s) selected from the substituent group 5, and the cyclic group may be substituted with a substituent(s) selected from the substituent group 4.

In the present invention, the halogen atom includes fluorine, chlorine, bromine and iodine.

In the present invention, C1-6 alkyl includes, for example, methyl, ethyl, n-propyl, isopropyl, n-butyl, isobutyl, *sec*-butyl, *tert*-butyl, pentyl and hexyl.

In the present invention, C2-6 alkenyl includes, for example, vinyl, propenyl, butenyl, pentenyl, hexenyl and hexadienyl.

In the present invention, C2-6 alkynyl includes, for example, ethynyl, propynyl, butynyl, pentynyl, hexynyl and hexadiynyl.

In the present invention, the hydroxyl which may be protected with C1-6 alkyl includes C1-6 alkoxy.

In the present invention, the C1-6 alkoxy includes, for example, methoxy, ethoxy, propoxy, isopropoxy, butoxy, isobutoxy, *sec*-butoxy, *tert*-butoxy, pentyloxy and hexyloxy.

In the present invention, the C1-15 alkoxy includes, for example, straight or branched C1-15 alkoxy, and examples include methoxy, ethoxy, propoxy, isopropoxy, butoxy, isobutoxy, *sec*-butoxy, *tert*-butoxy, pentyloxy, hexyloxy, heptyloxy, octyloxy, nonyloxy, decyloxy, undecyloxy, dodecyloxy, tridecyloxy, tetradecyloxy and pentadecyloxy.

In the present invention, the C1-6 alkylthio includes, for example, methylthio, ethylthio, propylthio, isopropylthio, n-butylthio, isobutylthio, *sec*-butylthio, *tert*-butylthio, pentylthio and hexylthio.

In the present invention, the C1-5 acyl includes, for example, formyl, acetyl, propanoyl, butanoyl, 2-methylpropanoyl and pivaloyl.

In the present invention, the C1-7 acyl includes, for example, formyl, acetyl, propanoyl, pivaloyl and benzoyl.

In the present invention, the (C1-6 alkoxy)carbonyl includes, for example, methoxycarbonyl, ethoxycarbonyl, propoxycarbonyl, isopropoxycarbonyl, butoxycarbonyl, isobutoxycarbonyl, *sec*-butoxycarbonyl, *tert*-butoxycarbonyl, pentyloxycarbonyl and hexyloxycarbonyl.

In the present invention, the carbamoyl in which a nitrogen atom may be protected with 1 or 2 of C1-6 alkyl includes N-(C1-6 alkyl)carbamoyl and N,N-di(C1-6 alkyl)carbamoyl.

In the present invention, the amino which may be protected includes amino protected with 1 or 2 of the following protecting groups, and unsubstituted amino. The amino-protecting groups include (a) C1-15 alkyl which may be substituted, (b) C2-15 alkenyl which may be substituted, (c) C2-15 alkynyl which may be substituted, (d) a cyclic group which may be substituted, (e) -COR²¹, in which R²¹ represents (aa) a hydrogen atom, (bb) C1-15 alkyl, C2-15 alkenyl or C2-15 alkynyl which each may be substituted or (cc) a cyclic group which may be substituted, (f) -COOR²¹, in which R²¹ has the same meaning as described above, and (g) -CON(R²²)₂, in which R²²_{S} each independently represents (aa) a hydrogen atom or (bb) C1-15 alkyl, C2-15 alkenyl or C2-15 alkynyl which each may be substituted.

In the present invention, hydroxyl which each may be protected includes, for example, (a) C1-15 alkyl which may be substituted, (b) C2-15 alkenyl which may be substituted, (c) C2-15 alkynyl which may be substituted, (d) a cyclic group which may be substituted, and (e) hydroxyl or hydroxyl protected with a protecting group having leaving ability. Herein, the protecting group having leaving ability includes, for example, trityl, methoxymethyl (MOM), 1-ethoxyethyl (EE), methoxyethoxymethyl (MEM), 2-tetrahydropyranyl (THP), trimethylsilyl (TMS), triethylsilyl (TES), t-butyldimethylsilyl (TBDMS), t-butyldiphenylsilyl (TBDPS), acetyl (Ac), pivaloyl, benzoyl, benzyl (Bn), p-methoxybenzyl, allyloxycarbonyl (Alloc) and 2,2,2-trichloroethoxycarbonyl (Troc). Also, the hydroxyl protected with C1-15 alkyl which may be substituted includes C1-15 alkoxy which may be substituted.

In the present invention, the mercapto which may be protected includes mercapto protected with (a) C1-15 alkyl which may be substituted, (b) C2-15 alkenyl which may be substituted, (c) C2-15 alkynyl which may be substituted or (d) a cyclic group which may be substituted, and unsubstituted mercapto.

In the present invention, the cyclic group includes a carbocyclic group and a heterocyclic group. The carbocyclic group includes a C3-12 monocyclic or bicyclic carbocyclic group, and examples include cyclopropane, cyclobutane, cyclopentane, cyclohexane, cycloheptane, cyclopentene, cyclohexene, cycloheptene, cyclopentadiene, cyclohexadiene, cycloheptadiene, benzene, pentalene, perhydropentalene, azulene, perhydroazulene, indene, perhydroindene, indane, naphthalene, dihydranaphthalene, tetrahydronaphthalene, perhydronaphthalene, heptalene, perhydroheptalene and bicyclo[3.1.1]heptane ring groups.

The heterocyclic group includes a C3-12 monocyclic or bicyclic heterocyclic ring containing 1 to 4 hetero atoms selected from a nitrogen atom, an oxygen atom and/or a sulfur atom which may be oxidized, and examples include oxirane, thiirane, aziridine, oxetane, thietane, azetidine, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrafuran, dihydropyran, tetrahydropyran, dihydrooxepine, tetrahydrooxepine, perhydrooxepine, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole (oxazolidine), dihydroisoxazole, tetrahydroisoxazole (isoxazolidine), dihydrothiazole, tetrahydrothiazole (thiazolidine), dihydroisothiazole, tetrahydroisothiazole (isothiazolidine), dihydrofurazan, tetrahydrofurazan, dihydrooxadiazole, tetrahydrooxadiazole(oxadiazolidine), dihydrooxazine, tetrahydrooxazine, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole (thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzooxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, pyrrole, imidazole, triazole, tetrazole, pyrazole, pyridine, pyrazine, pyrimidine, pyridazine, azepine, diazepine, furan, pyran, oxepine, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzoazepine, benzodiazepine, benzofurazan, benzothiadiazole and benzotriazole ring groups.

In the present invention, the cyclic group which may be substituted includes a carbocyclic group and a heterocyclic group, which each may be substituted with the substituent group 4. The carbocyclic group and the heterocyclic group include those groups described above.

The substituent group 4 includes (1) C1-6 alkyl, (2) C2-6 alkenyl, (3) C2-6 alkynyl, (4) hydroxyl which may be protected with C1-6 alkyl, C2-6 alkenyl, C2-6 alkynyl, a cyclic group or a protecting group having leaving ability, (5) mercapto which may be protected with C1-6 alkyl, C2-6 alkenyl, C2-6 alkynyl or a cyclic group, (6) amino which may be protected with 1 or 2 groups selected from C1-6 alkyl, C2-6 alkenyl, C2-6 alkynyl and a cyclic group, (7) carbamoyl which may be protected with 1 or 2 groups selected from C1-6 alkyl, C2-6 alkenyl, C2-6 alkynyl and a cyclic group, (8) sulfamoyl which may be protected with 1 or 2 groups selected from C1-6 alkyl, C2-6 alkenyl, C2-6 alkynyl and a cyclic group, (9) carboxyl which may be protected with C1-6 alkyl, C2-6 alkenyl, C2-6 alkynyl or a cyclic group, (10) nitro, (11) cyano, (12) amidino, (13) a halogen atom, (14) CF₃, (15) OCF₃, (16) C1-7 acyl, (17) oxo, and (18) thioxo. These substituents may be substituted on 1 to 5 substitutable positions. Furthermore, in the substituent group 4, the C1-6 alkyl, C2-6 alkenyl and C2-6 alkynyl may be substituted with a substituent(s) selected from the substituent group 5, and the cyclic group may be substituted with a substituent(s) selected from the substituent group 6.

The substituent group 5 includes (1) C1-6 alkoxy, (2) C1-6 alkylthio, (3) a halogen atom, (4) hydroxyl which may be protected with C1-6 alkyl, C2-6 alkenyl, C2-6 alkynyl, a cyclic group, cyclic group-C1-6 alkyl or a protecting group having leaving ability, (5) CF₃, (6) OCF₃, (7) nitro, (8) cyano, (9) carboxyl, (10) (C1-6 alkoxy)carbonyl, (11) benzyloxycarbonyl, (12) mercapto, (13) amino, (14) C1-6 alkylamino, (15) di(C1-6 alkyl)amino, (16) carbamoyl, (17) N-(C1-6 alkyl)carbamoyl, (18) N,N-di(C1-6 alkyl)carbamoyl, (19) sulfamoyl, (20) N-(C1-6 alkyl)sulfamoyl, (21) N-di(C1-6 alkyl)sulfamoyl, (22) C1-7 acyl, and (23) a cyclic group which may be substituted with the substituent group 6.

The substituent group 6 includes (1) C1-6 alkyl, (2) C2-6 alkenyl, (3) C2-6 alkynyl, (4) C1-6 alkoxy, (5) C1-6 alkylthio, (6) a halogen atom, (7) CF₃, (8) OCF₃, (9) nitro, (10) cyano, (11) hydroxyl which may be protected which may be protected with Cl-6 alkyl, C2-6 alkenyl, C2-6 alkynyl, a cyclic group, cyclic group-C1-6 alkyl or a protecting group having leaving ability, (12) carboxyl, (13) (C1-6 alkoxy)carbonyl, (14) benzyloxycarbonyl, (15) mercapto, (16) amino, (17) C1-6 alkylamino, (18) di(C1-6 alkyl)amino, (19) carbamoyl, (20) N-(C1-6 alkyl)carbamoyl, (21) N,N-di(C1-6 alkyl)carbamoyl, (22) sulfamoyl, (23) N-(C1-6 alkyl)sulfamoyl, (24) N-di(C1-6 alkyl)sulfamoyl, (25) C1-7 acyl, (26) oxo, and (27) thioxo.

In the present invention, the cyclic group-C1-6 alkyl includes carbocyclic group-C1-6 alkyl and heterocyclic group-C1-6 alkyl, such as C1-6 alkyl substituted with one carbocyclic group and C1-6 alkyl substituted with one heterocyclic group, respectively. The carbocyclic group, the heterocyclic group and the C1-6 alkyl have the same meanings as described above.

In the present invention, the unsaturated cyclic group which may be substituted includes an unsaturated carbocyclic group and an unsaturated heterocyclic group, which each may be substituted with 1 to 5 substituents selected from the substituent group 7.

The unsaturated carbocyclic group includes a C5-12 monocyclic or bicyclic unsaturated carbocyclic group, and examples include benzene, pentalene, indene, indane, naphthalene, dihydronaphthalene, tetrahydronaphthalene and azulene ring groups. In this connection, in the case of the indene, indane, dihydronaphthalene and tetrahydronaphthalene ring groups, the benzene ring in these ring groups is bound to the group Z.

The unsaturated heterocyclic group includes a 5- to 12-membered monocyclic or bicyclic unsaturated heterocyclic group containing 1 to 4 hetero atoms selected from a nitrogen atom, an oxygen atom and/or a sulfur atom which may be oxidized. Examples include pyrrole, imidazole, triazole, tetrazole, pyrazole, pyridine, pyrazine, pyrimidine, pyridazine, azepine, furan, pyran, oxepine, thiophene, thiopyran, thiepine, oxazole, isoxazole, thiazole, isothiazole, furazan, oxadiazole, oxazine, oxadiazine, thiadiazole, thiazine, thiadiazine, indole, isoindole, benzofuran, isobenzofuran, benzothiophene, isobenzothiophene, dithianaphthalene, indazole, quinoline, isoquinoline, phthalazine, quinoxaline, quinazoline, cinnoline, naphthyridine, benzoxazole, benzothiazole, benzimidazole, chromene, benzofurazan, benzothiadiazole, benzotriazole ring groups. In this connection, in the case of the indole, isoindole, benzofuran, isobenzofuran, benzothiophene, isobenzothiophene, dithianaphthalene, indazole, phthalazine, quinoxaline, quinazoline, cinnoline, benzoxazole, benzothiazole, benzimidazole, chromene, benzofurazan, benzothiadiazole and benzotriazole ring groups, the benzene ring in these ring groups is bound to the group Z.

The substituent group 7 includes (1) C1-15 alkyl which may be substituted, (2) C2-15 alkenyl which may be substituted, (3) C2-15 alkynyl which may be substituted, (4) hydroxyl which may be protected, (5) mercapto which may be protected, (6) amino which may be protected, (7) carbamoyl which may be protected, (8) sulfamoyl which may be protected, (9) carboxyl which may be protected, (10) sulfo which may be protected (-SO₃H), (11) sulfino which may be protected (-SO₂H), (12) nitro, (13) cyano, (14) amidino, (15) imino, (16) a halogen atom, (17) a cyclic group which may be substituted, (18) C1-7 acyl, (19) oxo, (20) thioxo and (21) sulfino which may be protected (-SOH). These substituents may be substituted on 1 to 5 substitutable positions.

In the present invention, the "protecting group" in the "carbamoyl which may be protected", the "sulfamoyl which may be protected", the "carboxyl which may be protected", the "sulfo which may be protected", the "sulfino which may be protected" and the "sulfino which may be protected" includes (a) C1-15 alkyl which may be substituted, (b) C2-15 alkenyl which may be substituted, (c) C2-15 alkynyl which may be substituted, and (d) a cyclic group which may be substituted.

In the present invention, the C5-12 monocyclic or bicyclic unsaturated carbocyclic ring which may be substituted includes a C5-12 monocyclic or bicyclic unsaturated carbocyclic ring which may be substituted with 1 to 5 substituents selected from the above substituent group 7, and examples include benzene, pentalene, indene, indane, naphthalene, dihydronaphthalene, tetrahydronaphthalene and azulene rings. In this connection, in the case of the indene, indane, dihydronaphthalene and tetrahydronaphthalene rings, the benzene ring in these rings is bound to Z^{a}.

In the present invention, the substituent in the pyridine which may be substituted includes 1 to 4 substituents selected from the above substituent group 7.

In the present invention, the bicyclic heterocyclic ring which may be substituted, in which benzene and a 5- or 6-membered monocyclic heterocyclic ring, includes a bicyclic heterocyclic ring which may be substituted with 1 to 5 substituents selected from the above substituent group 7, in which benzene and a 5- or 6-membered monocyclic heterocyclic ring are fused, and examples include indole, isoindole, indoline, isoindoline, benzofuran, isobenzofuran, dihydrobenzofuran, dihydroisobenzofuran, benzothiophene, isobenzothiophene, dihydrobenzothiophene, dihydroisobenzothiophene, chroman and isochroman rings, in which the benzene ring in these rings is bound to the group Z^{a}.

In the present invention, the bicyclic heterocyclic ring which may be substituted, in which a pyridine ring and C5-6 monocyclic carbocyclic ring are fused, include a bicyclic heterocyclic ring which may be substituted 1 to 5 substituents selected from the above substituent group 7, in which a pyridine ring and a C5-6 monocyclic carbocyclic ring are fused, and examples include quinoline, isoquinoline, tetrahydroquinoline and tetrahydroisoquinoline rings. In the case of the tetrahydroquinoline and tetrahydroisoquinoline rings, the pyridine ring binds to the group Z^{a}.

In the present invention, the bicyclic heterocyclic ring which may be substituted, in which a pyridine ring and a 5- or 6-membered monocyclic heterocyclic ring are fused, includes a bicyclic heterocyclic group which may be substituted with 1 to 5 substituents selected from the substituent group 7, in which a pyridine ring and a 5- or 6-membered monocyclic heterocyclic ring are fused, and examples include naphthyridine.

In the present invention, the C3-6 cycloalkyl which may contain 1 or 2 hetero atoms selected from an oxygen atom, a sulfur atom and a nitrogen atom includes cycldoropyl, cyclobutyl, cyclopentyl, cyclohexyl, oxirane, oxetane,- tetrahydrofuran, tetrahydropyran, thiirane, thietane, tetrahydrothiophene, tetrahydrothiopyran, aziridine, azetidine, pyrrolidine, piperidine, morpholine and thiomorpholine.

In the present invention, preferred rings as ring A are cyclopentane, cyclohexane, cyclopentene, cyclohexene, cyclopentadiene, cyclohexadiene, benzene, pyrroline, pyrrolidine, pyrrole, imidazoline, imidazolidine, imidazole, pyrazole, triazoline, triazolidine, triazole, tetrazoline, tetrazolidine, tetrazole, dihydrofuran, tetrahydrofuran, furan, dihydrothiophene, tetrahydrothiophene, thiophene, dihydrofurazan, tetrahydrofurazan, furazan, dihydrooxadiazole, tetrahydrooxadiazole (oxadiazolidine), dihydrothiadiazole, tetrahydrothiadiazole (thiadiazolidine) and thiadiazole. Particularly, cyclopentane, cyclopentene, pyrrole, imidazole, pyrazole, triazole, tetrahydrofuran, furan, furazan and thiadiazole are preferred.

In the present invention, ring A is preferably unsubstituted or substituted with 1 or 2 substituents selected from a halogen atom, CF₃, OCF₃, hydroxyl, mercapto, carboxyl, (C1-6 alkoxy)carbonyl, carbamoyl, nitro, cyano, oxo, and C1-6 alkyl, C2-6 alkenyl, C2-6 alkynyl, C1-6 alkoxy or C1-6 alkylthio which each may be substituted with 1 or 2 substituents selected from a halogen atom, CF₃ and hydroxyl. Ring A is more preferably unsubstituted ring A, or ring A substituted with 1 or 2 substituents selected from a halogen atom, CF₃, hydroxyl, carboxyl, (C1-6 alkoxy)carbonyl, cyano, oxo, and C1-6 alkyl or C1-6 alkoxy which each may be substituted with 1 or 2 substituents selected from a halogen atom, CF₃ and hydroxyl.

In the present invention, preferred rings as ring B are pyrrole, imidazole, pyridine, pyrimidine, pyridazine, triazine, azepine, diazepine, oxazine, oxazepine, thiazine and thiazepine. Particularly preferred are pyridine, pyrimidine, pyridazine and triazine.

In the present invention, ring B is preferably a ring having no substituent other than R¹ and -Z-R², or a ring further substituted with 1 or 2 substituents selected from the above substituent group 2. Ring B is more preferably ring B having no further substituent, or ring B further substituted with 1 or 2 substituents selected from C1-15 alkyl, C2-15 alkenyl or C2-15 alkynyl which each may be substituted, hydroxyl which may be protected, cyclopropane, cyclobutane, cyclopentane, cyclohexane, phenyl, a halogen atom, CF₃ and cyano in the above substituent group 2. Ring B is most preferably ring B having no further substituent or ring B further substituted with one substituent selected from C1-6 alkyl or C2-6 alkenyl which each may be substituted, hydroxyl which may be protected with C1-6 alkyl which may be substituted, cyclopropane, cyclobutane, a halogen atom, CF₃ and cyano. In this connection, the substituent of the C1-6 alkyl, C2-6 alkenyl or C2-6 alkynyl is preferably one substituent selected from C1-6 alkoxy, a halogen atom, hydroxyl, CN and COOH.

In the present invention, Z or Z^{a} is preferably -NR³-, in which R³ has the same meaning as described above, an oxygen atom, a sulfur atom which may be oxidized, -CR^{4a}R^{5a}-, in which R^{4a} and R^{5a} are taken together to represent (i) oxo, (ii) C2-5 alkylene in which one carbon atom may be substituted with one oxygen atom, nitrogen atom or sulfur atom which may be oxidized, the C2-5 alkylene being substituted with a substituent(s), or (iii) C1-6 alkylidene which may be substituted. Z or Z^{a} is preferably -NR³-, in which R³ has the same meaning as described above, or -CR^{4b}R^{5b}-, in which R^{4b} and R^{5b} are taken together to represent C2-5 alkylene in which one carbon atom may be substituted with one oxygen atom, nitrogen atom or sulfur atom which may be oxidized, the C2-5 alkylene being substituted with a substituent(s), and most preferably -NR³-, in which R³ has the same meaning as described above. Among these, -NR^{3a}-, in which R^{3a} is a hydrogen atom, C1-6 alkyl, substituted C1-6 alkyl, C2-6 alkenyl or substituted C2-6 alkenyl, is particularly preferred.

In the present invention, when the ring represented by R² and R^{2a} is a bicyclic unsaturated carbocyclic ring or bicyclic unsaturated heterocyclic ring containing benzene or a pyridine ring, the benzene or the pyridine ring is bound to the group Z or Z^{a}.

In the present invention, preferred compounds represented by formula (I) are those exemplified in the following Tables.

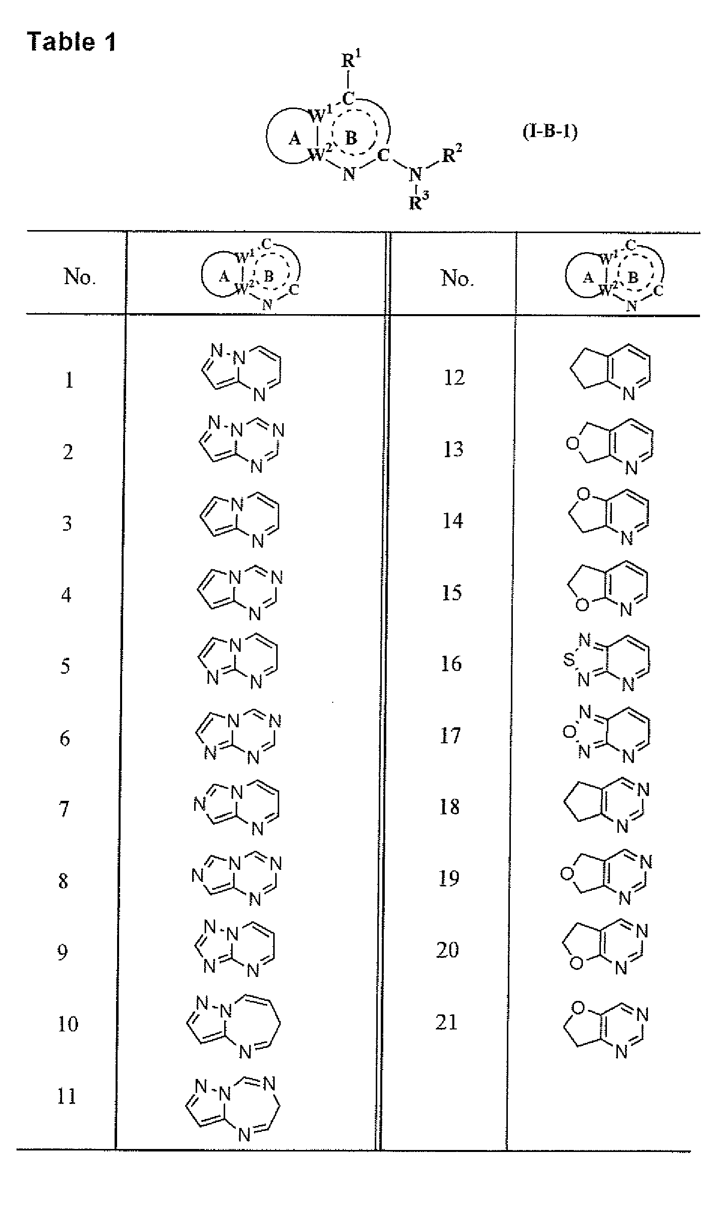

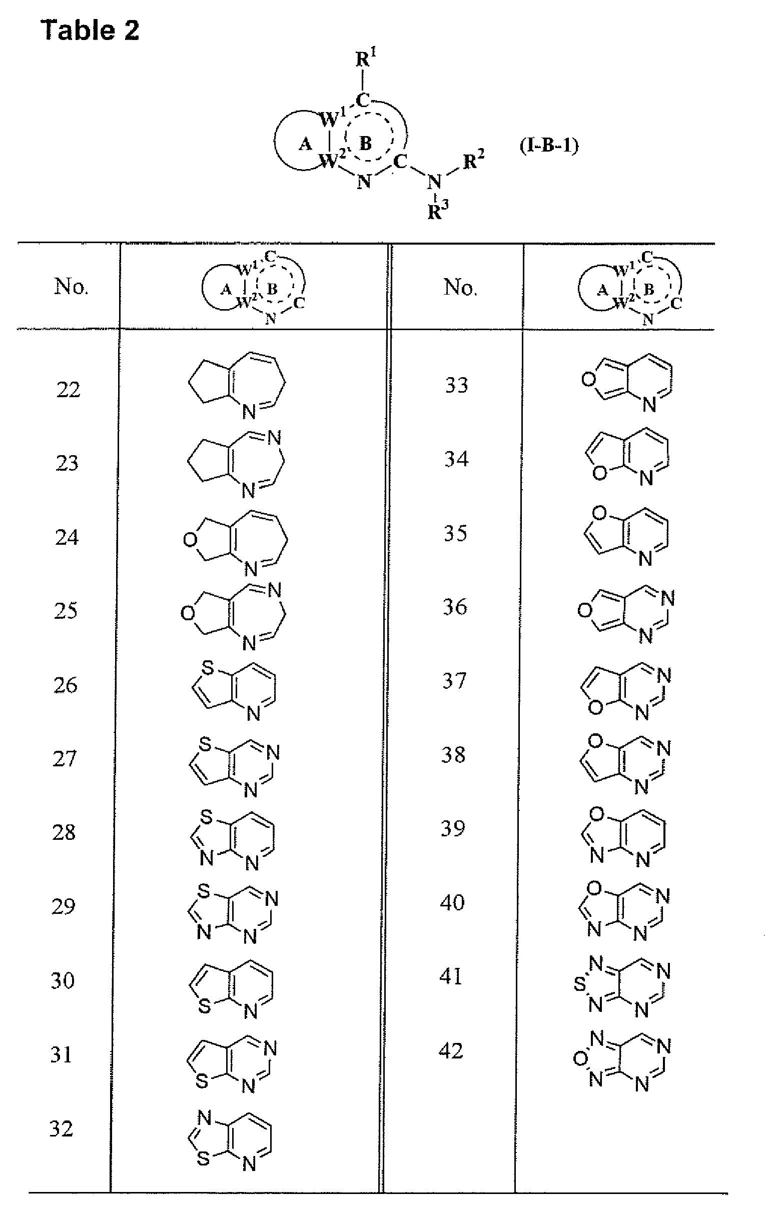

In the present invention, more preferred compounds are compounds of formula (I-A).

In the present invention, preferred compounds represented by formula (I-A) are those exemplified in the following Tables and following Example compounds.

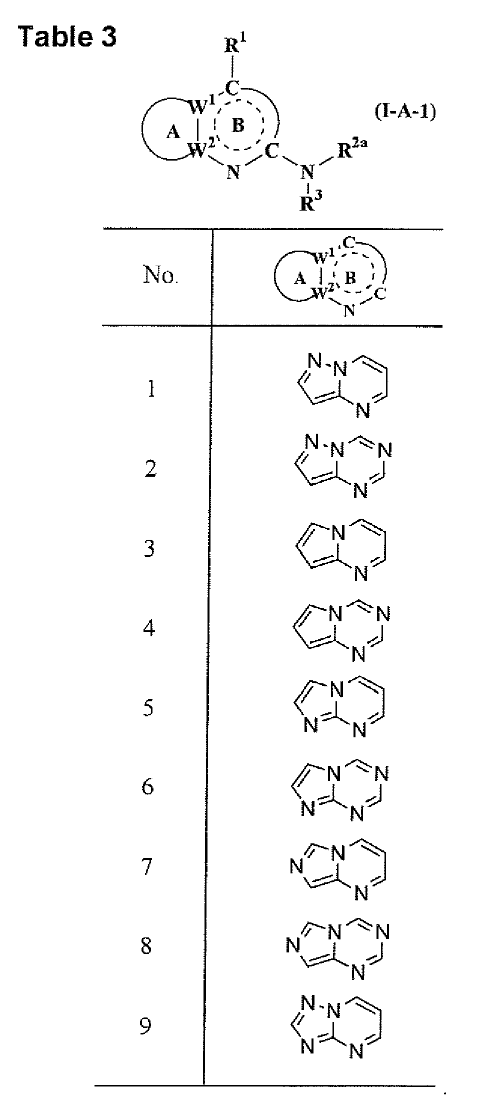

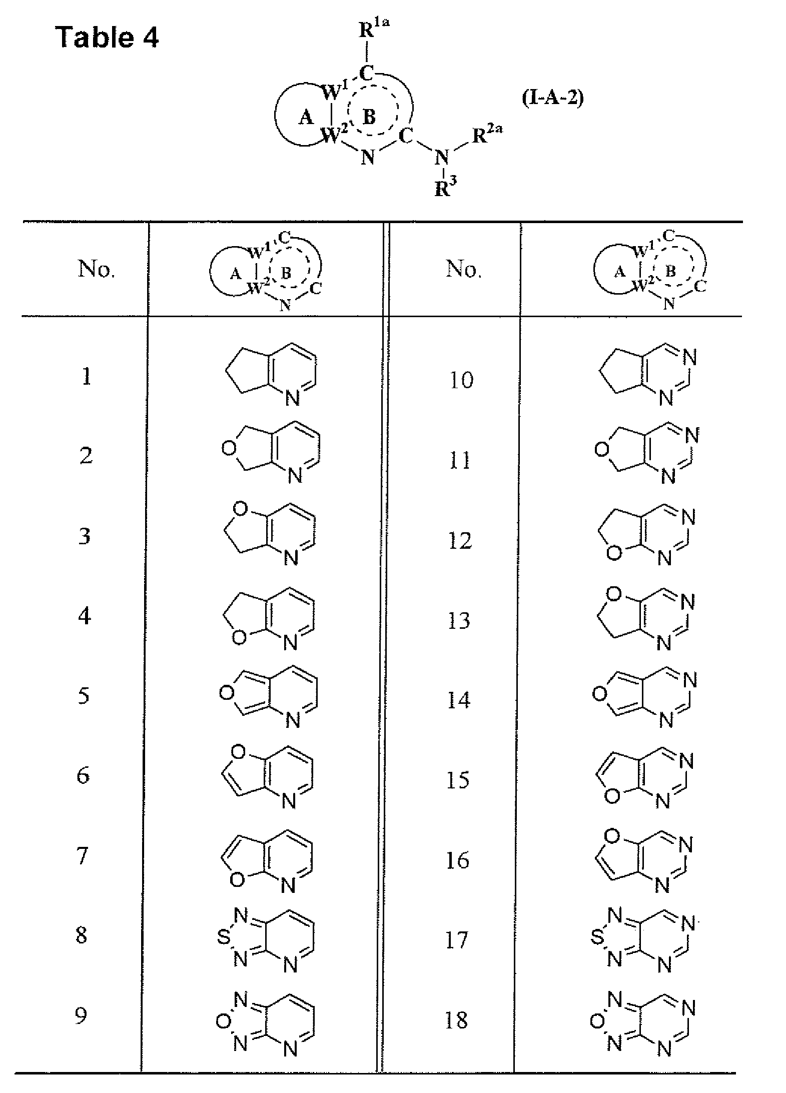

In Tables 1 to 4, ring A is preferably unsubstituted or substituted with 1 or 2 substituents selected from a halogen atom, CF₃, OCF₃, hydroxyl, mercapto, carboxyl, (C1-6 alkoxy)carbonyl, carbamoyl, nitro, cyano, oxo, and C1-6 alkyl, C2-6 alkenyl, C2-6 alkynyl, C1-6 alkoxy or C1-6 alkylthio which each may be substituted with 1 or 2 substituents selected from a halogen atom, CF₃ and hydroxyl. Ring A is more preferably unsubstituted ring A, or ring A substituted with 1 or 2 substituents selected from a halogen atom, CF₃, hydroxyl, carboxyl, (C1-6 alkoxy)carbonyl, cyano, oxo, and C1-6 alkyl or C1-6 alkoxy which each may be substituted with 1 or 2 substituents selected from a halogen atom, CF₃ and hydroxyl.

In Tables 1 to 4, each ring represented by ring B may be substituted with 1 or 2 substituents selected from the above substituent group 2 on substitutable position(s). Among the substituent group 2, C1-15 alkyl, C2-15 alkenyl or C2-15 alkynyl which each may be substituted, hydroxyl which may be protected, cyclopropane, cyclobutane, cyclopentane, cyclohexane, phenyl, a halogen atom, CF₃ and cyano are preferred. Ring B is more preferably unsubstituted ring B, or ring B substituted with one substituent selected from C1-6 alkyl or C2-6 alkenyl which each may be substituted, hydroxyl which may be protected with C1-6 alkyl which may be substituted, cyclopropane, cyclobutane, a halogen atom, CF₃ and cyano.

R¹ is preferably (i) C1-15 alkyl, C2-15 alkenyl or C2-15 alkynyl which each may be substituted, (ii) amino which may be protected, (iii) hydroxyl which may be protected, (iv) mercapto which may be protected, or (v) a cyclic group which may be substituted. When R¹ is a cyclic group which may be substituted and the cyclic group is a heterocyclic ring containing a nitrogen atom, the nitrogen atom is preferably bound to ring B.

In the present invention, R¹ is more preferably amino which may be protected. The amino which may be protected is preferably amino which may be protected with 1 or 2 of C1-15 alkyl which may be substituted, and more preferably amino substituted with one C1-15 branched or straight alkyl which may be substituted, or amino substituted with two C1-15 branched or straight alkyls which may be substituted. The C1-15 branched or straight alkyl which may be substituted is preferably unsubstituted C1-15 branched or straight alkyl, or C1-15 branched or straight alkyl substituted with 1 or 2 substituents selected from a halogen atom, CF₃, cyano, hydroxyl, C1-6 alkoxy, and C3-6 cycloalkyl which may contain 1 or 2 hetero atoms selected from an oxygen atom, a sulfur atom and a nitrogen atom.

R^{1a} is preferably (i) C1-15 alkyl, C2-15 alkenyl or C2-15 alkynyl which each may be substituted with the above substituent group 1, (ii) NR⁸R⁹, in which R⁸ and R⁹ have the same meanings as described above, (iii) OR¹⁰, in which R¹⁰ has the same meaning as described above. R^{1a} is more preferably NR⁸R⁹, in which R⁸ and R⁹ are each preferably (a) a hydrogen atom, or (b) C1-15 alkyl, C2-15 alkenyl or C2-15 alkynyl which each may be substituted with the above substituent group 1.

NR⁸R⁹ is preferably NR^{8A}R^{9A}, in which one of R^{8A} and R^{9A} is C1-15 alkyl which may be substituted with the substituent group 1, and another is a hydrogen atom or C1-15 alkyl which may be substituted with the above substituent group 1. More specifically, in NR^{8A}R^{9A}, preferred are (1) a combination in which R^{8A} is a hydrogen atom, and R^{9A} is C1-15 branched or straight alkyl which may be substituted with the above substituent group 1, and (2) a combination in which R^{8A} and R^{9A} are each C1-15 branched or straight alkyl which may be substituted with the above substituent group 1. The C1-15 branched or straight alkyl which may be substituted with the above substituent group 1 is preferably unsubstituted C1-15 branched or straight alkyl, or C1-15 branched or straight alkyl substituted with 1 or 2 substituents selected from a halogen atom, CF₃, cyano, hydroxyl and C1-6 alkoxy.

R² is preferably a monocyclic or bicyclic unsaturated carbocyclic ring which may be substituted, or a monocyclic or bicyclic unsaturated heterocyclic ring which may be substituted. R² is more preferably a ring represented by R^{2a}, that is, (1) a monocyclic or bicyclic unsaturated carbocyclic ring which may be substituted, (2) pyridine which may be substituted, (3) a bicyclic heterocyclic ring which may be substituted, in which benzene and a 5- or 6-membered monocyclic heterocyclic ring are fused, (4) a bicyclic heterocyclic ring which may be substituted, in which a pyridine ring and a C5-6 monocyclic carbocyclic ring are fused, or (5) a bicyclic heterocyclic ring which may be substituted, in which a pyridine ring and a 5- or 6-membered monocyclic heterocyclic ring are fused. R^{2a} is preferably a benzene, indene, indane, naphthalene, tetrahydronaphthalene, indole, isoindole, benzofuran, isobenzofuran, benzothiophene, isobenzothiophene, pyridine or naphthyridine ring, which may be substituted, and more preferably a benzene, naphthalene, tetrahydronaphthalene or pyridine ring, which may be substituted. In this connection, in the case of the indene, indane and tetrahydronaphthalene rings, the benzene ring in these rings is bound to Z or Z^{a}.

Also, the "substituent" substituted on the ring represented by R² and R^{2a} is preferably a substituent shown in the above substituent group 7. The substituent is preferably (1) C1-15 alkyl which may be substituted, (2) C1-15 alkenyl which may be substituted, (3) hydroxyl which may be protected, (4) mercapto which may be protected, (5) amino which may be protected, (6) carbamoyl which may be protected, (7) carboxyl which may be protected, (8) sulfo which may be protected, (9) sulfino which may be protected, (10) cyano, (11) a halogen atom, (12) a cyclic group which may be substituted, or (13) sulfino which may be protected. The substituent is more preferably (1) C1-6 alkyl, (2) C2-6 alkenyl, (3) unsubstituted hydroxyl, or hydroxyl protected with C1-6 alkyl which may be substituted or a protecting group having leaving ability (among these, particularly C1-6 alkoxy and trifluoromethoxy being preferred), (4) carboxyl, or carboxyl protected with C1-6 alkyl or benzyl, (5) cyano, (6) a halogen atom, or (7) a cyclic group which may be substituted. These substituents may be substituted on 1 to 5 substitutable positions on the ring represented by R² and R^{2a}, and 1, 2 or 3 substitution is particularly preferred. Particularly, when R² and R^{2a} are a 6-membered monocyclic ring, specifically benzene or a pyridine ring, substitution at (1) 2-position, (2) 3-position, (3) 4-position, (4) 2- and 4-positions, or (5) 2-, 4- and 6-positions is preferred.

R³ is preferably a hydrogen atom, C1-6 alkyl which may be substituted (for example, methyl, ethyl, n-propyl, isopropyl, n-butyl, isobutyl, *sec*-butyl, *tert*-butyl, pentyl or hexyl, which each may be substituted), or C2-6 alkenyl which may be substituted (for example, vinyl, propenyl, butenyl, pentenyl or hexenyl, which each may be substituted).

Among the compounds represented by formulae (I-A-1) and (I-A-2), 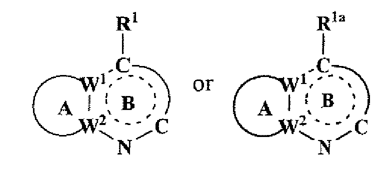 is more preferably 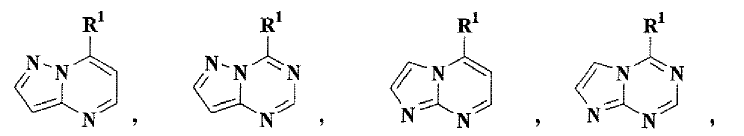 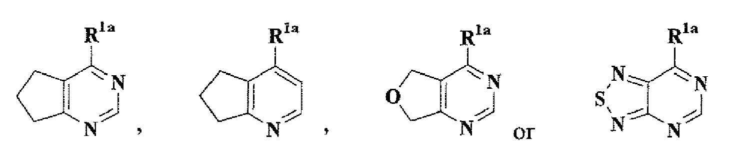 wherein ring A is unsubstituted, or substituted with 1 or 2 substituents selected from a halogen atom, CF₃, hydroxyl, carboxyl, (C1-6 alkoxy)carbonyl, cyano, oxo, and C1-6 alkyl or C1-6 alkoxy which each may be substituted with 1 or 2 substituents selected from a halogen atom, CF₃ and hydroxyl;
ring B is unsubstituted, or substituted with a substituent selected from C1-15 alkyl which may be substituted, C2-15 alkenyl, hydroxyl which may be protected, cyclopropane, cyclobutane, a halogen atom, CF₃ and cyano on substitutable position(s).

Also, the compound represented by formula (I-A) is more preferably a compound represented by formula (I-A-3): 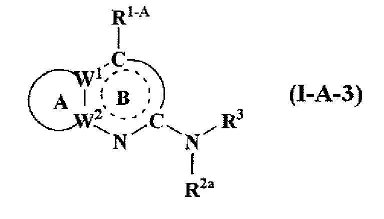 wherein 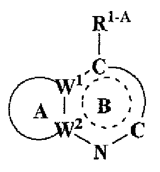 represents

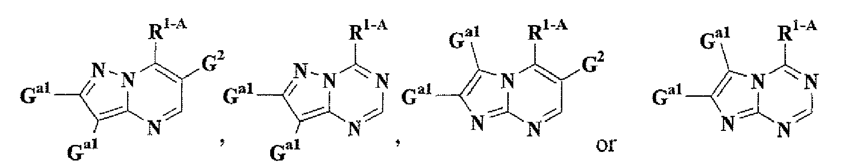

R^{1A} represents amino which may be protected with 1 or 2 of C1-15 alkyl which may be substituted; G^{a1}s each independently represents a hydrogen atom, a halogen atom, CF₃, OCF₃, hydroxyl, mercapto, carboxyl, (C1-6 alkoxy)carbonyl, carbamoyl, nitro, cyano, or C1-6 alkyl, C2-6 alkenyl, C2-6 alkynyl, C1-5 alkoxy or C1-6 alkylthio which each may be substituted with 1 or 2 substituents selected from a halogen atom, CF₃ and hydroxyl; G² represents a hydrogen atom, C1-15 alkyl, C2-15 alkenyl or C2-15 alkynyl which may be substituted, hydroxyl which may be protected, cyclopropane, cyclobutane, cyclopentane, cyclohexane, phenyl, a halogen atom, CF₃, or cyano, and other symbols have the same meanings as described above or
a compound represented by formula (I-A-4): 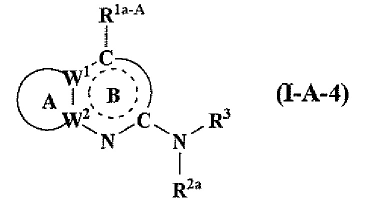 wherein 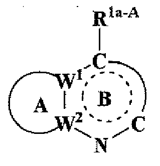 represents 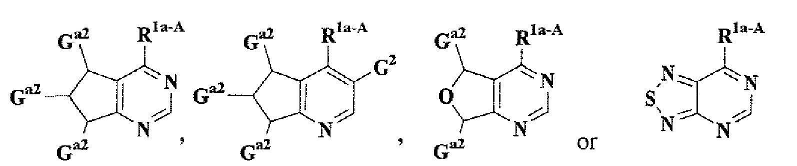 wherein R^{1aA} represents NR^{8A}R^{9A}, in which one of R^{8A} and R^{9A} represents C1-15 alkyl which may be substituted with the above substituent group 1, and another is a hydrogen atom or C1-15 alkyl which may be substituted with the above substituent group 1; G^{a2}S each independently represents a hydrogen atom, a halogen atom, CF₃, OCF₃, hydroxyl, mercapto, carboxyl, (C1-6 alkoxy)carbonyl, carbamoyl, nitro, cyano, oxo, or C1-6 alkyl, C2-6 alkenyl, C2-6 alkynyl, C1-6 alkoxy or C1-6 alkylthio which each may be substituted with 1 or 2 substituents selected from a halogen atom, CF₃ and hydroxyl; and other symbols have the same meanings as described above.

In the present invention, specific compounds are following compounds described in Examples. Preferable compounds are
(1) N⁵-(2-chloro-4-methaxyphenyl)-6-methyl-N⁷,N⁷-dipropylpyrazolo[1,5-a]pyrimidine-5,7-diamine,
(2) N⁵-(2-chloro-4-methoxyphenyl)-N⁷-(1-ethylpropyl)-6-methylpyrazolo[1,5-a]pyrimidine-5,7-diamine,
(3) N⁵-(2-chloro-4-methoxyphenyl)-6-ethyl-N⁷,N⁷-dipropylpyrazola[1,5-a]pyrimidine-5,7-diamine,
(4) N²-(2-chloro-4-methoxyphenyl)-N²-ethyl-N⁴,N⁴-dipropyl-6,7-dihydro-5H-cyclopenta[d]pyrimidine-2,4-diamine,
(5) N⁵-(2-chloro-4-methoxyphenyl)-6-methoxy-N⁷,N⁷-dipropylpyrazolo[1,5-a]pyrimidine-5,7-diamine,
(6) N²-aryl-N²-(2-chloro-4-methoxyphenyl)-N⁴,N⁴-dipropyl-6,7-dihydro-5H-cyclopenta[d]pyrimidine-2,4-diamine,
(7) 6-methyl-N⁵-[2-methyl-4-(trifluoromethoxy)phenyl]-N⁷,N⁷⁻dipropylpyrazolo[1,5-a]pyrimidine-5,7-diamine,
(8) N⁷-butyl-N⁵-(2-chloro-4-methoxyphenyl)-N⁷-ethyl-6-methylpyrazolo[1,5-a]pyrimidine-5,7-diamine,
(9) N⁵-(2-ethyl-4-methylphenyl)-6-methyl-N⁷,N⁷-dipropylpyrazolo[1,5-a]pyrimidine-5,7-diamine,
(10) 6-methoxy-N⁵-(4-methyl-2-vinylphenyl)-N⁷,N⁷-dipropylpyrazolo[1,5-a]pyrimidine-5,7-diamine, or
(11) N⁵-(2-ethyl-4-methylphenyl)-6-methoxy-N⁷,N⁷-dipropylpyrazolo[1,5-a]pyrimidine-5,7-diamine.

Unless otherwise specified, all isomers are included in the present invention. For example, alkyl, alkenyl, alkynyl, alkoxy, alkylthio, alkylene, alkenylene and alkynyl include straight and branched isomers. Isomers based on double bond, ring, fused ring (E, Z, cis, trans), isomers resulting from the presence of asymmetric carbon(s) (R-configuration, S-configuration, α-configuration, β-configuration, enantiomers, diastereoisomers), optically active compounds having optical rotation (D, L, d, 1-configuration), polar compounds obtained by chromatographic separations (highly polar compound, less polar compound), equilibrium compounds, rotational isomers, the mixtures are existed by free ratio, racemic mixtures are included in the present invention.

In the present invention, as is apparent to one skilled in the art, unless otherwise indicated,
the mark 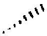 shows that the bond is on the other side of paper (α-configuration),
the mark 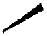 shows that the bond is in front of paper (β- configuration),
the mark 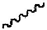 shows that the bond is α- configuration or β- configuration, and
the mark 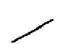 shows that the bond is a mixture of α- configuration and β-configuration.

The compound of the present invention of formula (I) may be converted into a non-toxic salt or a corresponding pharmaceutically acceptable salt by known methods. In the present invention, non-toxic salts or pharmaceutically acceptable salts are included. As pharmaceutically acceptable salts are non-toxic and water-soluble salts are preferable.

Appropriate salts are, salts of alkali metals, such as potassium, sodium, lithium; salts of alkaline-earth metals, such as calcium, magnesium; ammonium salts, such as tetramethylammonium, tetrabutylammonium; salts of organic amines, such as triethylamine, methylamine, dimethylamine, cyclopentylamine, benzylamine, phenethylamine, piperidine, monoethanolamine, diethanolamine, tris(hydroxymethyl)methylamine, lysine, arginine, N-methyl-D-glucamine, acid addition salts, for example, inorganic acids, such as hydrochloride, hydrobromide, hydroiodide, sulfate, phosphate, nitrate; salts of organic acid, such as acetate, trifluoroacetate, lactate, tartrate, oxalate, fumarate, maleate, benzoate, citrate, methanesulphonate, ethanesulphonate, benzenesulphonate, toluenesulphonate, isethionate, glucuronate, gluconate.

*N*-oxides are the compounds where nitrogen of the compound of formula (I) and formula (I-A) is oxidized. The present compound may be converted into an N-oxide compound by any known methods.

Besides, in the present invention, solvates of the compound of formula (I), solvates of the above-described a non-toxic salt or a corresponding pharmaceutically acceptable salt of formula (I) and solvates of the above-described an N-oxide compound of formula (I) are included. The solvates are preferably non-toxic and water-soluble. The appropriate solvates include, for example, solvates such as water, alcohol solvents (ethanol, *etc.),* and the like.

The prodrug for the compound of formula (I) or formula (I-A) means a compound which is converted to the compound of formula (I) or formula (I-A) by reaction with an enzyme, a gastric acid, or the like, in the living body. Examples of the prodrug for the compound of formula (I) or formula (I-A) include a compound wherein amino of the compound of formula (I) or formula (I-A) is substituted with acyl, alkyl, phosphoric acid, or the like (e.g., a compound wherein amino of the compound of formula (I) or formula (I-A) is substituted with eicosanyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl, acetoxymethy, *tert*-butyl, *etc.*)*;* a compound wherein hydroxyl of the compound of formula (I) or formula (I-A) is substituted with acyl, alkyl, phosphoric acid, boric acid, or the like (e.g., a compound wherein hydroxyl of the compound of formula (I) or formula (I-A) is modified with acetyl, palmitoyl, propanoyl, pivaloyl, succinyl, fumaryl, alanyl, dimethylaminomethylcarbonyl, *etc.);* a compound wherein carboxyl of the compound of formula (I) or formula (I-A) is modified with ester, amide, or the like (e.g., a compound wherein carboxyl of the compound of formula (I) or formula (I-A) is modified with ethyl ester, isopropyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl) methyl ester, cyclohexyloxycarbonylethyl ester, methyl amide, *etc.),* and the like. a compound wherein carboxyl of the compound of formula (I) or formula (I-A) is modified hydroxymethyl. These compounds may be prepared by per se known method. In addition, the prodrug for the compound of formula (I) or formula (I-A) may hydrate or non-hydrate.

### Production process of the compound of the present invention

The compound of the present invention can be produced, for example, by the following processes.

A compound represented by formula (I): 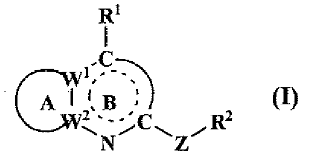 wherein all symbols have the same meanings as described above,
can be produced by reacting a compound represented by formula (II): 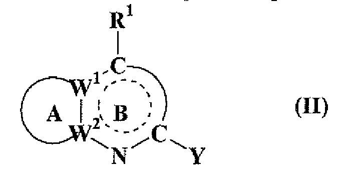 wherein Y represents a leaving group (for example, a halogen atom, mesyl, tosyl, mesyloxy, tosyloxy, trifluoromethansulfonyloxy, methylthio),
with a compound represented by formula (III):

**H-Z-R**^{**2**} **(III)**

wherein all symbols have the same meanings as described above.

The above reaction is known, and is carried out, for example, by heating at 50°C to 250°C in an organic solvent (for example, N-methylpyrrolidone, dimethylformamide, isopropanol) or without solvent. The heating is carried out by water bath, oil bath, sand bath or microwave.

Also, the compound can be produced by reacting a compound represented by formula (II) with a compound represented by formula (III-1):

**H**_{**2**}**N―R**^{**2**} **(III-1)**

wherein R² has the same meaning as described above,
to obtain a compound represented by formula (I-1): 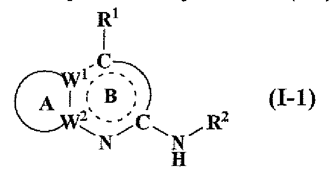 wherein all symbols have the same meanings as described above,
followed by N-alkylation reaction.

The reaction of the compound represented by formula (II) with the compound represented by formula (III-1) is carried out in the same manner as in the above reaction of the compound represented by formula (II) with the compound represented by formula (III).

The N-alkylation reaction is known and is carried out, for example, by using a corresponding alkyl halide (for example, methyl iodide) at 0 to 40°C in the presence of a base (for example, sodium hydride) in an organic solvent (for example, dimethylformamide, dimethylacetamide, 1,3-dimethyl-2-imidazolidinone, tetrahydrofuran)

Also, a compound in which the ring 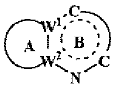 is a [1,2,5]thiadiazol[3,4-d]pyrimidine in the compounds represented by formula (I), can be produced by reacting a compound represented by formula (IV): 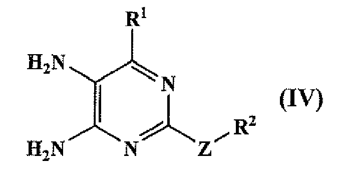 wherein all symbols have the same meanings as described above,
with thionyl chloride or thionylaniline.

The above reaction is known and is carried out, for example, at 50 to 120°C in an organic solvent or without solvent.

Furthermore, the compound represented by formula (I) can be produced by reacting a compound represented by formula (XI): 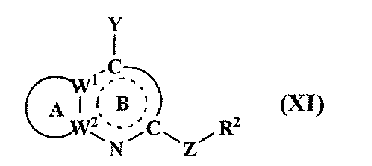 wherein all symbols have the same meanings as described above,
with a compound represented by formula (VI)

**H―R**^{**1**} **(VI)**

wherein R¹ has the same meaning as described above.

This reaction is known and is carried out, for example, at room temperature to reflux temperature in the presence of a tertiary amine (for example, triethylamine, diisopropylethylamine) in an organic solvent (for example, tetrahydrofuran, isopropanol) or at room temperature to reflux temperature without solvent.

Moreover, the compound represented by formula (I) in which Z is -CO- can be produced by reacting a compound represented by formula (XII): 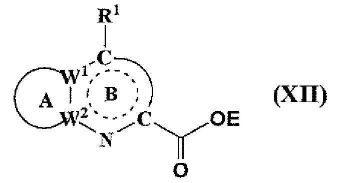 wherein E represents C1-4 alkyl, and other symbols have the same meanings as described above,
with a compound represented by formula (XIII):

**M-R**^{**2**} **(XIII)**

wherein M is magnesium-Y^{a}, in which Y^{a} represents a halogen atom, or lithium; and R² has the same meaning as described above.

The reaction is known and is carried out, for example, at -40°C to 0°C in an organic solvent (for example, tetrahydrofuran, diethyl ether). Additionally, the compound represented by formula (XIII) is produced, for example, by reacting a compound represented by formula (XIV):

**Y**^{**a**}**-R**^{**2**} **(XIV)**

wherein all symbols have the same meanings as described above,
with a Grignard reagent (for example, methyl magnesium bromide, isopropyl magnesium bromide, phenyl magnesium bromide, butyl lithium, phenyl lithium, or the like) or an alkyl lithium reagent (for example, butyl lithium, *sec*-butyl lithium, *tert*-butyl lithium, or the like).

The compound represented by formula (II) can be produced, for example, by a method shown in the following reaction scheme A, wherein all symbols have the same meanings as described above:

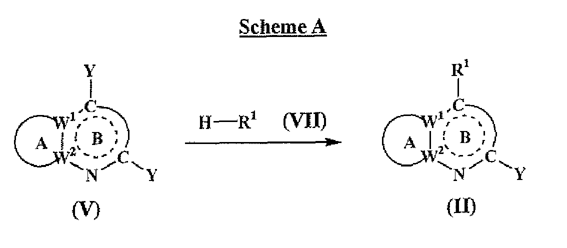

The compound represented by formula (IV) can be produced, for example, by a method shown in the following reaction scheme B, wherein all symbols have the same meanings as described above:

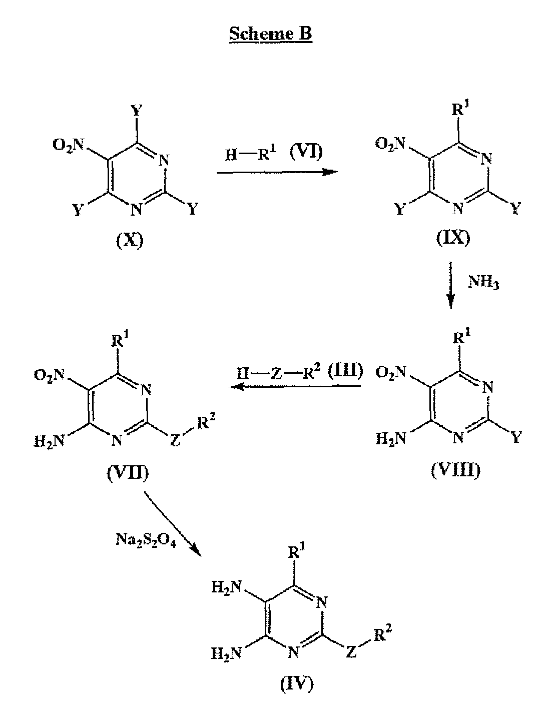

The compounds represented by formulae (V) and (X) *per se* are known or can be produced by known methods. For example, 2,4-dichloro-6,7-dihydro-5H-cyclopenta[d]pyrirnidine is described in *J. Amer. Chem. Soc.,* 81, 3118-3111 (1959).

Also, 4-chloro-2-(methylthio)pyrazolo[1,5-a][1,3,5]triazine can be produced, for example, by a method shown in the following reaction scheme C:

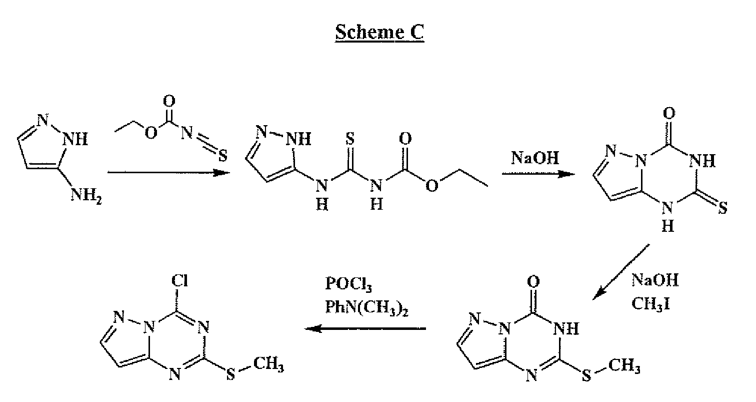

Furthermore, 2-ethylthiothieno[3,2-d]pyrimidin-4-on can be produced by the method described in JP-A-3-17083, and 2-ethylthiothieno[2,3-d]pyrimidin-4-on can be produced by the method described in U.S. Patent 4,146,716.

Moreover, 2,4-dichloro-5,7-dihydrofuro[3,4-d]pyrimidine, 5,7-dichloropyrazolo[1,5-a]pyrimidine, 5,7-dichloro-6-methylpyrazolo[1,5-a]pyrimidine, 5,7-dichloroimidazo[1,2-a]pyrimidine, and 5,7-dichloro-6-methylimidazo[1,2-a]pyrimidine can be respectively produced by the methods described in Examples later.

The compound represented by formula (XI) can be produced by reacting the compound represented by formula (V) with the compound represented by formula (III). Also, it can be produced by the method described in Example later.

The compound represented by formula (XII) can be produced, for example, by a method described in the following reaction scheme D, wherein all symbols have the same meanings as described above:

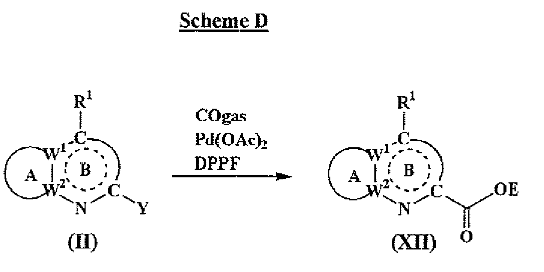

Also, in the present invention, other stating materials and each reagent *per se* are known or can be produced by known methods.

In each reaction in the present specification, reaction products can be purified by a usual purification means, for example, distillation under normal pressure or reduced pressure distillation, high-performance liquid chromatography using silica gel or magnesium silicate, thin-layer chromatography, column chromatography, washing, recrystallization or the like. The purification may be carried out after each reaction stage or after some reaction stages.

And the starting materials and reagents in the present invention may be known per se or may be prepared by known methods.

In each reaction in the present specification, reaction products may be purified by conventional purification techniques, e.g. by distillation under atmospheric or reduced pressure, by high performance liquid chromatography, by thin layer chromatography or by column chromatography using silica gel or magnesium silicate; or by washing or by recrystallization. Purification may be carried out after each reaction or after a series of reactions.

### Toxicity

The toxicity of the compounds of formula (I) and formula (I-A) of the present invention is very low and therefore, it is confirmed that these compounds are safe for use as medicine.

### Application to Pharmaceuticals

The compounds of the present invention of the formula (I) and formula (I-A) are useful, in order to possess CRF receptor binding activity and antagonistic activity, for the prevention and/or treatment of CRF mediated diseases, for example, psychiatric and neurologic disorders, digestive diseases, pulmonary disorders, endocrine disorders, metabolic disorders, cardiovascular disorders, dermatologic disorders, genitourinary disorders, ophthalmologic disorders or musculoskeletal disorders.

In particular, as psychiatric and neurologic disorders, for example, mood disorders such as depression, single episode depression, recurrent depression, postpartum depression, child abuse induced depression, bipolar disorder, premenstrual dysphoric disorder, dysphoric disorder in around the time of climacteric, perimenopausal dysphoric disorder; anxiety disorders such as generalized anxiety disorder, panic disorder, obsessive compulsive disorder, phobic disorders such as acrophobia, claustrophobia, agoraphobia, social phobia; adjustment disorders such as emotional injury, conduct disorder or disorder with both, physical complaint, isolating oneself from society, occupational stagnant, stagnant academic achievement; stress-related disorders such as posttraumatic stress disorder (PTSD), stress induced immunosuppression, stress induced headache, stress induced fever, stress induced pain, post operative stress, stress induced gastrointestinal disorder, irritable bowel syndrome; eating disorders such as anorexia nervosa, binge eating disorder, nervous vomiting; symptom caused by psychotropic substance or dependency thereon such as alcoholic withdrawal symptoms, alcohol dependence, drug addiction, drug dependency; organic mental disorder such as senile dementia of Alzheimer's type, multi-infarct dementia; schizophrenic disorder; attention-deficit hyperactivity disorder; neurodegenerative diseases such as Alzheimer's disease, Parkinson's disease, Huntington's disease, amyotrophic lateral sclerosis; pain; convulsive disorders such as convulsion, muscle spasm; episodic diseases such as epilepsy, attack, migraine; or sleep disorders such as nonorganic sleep disorder, fiber myalgic sleep disorder are given. As digestive diseases, for example, peptic ulcer; inflammatory bowel disease such as ulcerative colitis, Crohn's disease; irritable bowel syndrome; gastrointestinal disorder; diarrhea; constipation are given. As pulmonary disorders, for example, asthma, bronchitis, chronic obstructive pulmonary disease, allergic rhinitis is given. As endocrine disorders, for example, disturbed thyroid function, Cushing's disease or syndrome of inappropriate antidiuretic hormone secretion is given. As metabolic disorders, for example, obesity or hypoglycemia is given. As cardiovascular disorders, for example, hypertension, ischemic heart disease or cerebral vascular disease is given. As dermatologic disorders, for example, atopic dermatitis, allergic contact dermatitis or psoriasis is given. As genitourinary disorders, for example, urinary disturbance, pollakiuria or urinary incontinence is given. As ophthalmologic disorders, for example, uveitis is given. As musculoskeletal disorders, for example, chronic rheumatoid arthritis, osteoarthrosis or osteoporosis are given.

A combination agent obtained by combining the compound of formula (I) or formula (I-A) with other medicaments may be administered to accomplish the following purposes (1) to supplement and/or enhance the preventive and/or therapeutic effect of the present compound; (2) to improve the kinetics and/or absorption and reduce the dose of the present compound; and/or (3) to eliminate the side effects of the present compound.

A combination of the compound of formula (I) or formula (I-A) or a salt thereof, a solvate thereof or a prodrug thereof and other medicaments may be administered in the form of the formulations having these components incorporated in one preparation, or may be administered in separate preparations. In the case where these medicaments are administered in separate preparations, they may be administered simultaneously or at different times. In the latter case, the compound of formula (I) or formula (I-A) or a salt thereof, a solvate thereof or a prodrug thereof may be administered before the other medicaments. Alternatively, the other medicaments may be administered before the compound of formula (I) or formula (I-A) or a salt thereof, a solvate thereof or a prodrug thereof The method for the administration of these medicaments are the same or different.

The diseases on which the preventive and/or therapeutic effect of the above mentioned combination preparations works are not specifically limited but may be those for which the preventive and/or therapeutic effect of the compound represented by formula (I) or formula (I-A) or a salt thereof, a solvate thereof or a prodrug thereof or other medicaments is supplemented and/or enhanced.

The weight ratio of the compound of formula (I) or formula (I-A) or a salt thereof, a solvate thereof or a prodrug thereof and the other medicaments is not specifically limited.

The other medicaments are not limited to low molecular compound, may be macromolecular protein, polypeptide and polynucleotide (DNA, RNA, gene), antisense, decoy, antibody or vaccine *etc.* The dosage of other medicaments can be properly selected based on the dosage which can for on clinical it.

The weight ratio of the compound of formula (I) or formula (I-A) or a salt thereof, a solvate thereof or a prodrug thereof and the other medicaments is not specifically limited, and may be properly selected depending upon, for example, ages, body weights, the route of administration, the duration of the treatment, target disease, symptoms, the combination, etc. For example, the other medicaments may be used 0.01 - 100 percent by weight for the compound of formula (I) or a salt thereof, a solvate thereof or a prodrug thereof. The other medicaments may be administered as the combination of one or more selected from following homogeneous groups and the different kind groups by arbitrary rate.

As the other medicaments of a combination of the compound of formula (I) or formula (I-A) or a salt thereof, a solvate thereof or a prodrug thereof, there are as follows. And if it is a compound that has the action similar to the action mechanism of the medicaments, not only the one found by present but also the one that will be found in the future is included.

Examples of other medicaments for supplementing and/or enhancing the preventive and/or therapeutic effect of the compound (I) or formula (I-A) on mood disorders include antidepressant, such as a tricyclic antidepressant, a tetracyclic antidepressant, a monoamine oxidase (MAO) inhibitor, a serotonin and noradrenaline reuptake inhibitor (SNRI), a selective serotonin reuptake inhibitor (SSRI), a serotonin reuptake inhibitor; a psychoanaleptic, an antianxiety agent, an antipsychotic agent, a mitochondrial benzodiazepine receptor (MBR) ligand, an NK1 antagonist, and the like.

Examples of other medicaments for supplementing and/or enhancing the preventive and/or therapeutic effect of the compound (I) or formula (I-A) on anxiety disorders include an antianxiety agent, such as a benzodiazepine anxiolytic, a thienodiazepine anxiolytic, a non-benzodiazepine anxiolytic, a MBR ligand, and the like.

Examples of other medicaments for supplementing and/or enhancing the preventive and/or therapeutic effect of the compound (I) or formula (I-A) on irritable bowel syndrome include a gastrointestinal promotility agent, a 5-HT₃ antagonist, a 5-HT₄ agonist, an anticholinergic agent, an antidiarrheal drug, a lapactic, an autonomic modulating agent, an antidepressant, an antianxiety agent, and the like.

As an antidepressant, for example, a tricyclic antidepressant, such as amitriptyline hydrochloride, imipramine hydrochloride, clomipramine hydrochloride, dosulepin hydrochloride, nortriptyline hydrochloride, lofepramine hydrochloride, trimipramine maleate, amoxapine; a tetracyclic antidepressant, such as maprotiline hydrochloride, mianserin hydrochloride, setiptiline maleate; a MAO inhibitor, such as safrazine hydrochloride; SNRI, such as milnacipran hydrochloride, venlafaxine hydrochloride; SSRI, such as fluvoxamine maleate, paroxetine hydrochloride, fluoxetine hydrochloride, citalopram hydrochloride; a serotonin reuptake inhibitor, such as trazodone hydrochloride are given.

As an antianxiety agent, for example, a benzodiazepine anxiolytic, such as alprazolam, oxazepam, oxazolam, cloxazolam, clorazepate dipotassium, chlordiazepoxide, diazepam, tofisopam, triazolam, prazepam, fludiazepam, flutazolam, flutoprazepam, bromazepam, mexazolam, medazepam, ethyl loflazepate, lorazepam; a thienodiazepine anxiolytic, such as etizolam, clotiazepam; a non-benzodiazepine anxiolytic, such as tandospirone citrate and hydroxylzine hydrochloride are given.

As a psychoanaleptic, for example, methylphenidate hydrochloride and pemoline are given.

As an antipsychotic agent, for example, sulpiride, trazodone hydrochloride, serotonin-dopamine antagonist such as risperidone, perospirone hydrochloride hydrate, quetiapine fumarate and olanzapine are given.

As a gastrointestinal promotility agent, for example, trimebutine maleate and polycarbophil calcium are given.

As a 5-HT₃ antagonist, for example, alosetron is given.

As a 5-HT₄ agonist, for example, tegaserod, cisapride and mosapride citrate are given.

The weight ratio of the compound (I) or formula (I-A) and the other medicaments is not specifically limited.

Any combination of two or more other medicaments may be administered.

Furthermore, the other medicaments for supplementing and/or enhancing the preventive and/or therapeutic effect of the compound (I) or formula (I-A) include not only those found so far but also those which will be found on the basis of the above mentioned mechanism.

For the purpose above described, the compounds of formula (I) or formula (I-A), a non-toxic salt thereof, or a combination of the compounds of formula (I) and other medicaments may be normally administered systemically or locally, usually by oral or parenteral administration.

The doses to be administered are determined depending upon, for example, ages, body weights, symptoms, the desired therapeutic effects, the route of administration and the duration of the treatment. For the human adult, the doses per person are generally from 1 mg to 1000 mg, by oral administration, up to several times per day, and from 0.1 mg to 100 mg, by parenteral administration, up to several times per day, or continuous administration 1 to 24 hours per day from vein.

As mentioned above, the doses to be used depend upon various conditions. Therefore, there are cases in which doses lower than or greater than the ranges specified above may be used.

To administer the compounds in the present invention of formula (I), use is made of solid preparations for internal use and liquid preparations for internal use for oral administration as well as preparations for injections, external preparations, suppositories, eye drops, nasal drops, inhalations and the like for parenteral administration.

Examples of the solid preparations for internal use for oral administration include tablets, pills, capsules, powders, granules and the like. The capsules include hard capsules and soft capsules. The tablets include sublingual tablet, oral patch and orally disintegrating tablet.

Such a solid preparation for internal use is prepared by a formulation method commonly employed by using an active substances without modification, or a mixture of one or more active substances with an excipient (lactose, mannitol, glucose, nnicrocrystalline cellulose, starch, *etc.),* a binder (hydroxypropylcellulose, polyvinylpyrrolidone, magnesium metasilicate aluminate, etc.), a disintegrating agent (calcium cellulose glycolate, etc.), a lubricant (magnesium stearate, etc.), a stabilizer, a solubilizing agent (glutamic acid, aspartic acid, etc,). If necessary, it may be coated with a coating agent (sucrose, gelatin, hydroxypropylcellulose, hydroxypropylmethylcellulose phthalate, *etc.).* It may be coated with two or more layers. Moreover, capsules made of an absorbable material such as gelatin are involved in the scope thereof

The liquid preparations for internal use for oral administration include pharmaceutically acceptable aqueous solutions, suspensions, emulsions, syrups, elixirs and the like. Such a liquid preparation is prepared by dissolving, suspending or emulsifying one or more active substances in a diluent commonly employed (purified water, ethanol or a mixture thereof, *etc.).* Such liquid forms may also further comprise some additives such as humectants, suspending agents, emulsifying agents, sweetening agents, flavoring agents, aroma, preservatives, buffers and the like.

The external preparations for parenteral administration include ointments, gels, creams, fomentations, patches, liniments, atomized agents, inhalations, sprays, eye drops and nasal drops and the like. Such a preparation contains one or more active substances and is prepared by a well known method or a commonly employed formulation.

Atomized agents, inhalations and sprays may contain, in addition to a diluent commonly employed, a stabilizer such as sodium hydrogen sulfite, a buffering agent for imparting isotonicity, for example, an isotonic agent such as sodium chloride, sodium citrate or citric acid. The inhalations for parenteral administration include aerosols, powders for inhalation and liquids for inhalation. Such liquids for inhalations may be dissolved or suspended in water or another adequate medium for use. The inhalations may be prepared in accordance with a well known method. For example, liquid preparations for inhalation may be, if necessary, prepared by appropriately selecting a preservative (benzalkonium chloride, paraben, etc.), a colorant, a buffering agent (sodium phosphate, sodium acetate, etc.), an isotonic agent (sodium chloride, concentrated glycerin, *etc.),* a thickener (carboxyvinyl polymer, *etc.),* an absorption promoter, and the like.

Powders for inhalation may be prepared, if necessary, by appropriately selecting a lubricant (stearic acid and its salt, *etc.),* a binder (starch, dextrin, *etc.),* an excipient (lactose, cellulose, *etc.),* a colorant, a preservative (benzalkonium chloride, paraben, *etc.),* an absorption promoter, and the like.

When the liquids for inhalation are administered, a sprayer (atomizer, nebulizer) is usually used. When the powders for inhalation are used, an inhalation administration apparatus for powder agents is usually used.

Methods for producing a spray are described in detail in, for example, U.S. Patent No. 2,868,691 and U.S. Patent No. 3,095,355.

The injections for parenteral administration include solutions, suspensions, emulsions and solid injections to be dissolved or suspended before use. Such an injection is used by dissolving, suspending or emulsifying one or more active substances in a solvent. The solvent includes, for example, distilled water for injection, physiological saline, vegetable oils, alcohols such as propylene glycol, polyethylene glycol and ethanol, and mixtures thereof. The injection may further contain a stabilizer, a dissolution aid (glutamic acid, aspartic acid, Polysorbate 80 (registered trademark), etc.), a suspending agent, an emulsifier, a soothing agent, a buffer, a preservative, and the like. Such an injection may be produced by sterilizing at the final step or employing an aseptic process. Alternatively, it is also possible that an aseptic solid product such as a freeze-dried product is produced and sterilized or dissolved in aseptic distilled water for injection or another solvent before use.

Other compositions for parenteral administration include suppositories and pessaries for vaginal administration which contain one or more active substances, and are prepared in accordance with common formulations.

### Effect of the Invention

The compounds of the present invention possess CRF receptor binding activity and potent antagonistic activity.

### Best Mode for Carrying Out the Invention

Now, the present invention is described in greater detail by reference to the following Examples, although the present invention is not construed as being restricted thereto.

Solvents given in parentheses concerning chromatographic separation and TLC indicate each the elution solvent or the developing solvent employed and the ratio is expressed in ratio by volume.

Solvents given in parentheses concerning NMR indicate each the solvent employed in measurement.

The name of the compounds used in the present specification is designated according to ACD/Name™ (version 6.00, Advanced Chemistry Development Inc.).

### Example 1

### 5,7-dihydrofuro[3,4-d]pyrimidine-2,4(1H,3H)-dione:

To Methyl 4-oxotetrahydrofuran-3-carboxylate (18.30 g), urea (11.44 g), methanol (100 mL) and concentrated hydrochloric acid (5 mL) were added. The mixture was refluxed with heating for two hours. The obtained suspension was stirred for 15 minutes in an ice-bath. The precipitate was filtered under reduced pressure, and washed with water (20 mL x 2 times). 2 mol/L aqueous solution of sodium hydroxide (100 mL) and water (30 mL) were added to the obtained precipitate. The mixture was refluxed with heating for 1 hour. Concentrated hydrochloric acid was dropped to the reaction solution in an ice-bath. The precipitate was filtered under reduced pressure, and then the precipitate was washed with water and acetone, dried under reduced pressure to give the title compound (15.7 g) having the following physical data.
TLC: Rf 0.32 (methanol: ethyl acetate = 10 : 1);
¹H-NMR (300MHz, DMSO-d₆). δ 11.23, 11.44-11.10, 11.00, 4.70.

### Example 2

### 2,4-dichloro-5,7-dihydrofuro[3,4-d]pyrimidine:

Under argon gas atmosphere, phenylphosphonic dichloride (16.1 mL) was added to the compound prepared in Example 1 (15.7 g). The mixture was stirred for 6 hours at 135 °C, and then for 30 minutes at 165°C. After the reaction mixture was cooled, it was dropped into ice-water (100 mL). Ethyl acetate (100 mL) was added to the mixture solution. An insoluble matter was removed by filtration under reduced pressure, and was washed with ethyl acetate. The filtrate and the washings were combined, and then the mixture was shaken and separated. The organic layer was washed with a saturated sodium bicarbonate and a saturated sodium chloride, successively, dried over anhydrous sodium sulfate, and concentrated under reduced pressure, and then dried under vacuum to give the title compound (3.66 g) having the following physical data.
TLC: Rf 0.60 (hexane : ethyl acetate =1:1);
¹H-NMR (300MHz, CDCl₃): δ 5.17, 5.09.

### Example 3

### 2-chloro-4-N,N-di-n-propylamino-5,7-dihydrofuro[3,4-d]pyrimidine:

Under argon gas atmosphere, tetrahydrofuran (4 mL) was added to the compound prepared in Example 2 (757 mg), and then the mixture was stirred in an ice-bath. To the mixture, triethylamine (1.4 mL) and di-n-propylamine (1.3 mL) were dropped. The mixture was stirred for 4 hours at room temperature. The reaction mixture was poured into cooled 10% aqueous solution of citric acid, and then the mixture was extracted by ethyl acetate. The extract was washed with a saturated aqueous solution of sodium bicarbonate, and a saturated aqueous solution of sodium chloride, successively, dried over anhydrous sodium sulfate and concentrated under reduced pressure. The obtained residue was purified by column chromatography on silica gel (hexane : ethyl acetate = 9 : 1) to give the title compound (784 mg) having the following physical data.
TLC: Rf 0.71 (n-hexane : ethyl acetate =1:1);
¹H-NMR (300MHz, CDCl₃): δ 5.19, 4.86, 3.32, 1.62, 0.94.

### Example 4

### N²-(2-chloro-4-methoxyphenyl)-N⁴,N⁴-dipropyl-5,7-dihydrofura[3,4-d]pyrimidine-2,4-diamine:

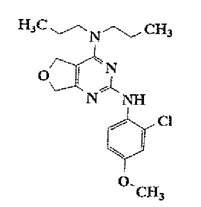

A mixture of the compound prepared in Example 3 (300 mg) and 2-chloro-4-methoxyaniline (554 mg) was reacted for 60 minutes by microwave (90 watt, 120°C). The reaction mixture was cooled to room temperature, and poured into a mixed solution of ethyl acetate / a saturated aqueous solution sodium bicarbonate and then the mixture was extracted by ethyl acetate. The extract was washed with water and a saturated aqueous solution of sodium chloride, dried over magnesium sulfate and concentrated under reduced pressure. The obtained residue was purified by column chromatography on silica gel (toluene : ethyl acetate = 20 :1 → hexane : ethyl acetate = 8 : 1 → 6 : 1) to give the title compound (385 mg) as a pale yellow powder having the following physical data.
TLC: Rf 0.29 (hexane : ethyl acetate = 4 : 1);
¹H-NMR (300MHz, CDCl₃): δ 0.93, 1.59, 3.30, 3.79, 4.82, 5.18, 6.81, 6.94, 7.03, 8.28.

### Example 4(1)-4(21)

The following compounds were obtained by the same procedure as a series of reactions of Example 4 using a corresponding compound.

### Example 4(1)

### N²-(2-chloro-4-methoxyphenyl)-N⁴,N⁴-dipropyl-6,7-dihydro-5H-cyclopenta[d]pyrimidine-2,4-diamine:

TLC: Rf 0.18 (hexane : ethyl acetate =4:1);
¹H-NMR (300MHz, CDCl₃): δ 0.92, 1.63, 2.00, 2.74, 2.97, 3.43, 3.78, 6.79, 6.93, 6.98, 8.34.

### Example 4(2)

### N²-(2-chloro-4-methoxyphenyl)-N⁴-(1-ethylpropyl)-6,7-dihydro-5H-cyclopenta[d]pyrimidine-2,4-diamine:

TLC: Rf 0.23 (hexane : ethyl acetate =2:1);
¹H-NMR (300MHz, CDCl₃): δ 0.94, 1.58, 2.08, 2.59, 2.80, 3.78, 4.02, 6.81, 6.93, 7.07, 8.46.

### Example 4(3)

### N²-(5,7-dimethyl-2,1,3-benzathiazol-4-yl)-N⁴,N⁴-dipropyl-6,7-dihydro-5H-cyclopenta[d]pyrimidine-2,4-diamine:

TLC: Rf 0.21 (hexane : ethyl acetate = 1 : 2);
¹H-NMR (300MHz, CDCl₃): δ 0.64, 1.31, 1.99, 2.41, 2.68, 2.73, 2.91, 3.14, 6.68, 7.28.

### Example 4(4)

### N²-(5,7-dimethyl-2,1,3-benzothiazol-4-yl)-N4-(1-ethylpropyl)-6,7-dihydro-5H-cyclopenta[d]pyrimidine-2,4-diamine:

TLC: Rf 0.18 (hexane : ethyl acetate = 1 : 2);
¹H-NMR (300MHz, CDCl₃): δ 0.75, 1.37, 2.06, 2.40, 2.55 2.68, 2.76, 3.68, 3.92, 6.87, 7.28.

### Example 4(5)

### N²-(2-chloro-4-methoxyphenyl)-N⁴-(1-ethylpropyl)-5,7-dihydrofuro[3,4-d]pyrimidine-2,4-diamine:

TLC: Rf 0.15 (hexane : ethyl acetate = 3 : 1);
¹H-NMR (300MHz, CDCl₃): δ 0.94, 1.58, 3.79, 4.03, 4.85, 4.98, 6.82, 6.94, 7.10, 8.33.

### Example 4(6)

### N²-(5,7-dimethyl-2,1,3-benzothiazol-4-yl)-N⁴,N⁴-dipropyl-5,7-dihydrofuro[3,4-d]pyrimidine-2,4-diamine:

TLC: Rf 0.30 (hexane : ethyl acetate = 2 : 1);
¹H-NMR (300MHz, CDCl₃): δ 0.69, 1.37, 2.41, 2.69, 3.05, 4.79, 5.14, 6.75, 7.29.

### Example 4(7)

### N²-(5,7-dimethyl-2,1,3-benzothiazol-4-yl)-N⁴-(1-ethylpropyl)-5,7-dihydrofuro[3,4-d]pyrimidine-2,4-diamine:

TLC: Rf 0.28 (hexane : ethyl acetate = 1 : 1);
¹H-NMR (300MHz, CDCl₃): δ 0.80, 1.40, 2.40, 2.69, 3.60, 4.00, 4.80, 4.94, 6.97, 7.29.

### Example 4(8)

### N⁵-(2-chloro-4-methoxyphenyl)-6-methyl-N⁷,N⁷-dipropylpyrazolo [1,5-a]pyrimidine-5, 7-diamine:

TLC: Rf 0.48 (hexane : ethyl acetate = 8 : 1);
¹H-NMR (300MHz, CDCl₃): δ 0.86, 1.48, 2.35, 3.35, 3.81, 6.23, 6.91, 6.98, 7.86, 8.62.

### Example 4(9)

### N⁵-(2-chloro-4-methoxyphenyl)-N⁷-(1-ethylpropyl)-6-methylpyrazolo[1,5-a]pyrimidine-5,7-diamine:

TLC: Rf 0.38 (hexane : ethyl acetate = 8 : 1);
¹H-NMR (300MHz, CDCl₃): δ 0.98, 1.66, 2.30, 3.72, 3.80, 5.78, 6.16, 6.74, 6.90, 6.97, 7.81,8.50.

### Example 4(10)

### N⁵-(2-chloro-4-methoxyphenyl)-6-methyl-N⁷,N⁷-dipropyl[1,2,4]triazolo[1,5-a]pyrimidine-5,7-diamine:

TLC: Rf 0.14 (hexane : ethyl acetate =2:1);
¹H-NMR (300MHz, CDCl₃): δ 0.85, 1.49, 2.33, 3.37, 3.80, 6.89, 6.97, 7.11, 8.12, 8.78.

### Example 4(11)

### N⁵-(2-chloro-4-methoxyphenyl)-N⁷-(1-ethylpropyl)pyrazolo[1,5-a]pyrimidine-5,7-diamine:

TLC: Rf0.24 (hexane : ethyl acetate = 4 : 1);
¹H-NMR (300MHz, CDCl₃): δ 0.97, 1.66, 3.29, 3.82, 5.29, 5.98, 6.13, 6.51, 6.87, 6.99, 7.83, 7.93.

### Example 4(12)

### N⁵-(2-chloro-4-methoxyphenyl)-N⁷,N⁷-dipropylpyrazolo[1,5-a]pyrimidine-5,7-diamine: TLC: Rf 0.30 (hexane : ethyl acetate = 4:1);

¹H-NMR (300MHz, CDCl₃): δ 0.90, 1.64, 3.58, 3.81, 5.36, 5.13, 6.48, 6.86, 6.99, 7.84, 7.94.

### Example 4(13)

### N⁵-(2-chloro-4-methoxyphenyl)-N⁷-(1-ethylpropyl)-6-methyl[1,2,4]triazolo[1,5-a]pyrimidine-5,7-diamine:

TLC: Rf0.21 (hexane : ethyl acetate =1:1);
¹H-NMR (300MHz, CDCl₃): δ 0.97, 1.65, 2.29, 3.79, 3.85, 5.33, 6.87, 6.95, 8.07, 8.67.

### Example 4(14)

### N⁵-(2-chloro-4-methoxyphenyl)-N⁷,N⁷-dipropyl[1,2,4]triazolo[1,5-a]pyrimidine-5,7-diamine:

TLC: Rf 0.16 (hexane : ethyl acetate = 1 : 1);
¹H-NMR (300MHz, CDCl₃): δ 0.92, 1.69, 3.64, 3.82, 5.33, 6.66, 6.86, 7.00, 7.94, 8.07.

### Example 4(15)

### N⁵-(2-chloro-4-methoxyphenyl)-N'-(1-ethylpropyl)[1,2,4]triazolo[1,5-a]pyrimidine-5,7-diamine:

TLC: Rf 0.14 (hexane : ethyl acetate =1:1);
¹H-NMR (300MHz, CDCl₃): δ 0.97, 1.67, 3.29, 3.82, 5.35, 5.71, 6.73, 6.87, 7.00, 7.97, 8.10.

### Example 4(16)

### N⁷-(2-chloro-4-methoxyphenyl)-N⁵-(1-ethylpropyl)imidazo[1,2-a]pyrimidine-5,7-diamine: TLC: Rf0.15 (ethyl acetate : methanol = 10 : 1);

¹H-NMR (300MHz, CDCl₃): δ 0.97, 1.70, 3.27, 3.81, 4.42, 5.33, 6.72, 6.84, 6.97, 7.13, 7.43, 8.17.

### Example 4(17)

### N⁷-(2-chloro-4-methoxyphenyl)-N⁵,N⁵-dipropylimidazo[1,2-a]pyrimidine-5,7-diamine: TLC: Rf 0.50 (ethyl acetate : methanol = 10 : 1);

¹H-NMR (300MHz, CDCl₃): δ 0.91, 1.64, 3.18, 3.81, 5.65, 6.73, 6.86, 6.97, 7.21, 7.42, 8.32.

### Example 4(18)

### N⁷-(2-chloro-4-methoxyphenyl)-N⁵-(1-ethylpropyl)-6-methylimidazo[1,2-a]pyrimidine-5,7-diamine:

TLC: Rf0.32 (ethyl acetate : methanol = 9 : 1);
¹H-NMR (300MHz, CDCl₃): δ 0.98, 1.58, 2.23, 3.51, 3.65, 3.78,6.86, 6.92, 6.94, 7.26, 7.40, 8.87.

### Example 4(19)

### N⁷-(2-chloro-4-methoxyphenyl)-6-methyl-N⁵,N⁵-dipropylimidazo[1,2-a]pyrimidine-5,7-diamine:

TLC: Rf 0.42 (methylene chloride : methanol =9:1);
¹H-NMR (300MHz, CDCl₃): δ 0.87, 1.52, 2.29, 3.18, 3.81, 6.90, 6.96, 7.03, 7.27, 7.40, 8.95.

### Example 4(20)

### N²-(2,4-dichlorophenyl)-3-methyl-N⁴,N⁴-dipropyl-6,7-dihydro-5H-cyclopenta[b]pyridine-2,4-diamine:

TLC: Rf0.57 (hexane : ethyl acetate = 9 : 1);
¹H-NMR (300MHz, CDCl₃: δ 8.35, 7.34, 7.17, 6.70, 3.00, 2.92-2.83, 2.20, 2.06, 1.43, 0.84.

### Example 4(21)

### N²-(2,4-diehlorophenyl)-N⁴,N⁴-diprapyl-6,7-dihydro-5H-cyclopenta[d]pyrimidine-2,4-diamine:

TLC: Rf0.15 (benzene : ethyl acetate =9:1);
¹H-NMR (300MHz, CDCl₃): δ 8.56, 7.34, 7.23, 7.17, 3.46, 2.98, 2.76, 2.01, 1.72-1.57, 0.94.

### Example 5

### N²-(2-chloro-4-methoxyphenyl)-N²-methyl-N⁴,N⁴-dipropyl-5,7-dihydrofuro[3,4-d]pyrimidine-2,4-diamine:

Under argon gas atmosphere, to a solution of the compound prepared in Example 4 (225 mg) in N, N-dimethylformamide (6 mL), sodium hydride (60% in oil suspension, 36 mg) and methyl iodide (0.075 mL), successively, were added in an ice-bath. The mixture was stirred for 1 hour at room temperature. To the reaction mixture, a saturated aqueous solution of ammonium chloride was added, and then the mixture was extracted with hexane / ethyl acetate (1/1). The organic layer was washed with water and a saturated aqueous solution of sodium chloride, successively, dried over magnesium sulfate and concentrated under reduced pressure. The obtained residue was purified by column chromatography on silica gel (hexane : ethyl acetate = 8 : 1 → 4 : 1) to give the title compound (224 mg) as a pale yellow powder having the following physical data.
TLC: Rf 0.30 (hexane; ethyl acetate = 4 : 1);
¹H-NMR (300MHz, CDCl₃): δ 0.74, 1.42, 3.04, 3.37, 3.81, 4.80, 5.13, 6.82, 6.99, 7.18.

### Example 5(1)-5(9)

The following compounds were obtained by the same procedure as a series of reactions of Example 5 using a compound prepared in Example 4(1), 4(3), 4(7), 4(8), 4(10), 4(12), 4(14), 4(17) or 4(21).

### Example 5(1)

### N²-(2-chloro-4-methoxyphenyl)-N²-methyl-N⁴, N⁴-dipropyl-6,7-dihydro-5H-cyclopenta[d]pyrimidine-2,4-diamine:

TLC: Rf 0.20 (toluene : ethyl acetate = 3 : 1);
¹H-NMR (300MHz, CDCl₃): δ 0.70, 1.40, 1.95, 2.75, 2.90, 3.14, 3.37, 3.80, 6.80, 6.98, 7.18.

### Example 5(2)

### N²-(5,7-dimethyl-2,1,3-benzothiazol-4-yl)-N²-methyl-N⁴,N⁴-dipropyl-6,7-dihydro-5H-cyclopenta[d]pyrimidine-2,4-diamine:

TLC: Rf0.49 (hexane : ethyl acetate =1:2),
¹H-NMR (300MHz, DMSO-d₆): δ 0.56, 1.21, 1.89, 2.25, 2.58, 2.64, 2.87, 3.07, 3.40, 7.41.

### Example 5(3)

### N²-(5,7-dimethyl-2,1,3-benzothiazol-4-yl)-N²-methyl-N⁴,N⁴-dipropyl-5,7-dihydrofuro[3,4-d]pyrimidine-2,4-diamine:

TLC: Rf 0.48 (hexane : ethyl acetate = 2 : 1);
¹H-NMR (300MHz, DMSO-d₆): δ 0.58, 1.24, 2.27, 2.65, 2.96, 3.42, 4.60, 5.03, 7.42.

### Example 5(4)

### N⁵-(2-chloro-4-methoxyphenyl)-N⁵,6-dimethyl-N⁷,N⁷-dipropylpyrazolo[1,5-a]pyrimidine-5,7-diamine:

TLC: Rf0.40 (hexane : ethyl acetate = 4:1);
¹H-NMR (300MHz, CDCl₃): δ 0.78, 1.40, 1.55, 3.31, 3.81, 6.32, 6.71, 6.82, 7.03, 7.90.

### Example 5(5)

### N⁵-(2-chloro-4-methoxyphenyl)-N⁵,6-dimethyl-N⁷,N⁷-dipropyl[1,2,4]triazolo[1,5-a]pyrimidine-5,7-diamine:

TLC: Rf 0.30 (hexane : ethyl acetate =1:1);
¹H-NMR (300MHz, CDCl₃): δ 0.77, 1.41, 1.51, 3.30, 3.37, 3.81, 6.73, 6.86, 7.02, 8.16.

### Example 5(6)

### N⁵-(2-chloro-4-methoxyphenyl)-N⁵-methyl-N⁷,N⁷-dipropylpyrazolo[1,5-a]pyrimidine-5,7-diamine:

TLC: Rf 0.30 (hexane : ethyl acetate = 4 : 1);
¹H-NMR (300MHz, CDCl₃): δ 0.77, 1.53, 3.41, 3.85, 4.78, 6.15, 6.89, 7.06, 7.23, 7.8 1

### Example 5(7)

### N⁵-(2-chloro-4-methoxyphenyl)-N⁵-methyl-N⁷,N⁷-dipropyl[1,2,4]triazolo[1,5-a]pyrimidine-5,7-diamine:

TLC: Rf 0.28 (toluene : ethyl acetate = 1 : 1);
¹H-NMR (300MHz, CDCl₃): δ 0.78, 1.55, 3.45, 3.85, 4.75, 6.90, 7.07, 7.23, 8.04.

### Example 5(8)

### N⁷-(2-chloro-4-methoxyphenyl)-N⁷-methyl-N⁵,N⁵-dipropylimidazo[1,2-a]pyrimidine-5,7-diamine:

TLC: Rf 0.36 (ethyl acetate : methanol = 10 : 1);
¹H-NMR (300MHz, CDCl₃): δ 0.80, 1.48, 2.97, 3.45, 3.86, 5.09, 6.89, 7.06, 7.12, 7.2 1, 7.37.

### Example 5(9)

### N²-(2,4-dichlorophenyl)-N²-methyl-N⁴,N⁴-dipropyl-6,7-dihydro-5H-eyclopenta[d]pyrimidine-2,4-diamine:

TLC: Rf 0.26 (hexane : ethyl acetate = 4 : 1);
¹H-NMR (300MHz, CDCl₃). δ 7.44, 7.26-7.19, 3.38, 3.14, 2.90, 2.75, 1.96, 1.41, 0.72.

### Example 6

### N²-(2-chloro-4-methoxyphenyl)-N⁴-(-ethylpropyl)-N²-methyl-6,7-dihydro-5H-cyclopenta[d]pyrimidine-2,4-diamine:

The mixture was 2-chloro-4-methoxy-N-methylaniline (150 mg) and 2-chloro-4-(1-ethylpropylamino)-6,7-dihydro-5H-cyclopenta[d]pyrimidine (322 mg) was heated for 9 hours at 120°C, and then for 6 hours at 160°C. The reaction mixture was cooled to room temperature, a crude product was purified by column chromatography on silica gel (toluene : ethyl acetate = 1 : 1 → 1 : 3) to give the title compound (147 mg) as dark brown oil having the following physical data.
TLC: Rf 0:21 (hexane : ethyl acetate = 1:1);
¹H-NMR (300MHz, CDCl₃): δ 0.76, 1.37, 2.04, 2.52, 2.77, 3.37, 3.56, 3.81, 6.81, 6.98, 7.18.

### Example 6(1)

### N⁵-(2-chloro-4-methoxyphenyl)-N⁷-(1-ethylpropyl)-N⁵-methyl[1,2,4]triazolo[1,5-a]pyrimidine-5,7-diamine:

The title compound having the following physical data was obtained by the same procedure as a series of reactions of Example 6 using a corresponding compound.
TLC: Rf 0.23 (toluene : ethyl acetate = 1: 1);
¹H-NMR (300NMz, CDCl₃): δ 0.85, 1.51, 3.00, 3.4b, 3.87, 4.78, 5.50, 6.91, 7.09, 7.24, 8.07.

### Example 7

### 2-(methylthio)-N,N-dipropylpyrazolo[1,5-a] [1,3,5]triazine-4-amine:

To a solution of 4-chloro-2-(methylthio)pyrazolo[1,5-a][1,3,5]triazine (1.0 g) in tetrahydrofuran (5.0 mL), triethylamine (1.0 mL) and di-n-propylamine (1.0 mL) were added at 0°C. The mixture was stirred overnight at room temperature. The reaction solution was filtered through celite, concentrated under reduced pressure. The residue was purified by column chromatography on silica gel (hexane : ethyl acetate = 10 : 1) to give the title compound (1.1 g) as a white powder having the following physical data.
TLC: Rf 0.85 (hexane : ethyl acetate = 3 : 1);
¹H-NMR (300MHz, CDCl₃): δ 0.95, 1.75, 2.52, 3.94, 6.13, 7.83.

### Example 8

### 2-(methylsulfonyl)-N,N-dipropylpyrazolo[1,5-a][1,3,5]triazine-4-amine:

To a solution of the compound prepared in Example 7 (660 mg) in methylene chloride (11 mL), m-chloroperbenzoic acid (1.1 g) was added, and the mixture was stirred for 4 hours at room temperature. To the reaction mixture, an aqueous solution of sodium sulfite was added. The mixture was extracted with methylene chloride. The organic layer was washed with 2N aqueous solution of sodium hydroxide and a saturated aqueous solution of sodium chloride, dried over anhydrous magnesium sulfate and concentrated under reduced pressure to give the title compound (590 mg) as a white powder having the following physical data.
TLC: Rf0.62 (hexane : ethyl acetate =1:1);
¹H-NMR (300MHz, CDCl₃): δ 0.98, 1.80, 3.27, 3.74, 4.30, 6.54, 8.02,

### Example 9

### N²-(2-chloro-4-methoxyphenyl)-N⁴,N⁴-dipropylpyrazolo[1,5-a][1,3,5]triazine-2,4-diamine:

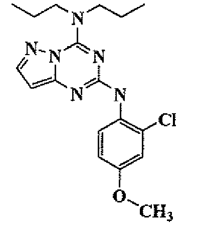

The title compound (70 mg) as a white powder having the following physical data was obtained by the same procedure as a series of reactions of Example 6 using a compound prepared in Example 8 (250 mg) and 2-chloro-4-methoxyaniline (280 mg).
TLC: Rf 0.68 (hexane : ethyl acetate = 3 : 1);
¹H-NMR (300MHz, CDCb): δ 0.94, 1.74, 3.78, 3.89, 5.96, 6.83, 6.90, 6.94, 7.77, 8.22.

### Example 10

### N²-(2-chloro-4-methoxyphenyl)-N²-methyl-N⁴,N⁴-dipropylpyrazolo[1,5-a][1,3,5]triazine-2,4-diamine:

The title compound (41 mg) as a white powder having the following physical data was obtained by the same procedure as a series of reactions of Example 5 using a compound prepared in Example 9 (42 mg).
TLC: Rf 0.67 (hexane : ethyl acetate = 3 : 1);
¹H-NMR (300MHz, CDCl₃): δ 0.75, 1.53, 3.57, 5.96, 6.81, 6.98, 7.17, 7.71.

### Example 11

### 2,6-dichloro-5-nitro-N,N-dipropylpyrimidine-4-amine:

To a solution of 2,4,6-trichloro-5-nitropyrimidine (1.7 g) in tetrahydrofuran (20 mL), di-n-propylamine (1.03 mL) and triethylamine (1.04 mL) were dropped at 0°C. The mixture was stirred for 2 hours. The reaction mixture was diluted with ethyl acetate. The diluted solution was washed with water and a saturated aqueous solution of sodium chloride, dried over anhydrous magnesium sulfate and concentrated under reduced pressure to give the title compound having the following physical data.
TLC: Rf 0.50 (hexane : ethyl acetate = 20 : 1);
¹H-NMR (300MHz, CDCl₃): δ 3.36, 1.61, 0.91.

### Example 12

### 2-chloro-5-nitro-N,N-dipropylpyrimidine-4,6-diamine:

To a solution of the compound prepared in Example 11 (2.26 g) in ethanol (15 mL), a solution of ammonia in ethanol (7 mL) was added, and then the mixture was stirred for 2 hours at room temperature. The reaction mixture was diluted with ethyl acetate. The diluted solution was washed with water and a saturated aqueous solution of sodium chloride, dried over anhydrous sodium sulfate and concentrated under reduced pressure. The residue was purified by column chromatography on silica gel (hexane : ethyl acetate = 8 : 1) to give the title compound (534 mg) as a yellow crystal having the following physical data.
TLC: Rf 0.24 (hexane : ethyl acetate =9:1);
¹H-NMR (300MHz, CDCl₃) : δ 3.37, 1.62, 0.90.

### Example 13

### N²-(2-chloro-4-methoxyphenyl)-N⁴,N ⁴-dipropyl-N²-methyl-5-nitropyrimidine-2,4,6-triamine;

The title compound (691 mg) as pale yellow oil having the following physical data was obtained by the same procedure as a series of reactions of Example 6 using a compound prepared in Example 12 (534 mg) and N-(2-chloro-4-methoxyphenyl)-N-methylamine (1.3 g).
TLC: Rf0.33 (hexane : ethyl acetate =4:1).

### Example 14

### N²-(2-chlora-4-methoxyphenyl)-N⁴,N⁴-dipropyl-N²-methylpyrimidine-2,4,5,6-tetraamine:

To a solution of the compound prepared in Example 13 (440 mg) in ethanol (12 mL), water (10 mL) was added, and then sodium dithionite (1.5 g) was added at 50°C. The mixture was stirred for 30 minutes. After the reaction mixture was cooled, it was diluted with ethyl acetate. The diluted solution was washed with water and a saturated aqueous solution of sodium chloride, dried over magnesium sulfate and concentrated under reduced pressure to give the title compound (443 mg) as a brown solid having the following physical data.
TLC: Rf 0.33 (hexane : ethyl acetate = 1 : 1);
¹H-NMR (300MHz, CDCl₃): δ 7.16, 6.99, 6.84, 6.01, 3.81, 3.40, 3.23, 1.47, 0.75.

### Example 15

### N⁵-(2-chlaro-4-methoxyphenyl)-N⁵-methyl-N⁷,N⁷-diprapyl[1,2,5]thiadiazol[3,4-d]pyrimidine-5,7-diamine:

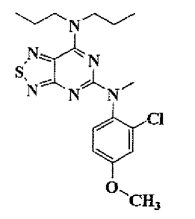

Thionyl chloride (5.0 mL) was added to the compound prepared in Example 14 (437 mg), and the mixture was refluxed for 2.5 hours. After the reaction mixture was cooled, it was poured into an ice-water. The solution was basified by adding a saturated aqueous solution of sodium bicarbonate and extracted with ethyl acetate. The organic layer was washed with a saturated aqueous solution of sodium bicarbonate and a saturated aqueous solution of sodium chloride, dried over anhydrous magnesium sulfate and concentrated under reduced pressure. The residue was purified by column chromatography on silica gel (hexane : ethyl acetate = 5:1) to give the title compound (344 mg) as a pale yellow solid having the following physical data.
TLC: Rf 0.45 (hexane: ethyl acetate = 4 : 1);
¹H-NMR (300MHz, DMSO-d₆): δ 0.77, 1.55, 3.74, 6.36, 7.10, 7.30.

### Example 16

### N²-mesityl-N⁴,N⁴-dipropyl-6,7-dihydro-5H-cyclopenta[d]pyrimidine-2,4-diamine:

The title compound having the following physical data was obtained by the same procedure as a series of reactions of Example 3 (using a compound prepared in a series of reactions of Example 1 → Example 2 using a corresponding compound) → Example 4 (using a corresponding compound).
TLC: Rf 0.34 (ethyl acetate : methanol = 100 : 1);
¹H-NMR (300MHz, CDCl₃): δ 0.68, 1.39, 1.98, 2.19, 2.26, 2.71, 2.91, 3.15, 5.85, 6.87.

### Example 17

### 2-[(2-chloro-4-methoxyphenyl)amino]-4-(dipropylamino)-6,7-dihydro-5H-cyclopenta[b]pyridine-3-carbonitrile:

The title compound having the following physical data was obtained by the same procedure as a series of reactions of Example 3 (using a compound prepared in a series of reactions of Example 1 → Example 2 using a corresponding compound) → Example 4.
TLC: Rf 0.68 (hexane : ethyl acetate =4:1);
¹H-NMR (300MHz, CDCl₃). δ 0.89, 1.64, 1.78, 2.00, 2.66, 3.51, 3.77, 6.25, 6.73, 6.92, 6.99.

### Example 18

### N²-(2-chloro-4-methoxyphenyl)-3-methyl-N⁴,N⁴-dipropyl-6,7-dihydro-5H-cyclopenta[b]pyridine-2,4-diamine:

The title compound having the following physical data was obtained by the same procedure as a series of reactions of Example 3 (using a compound prepared in a series of reactions of Example 1 → Example 2 using a corresponding compound) → Example 4.
TLC: Rf 0.61 (hexane : ethyl acetate = 5 : 1);
¹H-NMR (300MHz, CDCl₃): δ 8.28, 6.94, 6.82, 6.48, 3.77, 2.93-3.04, 2.80-2.92, 2.20, 1.97-2.12, 1.34-1.52, 0.84.

### Example 19

### 6-methylpyrazolo[1,5-a]pyrimidin-5,7-diol:

Under argon gas atmosphere, sodium (6.92 g) was added to ethanol (250 mL) by little and little, and the ethanol solution was stirred until sodium was solved completely at room temperature. A solution of 3-aminopyrazole (25.0 g) in ethanol (20 mL) and diethyl methylmalonate (52 mL) were dropped, successively, to the above solution. The mixture was refluxed at 90°C. After the reaction mixture was cooled to room temperature, the mixture was filtrated under vacuum. The solid was solved to cooled 5N hydrochloric acid (70 mL). The precipitate was collected by filtration and dried under vacuum to give the title compound (36.7 g).

### Example 20

### 5,7-dichloro-6-methylpyrazolo[1,5-a]pyrimidine:

Under argon gas atmosphere, phosphoryl chloride (102 g) and N,N-diethylaniline (8.4 g), successively, was dropped into the compound prepared in Example 19. The mixture was refluxed for 4 hours at 150°C. Besides, N,N-diethylaniline (8.4 g) was dropped into the above mixture, and the mixture was refluxed at same temperature. After the reaction mixture was cooled to room temperature, it was concentrated under reduced pressure. The residue was solved into an ice-water. An aqueous solution of sodium bicarbonate was added to the solution. The mixture was neutralized and then filtrated through celite. The filtrate was extracted twice with ethyl acetate. The organic layer was water and normal saline solution, successively, dried over magnesium sulfate, filtrated and concentrated under reduced pressure. The obtained residue was purified by column chromatography on silica gel (hexane : ethyl acetate ==12:1, 10:1,8: 1,6:1) to give the title compound (22.2 g) having the following physical data.
TLC: Rf0.46 (hexane : ethyl acetate = 4 : 1);
¹H-NMR (300MHz, CDCl₃): δ 2.56, 6.70, 8.16.

### Example 21

### N⁵-(4-methoxy-2-methylphenyl)-6-methyl-N⁷,N⁷-dipropylpyrazolo[1,5-a]pyrimidine-5,7-diamine:

The title compound having the following physical data was obtained by the same procedure as a series of reactions of Example 3 using 5,7-dichloro-6-methylpyrazolo[1,5-a]pyrimidine → corresponding Example 4.
TLC: Rf 0.29 (hexane : ethyl acetate = 5 : 1);
¹H-NMR (300MHz, CDCl₃): δ 0.87, 1.49, 2.28, 2.29, 3.34, 3.81, 6.04, 6.14, 6.81, 7.70, 7.81.

### Example 21(1)-21(89)

The following compounds were obtained by the same procedure as a series of reactions of Example 19 → Example 20 → Example 3 → Example 4, using a corresponding compound.

### Example 21(1)

### N⁵-(2-chloro-4-methoxyphenyl)-6-methyl-N⁷,N⁷-dipropylpyrazolo[1,5-a]pyrimidine-5,7-diamine:

TLC: Rf 0.18 (hexane : ethyl acetate = 10 : 1);
¹H-NMR (300MHz, CDCl₃): δ 0.86, 1.42, 1.50, 2.34, 3.34, 4.03, 6.23, 6.90, 6.98, 7.86, 8.61.

### Example 21(2)

### N⁵-mesityl-6-methyl-N⁷,N⁷-dipropylpyrazolo[1,5-a]pyrimidine-5,7-diamine:

TLC: Rf 0.52 (hexane : ethyl acetate = 5:1);
¹H-NMR (300MHz, CDCl₃): δ 0.88, 1.51, 2.20, 2.31, 2.32, 3.34, 5.76, 6.07, 6.95, 7.77

### Example 21(3)

### N⁵-(2,4-dimethylphenyl)-6-methyl-N⁷,N⁷-dipropylpyrazolo[1,5-a]pyrimidine-5,7-diamine: TLC: Rf 0.50 (hexane : ethyl acetate =5:1);

¹H-NMR (300MHz, CDCl₃): δ 0.87, 1.40-1.58, 2.28, 2.30, 2.32, 3.35, 6.10- 6.20, 7.01-7.12, 7.77-7.88.

### Example 21(4)

### N^{S}-(2-fluoro-4-methylphenyl)-6-methyl-N',N'-dipropylpyrazolo[1,5-a]pyrimidine-5,7-diamine:

TLC: Rf 0.64 (hexane : ethyl acetate =5:1);
¹H-NMR (300MHz, CDCl₃): δ 0.86, 1.32-1.64, 2.32, 2.33-2.36, 3.36, 6.25, 6.63, 6.83-7.09, 7.87, 8.52.

### Example 21(5)

### N⁵-(2-fluoro-4-methylphenyl)-6-methyl-N⁷,N⁷-dipropylpyrazolo[1,5-a]pyrimidine-5,7-diamine:

TLC: Rf 0.68 (hexane : ethyl acetate = 5:1);
¹H-NMR (300MHz, CDCl₃): δ 0.87, 1.23-1.77, 2.33, 2.36, 3.36, 6.26, 7.08, 7.14, 7.22, 7.88, 8.67.

### Example 21(6)

### 3-chloro-4-{[7-(dipropylamino)-6-methylpyrazolo[1,5-a]pyrimidin-5-yl]amino}benzonitrile:

TLC: Rf 0.53 (hexane : ethyl acetate = 5 : 1);
¹H-NMR (300MHz, CDCl₃): δ 0.86, 1.30-1.70, 2.38, 3.09-3.64, 6.36, 7.47, 7.62, 7.69, 7.94, 9.15.

### Example 21(7)

### N⁵-(2,4-dimethoxyphenyl)-6-methyl-N⁷,N⁷-dipropylpyrazolo[1,5-a]pyrimidine-5,7-diamine:

TLC: Rf 0.43 (hexane : ethyl acetate = 5:1);
¹H-NMR (300MHz, CDCl₃): δ 0.86, 1.35-1.63, 2.31, 3.18-3.50, 3.83, 3.91, 6.21, 6.48-6.65, 7.06, 7.84, 8.64.

### Example 21(8)

### N5-(4-fluoro-2-methylphenyl)-6-methyl-N⁷,N⁷-dipropylpyrazolo[1,5-a]pyrimidine-5,7-diamine:

TLC: Rf0.39 (hexane : ethyl acetate =5:1);
¹H-NMR (300MHz, CDCl₃): δ 7.89, 7.83, 6.96, 6.16, 6.09, 3.35, 2.30, 1.48, 0.87.

### Example 21(9)

### 4-{ [7-(diprapylamino)-6-methylpyrazolo[1, 5-a]pyrimidin-5-yl]amino}-3-methylbenzonitrile:

TLC: Rf0.68 (hexane : ethyl acetate = 2 : 1);
¹H-NMR (300MHz, CDCl₃): δ 8.70, 7.92, 7.58, 7.49, 6.53, 6.32, 3.39, 2.38, 2.35, 1.49, 0.87.

### Example 21(10)

### N⁵-(4-chloro-2-methylphenyl)-6-methyl-N⁷,N⁷-dipropylpyrazolo[1,5-a]pyrimidine-5,7-diamine:

TLC: Rf 0.46 (hexane : ethyl acetate = 5 : 1);
¹H-NMR. (300MHz, CDCl₃): δ 8.09, 7.85, 7.12-7.32, 6.20, 6.18, 3.26-3.45,2.31, 1.40-1.60, 0.87.

### Example 21(11)

### N⁵-(4-chloro-2-fluorophenyl)-6-methyl-N⁷,N⁷-dipropylpyrazolo[1,5-a]pyrimidine-5,7-diamine:

TLC: Rf 0.62 (toluene : ethyl acetate = 9 : 1);
¹H-NMR (300MHz, CDCl₃): δ 8.73, 7.89, 7.09-7.23, 6.68, 6.27, 3,25-3 -45, 2.32, 1.38-1.57, 0.86.

### Example 21(12)

### N⁵-[2-fluoro-4-(trifluoromethyl)phenyl]-6-methyl-N⁷,N⁷-dipropylpyrazolo[1,5-a]pyrimidine-5,7-diamine:

TLC: Rf0.66 (toluene : ethyl acetate = 9 : 1);
¹H-NMR (300MHz, CDCl₃): δ 8.98, 7.92, 7.47, 7.38, 6.90, 6.32, 3.21-3.55, 2.35, 1.38-1.60,0.86.

### Example 21(13)

### N⁵-(2,4-difluorophenyl)-6-methyl-N⁷,N⁷-dipropylpyrazolo[1,5-a]pyrimidine-5,7-diamine: TLC: Rf 0.54 (hexane : ethyl acetate = 5 : 1);

¹H-NMR (300MHz, CDCl₃): δ 8.58-8.71, 7.88, 6.83-7.02, 6.56, 6.25, 3.36, 2.32, 1.39-1.60, 0.86.

### Example 21(14)

### N⁵-[2-chloro-4-(trifluoromethyl)phenyl]-6- methyl-N⁷,N⁷-dipropylpyrazolo[1,5-a]pyrimidine-5,7-diamine:

TLC: Rf 0.64 (hexane : ethyl acetate = 5 : 1);
¹H-N-NM (300MHz, CDCl₃): δ 9.08, 7.93, 7.67, 7.59, 7.37, 6.33, 3,26-3,50, 2.39, 1.39-1.59,0.87.

### Example 21(15)

### 4-{[7-(dipropylamino)-6-methylpyrazolo[1,5-a]pyrimidin-5-yl]amino}-3-ethylbenzonitrile; TLC: Rf 0.29 (hexane : ethyl acetate =5:1);

¹H-NMR (300MHz, CDCl₃): δ 8.66, 7.91, 7.57, 7.50, 6.62, 6.30, 3.27-3.49, 2.71, 2.34, 1.42-1.61, 1.36, 0.87.

### Example 21(16)

### N⁵-(2-chloro-4-fluorophenyl)-6-methyl-N⁷,N⁷-dipropylpyrazalo[1,5-a]pyrimidine-5,7-diamine:

TLC: Rf0.59 (hexane : ethyl acetate = 5 : 1);
¹H-NMR (300MHz, CDCl₃): δ 8.81, 7.89, 7.17, 7.04-7.13, 7.02, 6.26, 3.37, 2.36, 1.41-1.58, 0.87.

### Example 21(17)

### N⁵-(2,4-dichlorophenyl)-6-methyl-N⁷,N⁷-dipropylpyrazolo[1,5-a]pyrimidine-5,7-diamine: TLC: Rf 0.65 (hexane : ethyl acetate =5:1);

¹H-NMR (300MHz, CDCl₃) : δ 8.86, 7.90, 7.41, 7.31, 7.14, b.29, 3.37, 2.36, 1.38-1.61, 0.86.

### Example 21(18)

### N⁵-(4-chloro-2,5-dimethoxyphenyl)-6-methyl-N⁷,N⁷-dipropylpyrazolo[1,5-a]pyrimidine-5,7-diamine:

TLC: Rf0.48 (hexane : ethyl acetate = 5 : 1);
¹H-NMR (300MHz, CDCl₃): δ 8.81, 7.88, 7.31, 6.91, 6.23, 3.97, 3.91, 3.35, 2.32, 1.38-1.55, 0.86.

### Example 21 (19)

### N⁵-[4-fluoro-2-(trifluoromethyl)phenyl]-6-methyl-N⁷,N⁷-dipropylpyrazolo[1,5-a]pyrimidine-5,7-diamine:

TLC: Rf 0.51 (hexane : ethyl acetate = 5 : 1);
¹H-NMR (300MHz, CDCl₃): δ 8,52, 7.88, 7.19-7.44, 6.79, 6.22, 3.22-3,48, 2.29, 1.39-1.62, 0.87.

### Example 21(20)

### N⁵-[4-chloro-2-(trifluoromethyl)phenyl]-6-methyl-N⁷,N⁷-dipropylpyrazolo[1,5-a]pyrimidine-5,7-diamine:

TLC: Rf 0.56 (hexane : ethyl acetate = 5 : 1);
¹H-NMR (300MHz, CDCl₃): δ 8.64, 7.89, 7.59, 7.54, 6.91, 6.25, 3.29-3.43, 2.29, 1.33-1.60, 0.87.

### Example 21(21)

### N⁵-(2-chloro-4,6-dimethylphenyl)-6-methyl-N⁷,N⁷-dipropylpyrazolo[1,5-a]pyrimidine-5,7-diamine:

TLC: Rf 0.40 (hexane : ethyl acetate = 5 : 1);
¹H-NMR (300MHz, CDCl₃): δ 7.80, 7.13, 7.02, 6.10, 6.07, 3.30-3.41, 2.35, 2.32, 2.24, 1.38-1.63, 0.88.

### Example 21(22)

### 6-methyl-N⁵-[2-methyl-4-(trifluoromethoxy)phenyl]-N⁷,N⁷-dipropylpyrazolo[1,5-a]pyrimidine-5,7-diamine:

TLC: Rf 0.45 (hexane : ethyl acetate = 5 : 1);
¹H-NMR (300MHz, CDCl₃): δ 8.19, 7.86, 7.05-7.18, 6.15-6.27, 3.25-3,46, 2.34, 2.32, 1.40-1.59,0.87.

### Example 21(23)

### 6-methyl-N⁷,N⁷-dipropyl-N⁵-(2,4,5-trimethylphenyl)pyrazolo[1,5-a]pyrimidine-5,7-diamine

TLC: Rf0.45 (hexane : ethyl acetate =5:1);
¹H-NMR (300MHz, CDCl₃): δ 7.82, 7.61, 7.00, 6.17, 6.09, 3.35, 2.28, 2.26, 2.24, 2.23, 1.41-1.57, 0.87.

### Example 21(24)

### N⁵-(5-chloro-2,4-dimethoxyphenyl)-6-methyl-N⁷,N⁷-dipropylpyrazolo[1,5-a]pyrimidine-5,7-diamine

TLC: Rf 0.37(hexane : ethyl acetate = 2 : 1);
¹H-NMR (300MHz, CDCl₃): δ 8.85, 7.86, 7.05, 6.58, 6.28, 3.96, 3.91, 3.34, 2.30, 1.37-1.55, 0.86.

### Example 21(25)

### N⁵-(4,5-dimethoxy-2-methylphenyl)-6-methyl-N⁷,N⁷-dipropylpyrazolo[1,5-a]pyrimidine-5,7-diamine:

TLC: Rf 0.26 (hexane : ethyl acetate = 2 : 1);
¹H-NMR (300MHz, CDCl₃): δ 7.83, 7.53, 6.74, 6.15, 6.08, 3.89, 3.88, 3.35, 2.30, 2.25, 1.42-1.56,0.87.

### Example 21(26)

### N⁵-(6-methoxy-2,3-dihydro-1H-inden-5-yl)-6-methyl-N⁷,N⁷-dipropylpyrazolo[1,5-a]pyrimidine-5,7-diamine:

TLC: Rf 0.51 (hexane : ethyl acetate = 5 : 1);
¹H-NMR (300MHz, CDCl₃): δ 8.58, 7.85, 6.81, 6.25, 3.91, 3.21-3.47, 2.95, 2.89, 2.32, 2.01-2.15, 1.39-1.55, 0.86.

### Example 21 (27)

### N⁵-(2,6-dimethyl-3-pyridinyl)-6-methyl-N⁷,N⁷-dipropylpyrazolo[1,5-a]pyrimidine-5,7-diamine:

TLC: Rf0.26 (hexane : ethyl acetate =1:3);
¹H-NMR (300MHz, CDCl₃): δ 8.34, 7.86, 7.07, 6.19, 6.16, 3.25-3.47, 2.55, 2.53, 2.33, 1.40-1.58, 0.87.

### Example 21 (28)

### N⁵-(2,6-dimethylphenyl)-6-methyl-N⁷,N⁷-dipropylpyrazolo[1,5-a]pyrimidine-5,7-diamine: TLC: Rf 0.40 (hexane : ethyl acetate = 5 : 1);

¹H-NMR (300MHz, CDCl₃): δ 7.78, 7.13, 6.06, 5.81, 3.21-3.46, 2.33, 2.24, 1.41-1.62, 0.89.

### Example 21(29)

### N⁵-(4-chloro-2-methoxy-5-methylphenyl)-6-methyl-N⁷,N⁷-dipropylpyrazolo[1,5-a]pyrimidine-5,7-diamine:

TLC: Rf 0.42 (hexane : ethyl acetate = 5 : 1);
¹H-NMR (300MHz, CDCl₃): δ 8.68, 7.87, 7.19, 6.88, 6.28, 3.92, 3.35, 2.39, 2.31, 1.34-1.59, 0.86.

### Example 21 (30)

### Methyl 3-chloro-4-{[7-(dipropylamino)-6-methylpyrazolo[1,5-a]pyrimidin-5-yl]amino}benzoate:

TLC: Rf0.37 (hexane : ethyl acetate=5:1);
¹H-NMR (300MHz, CDCl₃): δ 9.04, 8.10, 8.02, 7.93, 7.47, 6.35, 3.92, 3.29-3.46, 2.39, 1.40-1.61,0.86.

### Example 21 (31)

### 6-methyl-N⁵-(4-methyl-2-vinylphenyl)-N⁷,N⁷-dipropylpyrazolo[1,5-a]pyrimidine-5,7-diamine

TLC: Rf0.41 (hexane : ethyl acetate = 5 : 1);
¹H-NMR (300MHz, CDCl₃): δ 7.88, 7.83, 7.28, 7.14, -6.87, 6:30; 6.17, 5.70, 5.38, 3.35, 2.36, 2.28, 1.39-1.61, 0.87.

### Example 21(32)

### N⁵-[4-chloro-2-(trifluoromethoxy)phenyl]-6-methyl-N⁷,N⁷-dipropylpyrazolo[1,5-a]pyrimidine-5,7-diamine:

TLC: Rf0.73 (toluene : ethyl acetate = 5 : 1);
¹H-NMR (300MHz, CDCl₃): δ 8.87, 7.89, 7.26-7.36, 6.89, 6.28, 3.27-3.45, 2.31, 1.40-1.56, 0.86.

### Example 21(33)

### N⁵-(2-ethyl-4-methoxyphenyl)-6-methyl-N⁷,N⁷-dipropylpyrazolo[1,5-a]pyrimidine-5,7-diamine:

TLC: Rf 0.42 (hexane : ethyl acetate = 3 : 1);
¹H-NMR (300MHz, CDCl₃): δ 7.80, 7.67, 6.71-6.89, 6.12, 6.06, 3.82, 3.19- 3.47, 2.63, 2.28, 1.39-1.58, 1.25, 0.87.

### Example 21(34)

### 6-methyl-N⁷,N⁷-dipropyl-N⁵-(2,4,6-trimethyl-3-pyridinyl)pyrazolo[1,5-a]pyrimidme-5,7-diamine:

TLC: Rf 0.51 (ethyl acetate : methanol = 9 : 1);
¹H-NMR (300MHz, CDCl₃): δ 7.78, 6.94, 6.05, 5.75, 3.18-3.50, 2.51, 2.44, 2.33, 2.20, 1.37-1.63, 0.88.

### Example 21(35)

### 6-methoxy-N⁷-(2-methoxyethyl)-N⁷-propyl-N⁵-(2,4,5-trimethylphenyl)pyrazolo[1,5-a]pyrimidine-5,7-diamine:

TLC: Rf 0.36 (hexane : ethyl acetate = 3 : 1);
¹H-NMR (300MHz, CDCl₃): δ 7.89, 7.81, 6.99, 6.94, 6.16, 3.99, 3.86, 3,56- 3.63, 3.52, 3.25, 2.25-2.30, 2.22, 1.54-1.67, 0.87.

### Example 21(36)

### 3-chloro-4-{[7-(dipropylamino)-6-methylpyrazolo[1,5-a]pyrimidin-5-yl]amino}-N-methylbenzamide:

TLC: Rf 0.57 (methylene chloride : methanol = 9: 1);
¹H-NMR (300MHz, CDCl₃): δ 8.99, 7.89-7.93, 7.65, 7.38, 6.32, 6.07, 3.30-3.45, 2.99-3.05, 2.38, 1-41-1.56,0.86.

### Example 21(37)

### N⁵-(3,5-dichloro-2-pyr-idinyl)-6-methyl-N⁷,N ⁷-dipropylpyrazolo[1,5-a]pyrimidine-5,7-diamine:

TLC: Rf 0.48 (hexane : ethyl acetate = 2 : 1);
¹H-NMR (300MHz, CDCl₃): δ 0.86, 1.41-1.63, 2.19, 3.29-3,59, 6.36, 7.08, 7.68, 7.93, 8.16.

### Example 21(38)

### N⁵-[2-chloro-4-(trifluoromethoxy)phenyl]-6-methyl-N⁷N⁷-dipropylpyrazolo[1,5-a]pyrimidine-5,7-diamine:

TLC: Rf 0.61 (toluene : ethyl acetate =9:1);
¹H-NMR (300MHz, CDCl₃): δ 0.87, 1.38-1.59, 2.37, 3.22-3.50, 6.28, 7.14, 7.17-7.27, 7.31, 7.89, 8.93.

### Example 21(39)

### N⁵-[4-isopropyl-2-(methylsulfanyl)phenyl]-6-methyl-N⁷,N⁷-dipropylpyrazolo[1,5-a]pyrimidine-5,7-diamine:

TLC: Rf0.58 (toluene : ethyl acetate = 9 : 1);
¹H-NMR (300MHz, CDCl₃): δ 0.87, 1.26, 1.40-1.57, 2.37, 2.41, 2.79-2.96, 3,35, 6.23, 7.23, 7.37, 7,85, 7.89, 8.63.

### Example 21(40)

### N⁵-(2-ethyl-4-methylphenyl)-6-methyl-N⁷,N⁷-dipropylpyrazolo[1,5-a]pyrimidine-5,7-diamine:

TLC: Rf D.44 (hexane : ethyl acetate = 5 : 1);
¹H-NMR (300MHz, CDCl₃): δ 0.87, 1.26, 1.39-1.58, 2.29, 2.34, 2.64, 3.35, 6.16, 6.19, 7.01-7.12, 7.75-7.86.

### Example 21(41)

### N⁵-(4-chloro-2-ethylphenyl)-6-methyl-N⁷,N⁷-dipropylpyrazolo[1,5-a]pyrimidine-5,7-diamine:

TLC: Rf 0.38 (hexane : ethyl acetate = 5 : 1);
¹H-NMR (300MHz, CDCl₃): δ 0.87, 1.29, 1.41-1.57, 2.30, 2.65, 3.36, 6.19, 6.24, 7.18-7.28, 7.85, 8.06.

### Example 21(42)

### N⁵-mesityl-6-methoxy-N⁷,N⁷-dipropylpyrazolo[1,5-a]pyrimidine-5,7-diamine:

TLC: Rf 0.49 (hexane : ethyl acetate = 1 : 1);
¹H-NMR (300MHz, CDCl₃): δ 0.90, 1,53-1.71, 2.23, 2.30, 3.51-3.66, 3.84, 6.04, 6.42, 6.95, 7.77.

### Example 21(43)

### N⁵-mesityl-N⁷,N⁷-bis(2-methoxyethyl)-6-methylpyrazolo[1,5-a]pyrimidine-5,7-diamine: TLC: Rf 0.36 (hexane : ethyl acetate =1:1);

¹H-NMR (300MHz, CDCl₃): δ 7.76, 6.95, 6.07, 5.79, 3.56-3.68, 3.45, 3.28, 2.35, 2.31, 2.19.

### Example 21 (44)

### N⁵-(4,6-dimethyl-3-pyridinyl)-6-methyl-N⁷,N⁷-dipropylpyrazolo[1,5-a]pyrimidine-5,7-diamine:

TLC: Rf 0.26 (hexane : ethyl acetate = 1 : 1);
¹H-NMR (300MHz, CDCl₃): δ 0.87, 1.41-1.58, 2.26, 2.32, 2.53, 3.28-3.43, 6,05, 6.13, 7.05, 7.83, 8.80.

### Example 21(45)

### N⁵-(4-chloro-2-methoxyphenyl)-6-methyl-N⁷,N⁷-dipropylpyrazolo[1,5-a]pyrimidine-5,7-diamine:

TLC: Rf 0.56 (hexane : ethyl acetate =5:1);
¹H-NMR (300MHz, CDCl₃): δ0.85, 1.37-1.56, 2.32, 3.34, 3.94, 6.26, 6.88, 7.01, 7.24, 7.86, 8.78.

### Example 21 (46)

### 6-methoxy-N⁷,N⁷-dipropyl-N⁵-(2,4, 5-trimethylphenyl)pyrazolo[1,5-a]pyrimidine-5,7-diamine:

TLC: Rf 0.40 (hexane : ethyl acetate = 5 : 1);
¹H-NMR (300MHz, CDCl₃): δ 0.89, 1.51-1.69, 2.22, 2.27, 2.28, 3.52.-3.64, 3.82, 6.16, 6.90, 6.98, 7.82, 7.87.

### Example 21 (47)

### 6-methoxy-N⁵-(4-methyl-2-vinylphenyl)-N⁷,N⁷-dipropylpyrazolo[1,5-a]pyrimidine-5,7-diamine:

TLC: Rf 0.49 (hexane : ethyl acetate = 5 : 1);
¹H-NMR (300MHz, CDCl₃): δ 0.88, 1.52-1,68, 2,35, 3.52-3,63, 3.81, 5.39, 5.70, 6.16, 6.89, 7.12, 7.15, 7.23-7.30, 7.83, 8.10.

### Example 21(48)

### N⁵-[2-chloro-4-(methylsulfonyl)phenyl]-6-methyl-N⁷,N⁷-dipropylpyrazolo[1,5-a]pyrimidine-5,7-diamine:

TLC: Rf 0.61 (hexane : ethyl acetate = 1 : 1);
¹H-NMR (300MHz, CDCl₃): δ 0.87, 1.41-1.57, 2.40, 3.08, 3.31-3.51, 6.36, 7.49, 7.89, 7.95, 7.99, 9.18.

### Example 21(49)

### N⁵-(2-ethyl-4,5-dimethoxyphenyl)-6-methyl-N⁷,N⁷-dipropylpyrazolo[1,5-a]pyrimidine-5,7-diamine:

TLC: Rf 0.49 (hexane : ethyl acetate =1:1);
¹H-NMR (300MHz, CDCl₃): δ 0.88, 1.25, 1.42-1.57, 2.29, 2.62, 3.35, 3.88, 3.90, 6.13, 6.14,6.76, 7.49, 7.82.

### Example 21(50)

### N⁵-(2-ethyl-4-methylphenyl)-6-methoxy-N⁷,N⁷-dipropylpyrazolo[1,5-a]pyrimidine-5,7-diamine:

TLC: Rf0.34 (hexane : ethyl acetate = 8 : 1);
¹H-NMR (300MHz, CDCl₃): δ 8.07, 7.82, 7.00-7.11, 6.14, 3.82, 3.53-3.63, 2.66, 2.33, 1.53-1.68, 1.27,0.88.

### Example 21(51)

### N⁵-(4-chloro-2-ethylphenyl)-6-methoxy-N⁷,N⁷-dipropylpyrazolo[1,5-a]pyrimidine-5,7-diamine:

TLC: Rf0.40 (hexane : ethyl acetate = 8 : 1);
¹H-NMR (300MHz, CDCl₃): δ 8.34, 7.85, 7.16-7.29, 7.12, 6.18, 3.82, 3.53- 3.65, 2.67, 1.52-1.69, 1.30,0.88.

### Example 21(52)

### N⁵-(2,4-dimethyl-6-vinylphenyl)-6-methyl-N⁷N⁷-dipropylpyrazolo[1,5-a]pyrimidine-5,7-diamine:

TLC: Rf0.27 (hexane : acetone =5:1);
¹H-NMR (300MHz, CDCl₃): δ 0.89, 1.43-1.60, 2.18, 2.31, 2.35, 3.25-3.44, 5.22, 5.67, 5.81, 6.07, 6.81, 7.05, 7.27, 7.78.

### Example 21(53)

### N⁵-(2-ethyl-4,6-dimethylphenyl)-6-methyl-N⁷,N⁷-dipropylpyrazolo [1,5-a]pyrimidine-5,7-diamine:

TLC: Rf0.30 (hexane : acetone =5:1);
¹H-NMR (300MHz, CDCl₃): δ 0.89, 1.17, 1.43-1.59, 2.19, 2.32, 2.33, 2.57, 3.34, 5.77, 6.06, 6.97, 7.77.

### Example 21(54)

### N⁵-(2-isopropenyl-4-methylphenyl)-6-methyl-N⁷,N⁷-dipropylpyrazolo[1,5-a]pyrimidine-5,7-diamine;

TLC: Rf 0.58 (hexane : ethyl acetate =3:1);
¹H-NMR (300MHz, CDCl₃) : δ 0.86, 1.37-1.56, 2.09, 2.22, 2.33, 3.33, 5.10, 5.37-5,46, 6.22, 6.97, 7.02, 7.13, 7.84, 8.47.

### Example 21(5 5)

### N⁵-(2-isopropyl-4-methylphenyl)-6-methyl-N⁷,N⁷-dipropylpyrazolo[1,5-a]pyrimidine-5,7-diamine:

TLC: Rf 0.50 (hexane : ethyl acetate = 3 : 1);
¹H-NMR (300MHz, CDCl₃): δ 0.88, 1.27, 1.42-1.58, 2.29, 2.36, 3.01-3.18, 3,35, 6.13, 6.15, 7.05, 7.13, 7.61, 7.81.

### Example 21(56)

### N⁵-(2-ethyl-4-methoxyphenyl)-6-methoxy-N⁷,N⁷-dipropylpyrazolo[1,5-a]pyrimidine-5,7-diamine:

TLC: Rf0.31 (hexane : ethyl acetate = 5 : 1);
¹H-NMR (300MHz, CDCl₃): δ 7.89-7.96, 7.81, 6.87, 6.79-6.84, 6.11, 3.83, 3.82, 3.54-3.62, 2.66, 1.53-1.69, 1.27, 0.89.

### Example 21(57)

### 6-methyl-N⁵-[4-(methylsulfanyl)-2-(trifluoromethyl)phenyl]-N⁷,N⁷-dipropylpyrazolo[1,5-a]pyrimidine-5,7-diamine:

TLC: Rf 0.51 (hexane : ethyl acetate = 5 : 1);
¹H-NMR (300MHz, CDCl₃): δ 0.87, 1.41-1.59, 2.29, 2.52, 3.29-3.44, 6.23, 6.87, 7,46-7.55, 7.88, 8.53.

### Example 21(58)

### N⁵-[4-isopropyl-2-(methylsulfnyl)phenyl]-6-methyl-N⁷,N⁷-dipropylpyrazolo[1,5-a]pyrimidine-5,7-diamine:

TLC: Rf 0.45 (hexane : ethyl acetate = 2 : 1);
¹H-NMR (300MHz, CDCl₃): δ 0.86, 1.25, 126, 1.42-1.58, 2.32, 2.83-2.96, 2.93, 3.23-3.49, 6.23, 7.16, 7.40, 7.88, 8.64, 9.72.

### Example 21(59)

### N⁵-[4-isopropyl-2-(methylsulfonyl)phenyl]-6-methyl-N⁷,N⁷-dipropylpyrazolo[1,5-a]pyrimidine-5,7-diamine:

TLC: Rf 0.62 (hexane : ethyl acetate = 2 : 1);
¹H-NMR (300MHz, CDCl₃): δ 0.87, 1.28, 1.42-1.59, 2.32, 2.90-3.03, 3.07, 3.31-3.44, 6.25, 7.53, 7.77, 7.90, 8.61, 8.78.

### Example 21(60)

### N⁵-(2-chloro-4,5-dimethylphenyl)-6-methyl-N⁷,N⁷-dipropylpyrazolo[1,5-a]pyrimidine-5,7-diamine:

TLC: Rf 0.72 (hexane : ethyl acetate =3:1);
¹H-NMR (300MHz, CDCl₃): δ 0.86, 1.39-1.57, 2.22, 2.30, 2.34, 3.36, 6.27, 6.98, 7.15, 7.87, 8.50.

### Example 21(61)

### N⁵-(5-chloro-2,3-dimethylphenyl)-6-methyl-N⁷,N⁷-dipropylpyrazolo[1,5-a]pyrimidine-5,7-diamine:

TLC: Rf 0.62 (hexane : ethyl acetate = 3 : 1);
¹H-NMR (300MHz, CDCl₃): δ 0.87, 1.42-1.56, 2.17, 2.30, 2.31, 3.30-3.42, 6.19, 6.21, 6.98, 7.84, 7.85.

### Example 21(62)

### N⁵-[2-(dimethylamino)-4-methylphenyl]-6-methyl-N⁷,N⁷-dipropylpyrazolo[1,5-a]pyrimidine-5,7-diamine:

TLC: Rf 0.68 (toluene : ethyl acetate = 9 : 1);
¹H-NMR (300MHz, CDCl₃): δ 0.86, 1.38-1.56, 2.32, 2.70, 3.34, 6.23, 6.96- 7.02, 7.85, 8.16, 5.65.

### Example 21(63)

### 6-methoxy-N⁷-(2-methoxyethyl)-N⁵-(4-methyl-2-vinylphenyl)-N⁷-propylpyrazolo[1,5-a]pyrimidine-5,7-diamine:

TLC: Rf 0.38 (hexane : ethyl acetate = 3 : 1);
¹H-NMR (300MHz, CDCl₃): δ 8.12, 7.82, 7.24-7.29, 7.12-7.19, 6.89, 6.16, 5.70, 5.40, 3.99, 3.85, 3.55-3.64, 3.48-3.55, 3.25, 2.35, 1.54-1.65, 0.87.

### Example 21(64)

### N⁵-(2-ethyl-4-methylpheny)-6-methoxy-N⁷-(2-methoxyethyl)-N⁷-propylpyrazolo [1,5-a]pyrimidine-5,7-diamine:

TLC: Rf 0.3 5 (hexane : ethyl acetate =3:1);
¹H-NMR (300MHz, CDCl₃): δ 8.09, 7.81, 7.02-7.13, 6.15, 3.98, 3.86, 3.55- 3.64, 3.52, 3.25, 2.67, 2.33, 1.52-1.66, 1.28, 0.87.

### Example 21(65)

### N⁵-(4-chloro-2-ethylphenyl)-6-methoxy-N⁷-(2-methoxyethyl)-N⁷-propylpyrazolo[1,5-a]pyrimidine-5, 7-diamine:

TLC: Rf0.39 (hexane : ethyl acetate =3:1);
¹H-NMR (300MHz, CDCl₃): δ 8.36, 7.84, 7.21-7.27, 7.20, 7.17, 6.19, 4.01, 3.87, 3.56-3.65, 3.52, 3.24, 2.68, 1.52-1.67, 1.31, 0.87.

### Example 21(66)

### 6-methyl-N⁷,N⁷-dipropyl-N⁵-(2,3,5-trimethyl-4-pyridinyl)pyrazolo[1,5-a]pyrimidine-5,7-diamine:

TLC: Rf0.34 (methylene chloride : methanol = 9 : 1);
¹H-NMR (300MHz, CDCl₃): δ 0.89, 1.43-1.64, 2.15, 2.18, 2.34, 2.54, 3.28-3.45, 5.85, 6.12, 7.82, 8.24.

### Example 21(67)

### N⁵-(2-cyclopropyl-4-methylphenyl)-6-methyl-N⁷,N⁷-dipropylpyrazolo[1,5-a]pyrimidine-5,7-diamine:

TLC: Rf 0.61 (hexane : ethyl acetate = 3 : 1);
¹H-NMR (300MHz, CDCl₃): δ 0.66-0.78, 0.87, 0.94-1.07, 1.39-1.57, 1.74- 1.92, 2.31, 2.35, 3.35, 6.23, 7.00, 7.10, 7.17, 7.85, 8.42.

### Example 21(68)

### N⁵-[4-isapropyl-2-(methylsulfanyl)phenyl]-6-methoxy-N⁷,N⁷-dipropylpyrazolo[1,5-a]pyrimidine-5,7-diamine:

TLC: Rf 0.60 (hexane : ethyl acetate = 8 : 1);
¹H-NMR (300MHz, CDCl₃): δ 8.69, 8.45, 7.86, 7.38, 7.23, 6.21, 3.85, 3.54- 3.66, 2.81-2.96, 2.42, 1.52-1.68, 1.26, 0.88.

### Example 21(69)

### N⁵-(4-cyclopropyl-2-methylphenyl)-6-methyl-N⁷,N⁷-dipropylpyrazolo[1,5-a]pyrimidine-5,7-diamine:

TLC: Rf0.61 (hexane : ethyl acetate =3:1);
¹H-NMR (300MHz, CDCl₃): δ 0.64-0.72, 0.87, 0.90-0.97, 1.40-1.57, 1.80- 1.93, 2.28, 2.29, 3.35, 6.14, 6.16, 6.92-6.99, 7.80-7.89.

### Example 21(70)

### N⁵-(2-ethyl-4-methoxyphenyl)-N⁷-(2-methoxyethyl)-6-methyl-N⁷-propylpyrazolo[1,5-a]pyrimidine-5,7-diamine:

TLC: Rf 0.25 (hexane : ethyl acetate = 3 : 1);
¹H-NMR (300MHz, CDCl₃): δ 7.80, 7.69, 6.77-6.85, 6.13, 6.10, 3.83, 3.56- 3.68, 3.42, 3.31-3.39, 3.28, 2.64, 2.30, 1.43-1.58, 1.25, 0.88.

### Example 21(71)

### N⁵-(2-ethyl-4-methylphenyl)-N⁷-(2-methoxyethyl)-6-methyl-N⁷-propylpyrazolo[1,5-a]pyrimidine-5,7-diamine:

TLC: Rf 0.33 (hexane : ethyl acetate = 3 : 1);
¹H-NMR (300MHz, CDCl₃): δ 7.77-7.85, 7.03-7.11, 6.22, 6.16, 3.56-3.67, 3.42, 3.33-3.39, 3.27, 2.64, 2.34, 2.31, 1.43-1.58, 1.26, 0.87.

### Example 21(72)

### N⁵-(4-chloro-2-ethylphenyl)-N⁷-(2-methoxyethyl)-6-methyl-N⁷-propylpyrazolo[1,5-a]pyrimidine-5,7-diamine:

TLC: Rf 0.38 (hexane : ethyl acetate =3:1);
¹H-NMR (300MHz, CDCl₃): δ 8.02-8.11, 7.84, 7.18-7.28, 6.27, 6.20, 3.58- 3.69, 3.42, 3.32-3.38, 3.26, 2.65, 2.32, 1.42-1.58, 1.29, 0.87.

### Example 21(73)

### N⁵-(2-methyl-4-ethylphenyl)-6-methyl-N⁷,N⁷-dipropylpyrazolo[1,5-a]pyrimidine-5,7-diamine:

TLC: Rf 0.52 (hexane : ethyl acetate = 5 : 1);
¹H-NMR (300MHz, CDCl₃): δ 0.87, 1.24, 1.42-1.57, 2.30, 2.62, 3.35, 6.16, 6.17, 7.03.-7.14, 7.83, 7.89.

### Example 21(74)

### N⁵-(2-methyl-4-vinylphenyl)-6-methyl-N⁷,N⁷-dipropylpyrazolo[1,5-a]pyrimidine-5,7-diamine:

TLC: Rf 0.52 (hexane : ethyl acetate =5:1);
¹H-NMR (300MHz, CDCl₃): δ 0.87, 1.41-1.56, 2.31, 2.34, 3.36, 5.18, 5.69, 6.21, 6.28, 6.69, 7.28, 7,33, 7.85, 8.14.

### Example 21(75)

### N⁵-(3,5-dimethyl-2-pyridinyl)-6-methyl-N⁷,N⁷-dipropylpyrazolo[1,5-a]pyrim1dine-5,7-diamine:

TLC: Rf 0.61 (hexane : ethyl acetate = 1 : 5);
¹H-NMR (300MHz, CDCl₃): δ 0.86, 1.42-1.57, 2.24, 2.25, 2.30, 3.27-3.46, 6.16, 6.57, 7.39, 7.84, 8.04.

### Example 21 (76)

### 6-methyl-N⁵-[4-methyl-2-(methylsulfanyl)phenyl]-N⁷,N⁷- dipropylpyrazolo 1,5-a]pyrimidine-5,7-diamine:

TLC: Rf 0.63 (hexane : ethyl acetate = 5 : 1);
¹H-NMR (300MHz, CDCl₃): δ 0.87, 1.40-1.57, 2.33, 2.37, 2.40, 3.35, 6.24, 7.17, 7.33, 7.86, 7.88, 8.62.

### Example 21(77)

### 6-methyl-N⁵-[2-methyl-4-(methylsulfanyl)phenyl]-N⁷,N⁷-dipropylpyrazolo[1,5-a]pyrimidine-5,7-diamine:

TLC: Rf 0.57 (hexane : ethyl acetate = 3: 1);
¹H-NMR (300MHz, CDCl₃): δ 0.87, 1.41-1.56, 2.30, 2.49, 3.28-3.43, 6.16-6.22, 7.14-7.24, 7.84, 8.03.

### Example 21(78)

### 6-methyl-N⁵-[2-methyl-4-(methylsulfinyl)phenyl]-N⁷,N⁷-dipropylpyrazolo[1,5-a]pyrimidine-5,7-diamine:

TLC: Rf0.53 (methanol : ethyl acetate = 1:9);
¹H-NMR (300MHz, CDCl₃): δ 0.87, 1.40-1.57, 2.34, 2.42, 2.74, 3.27-3.47, 6.26, 6.43, 7.49, 7.58, 7.89, 8.54.

### Example 21(79)

### 6-methyl-N⁵-[2-methyl-4-(methylsulfonyl)phenyl]-N⁷,N⁷-dipropylpyrazolo[1,5-a]pyrimidine-5,7-diamine:

TLC: Rf 0.33 (hexane : ethyl acetate =1:1);
¹H-NMR (300MHz, CDCl₃): δ 0.87, 1.41-1.58, 2.35, 2.42, 3.05, 3.27-3.53, 6.31, 6.54, 7.77, 7.83, 7.91, 8.70.

### Example 21(80)

### N⁵-(4-isopropyl-2-methylphenyl)-6-methyl-N⁷,N⁷-dipropylpyrazolo[1,5-a]pyrimidine-5,7-diamine:

TLC: Rf 0.63 (hexane : ethyl acetate =3:1);
¹H-NMR (300MHz, CDCl₃): δ 0.87, 1.25, 1.41-1.56, 2.29, 2.31, 2.79-2.96, 3.35, 6.13-6.20, 7.08, 7.12, 7.83, 7.92.

### Example 21(81)

### N⁷-(methoxyethyl)-6-methyl-N⁵-(4-methyl-2-vinylphenyl)-N⁷-propylpyrazolo[1,5-a]pyrimidine-5,7-diamine:

TLC: Rf 0.38 (hexane : ethyl acetate=3:1);
¹H-NMR (300MHz, CDCl₃): δ 0.87, 1.41-1.58, 2.29, 2.36, 3.27, 3.32-3.39, 3.42, 3.57-3.68, 5.38, 5.70, 6.18, 6.33, 6.86, 7.15, 7.28, 7.82, 7.88.

### Example 21(82)

### N⁵-[4-isopropyl-2-(methylsulfanyl)phenyl]-N⁷-(2-methoxyethyl)-6-methyl-N⁷⁻propylpyrazolo[1,5-a]pyrimidine-5,7-diamine:

TLC: Rf 0.56 (hexane : ethyl acetate = 3 : 1);
¹H-NMR (300MHz, CDCl₃): δ 0.87, 1.26, 1.43-1.58, 2.39, 2.41, 2.83-2.95, 3.26, 3.33-3.39, 3.42, 3.57-3.69, 6.25, 7.20-7.28, 7.39, 7.86, 7.93, 8.65.

### Example 21(83)

### N⁵-(2-ethyl-4,5-dimethoxyphenyl)-N⁷-(2-methoxyethyl)-6-methyl-N⁷-propylpyrazolo[1,5-a]pyrimidine-5,7-diamine:

TLC: Rf0.43 (hexane : ethyl acetate = 1 : 1);
¹H-NMR (300MHz, CDCl₃): δ 0.88, 1.25, 1.44-1.60, 2.31, 2.61, 3.28, 3.32- 3.40, 3.43, 3.57-3.68, 3.88, 3.90, 6.14, 6.15, 6.76, 7.50, 7.82.

### Example 21(84)

### N⁵-(4-ethyl-6-methyl-3-pyridinyl)-6-methyl-N⁷,N⁷-dipropylpyrazolo[1,5-a]pyrimidine-5,7-diamine:

TLC: Rf 0.31 (hexane : ethyl acetate = 1 : 2);
¹H-NMR (300 MHz, CDCl₃). δ 0.87, 1.26, 1.41-1.59, 2.31, 2.55, 2.62, 3.25- 3,51, 6.05, 6.13, 7.08, 7.83, 8.81.

### Example 21(85)

### N⁵-(4-isopropyl-2-vinylphenyl)-6-methyl-N⁷,N⁷-dipropylpyrazolo[1,5-a]pyrimidine-5,7-diamine:

TLC: Rf 0.50 (hexane : ethyl acetate =5:1);
¹H-NMR (300 MHz, CDCl₃): δ 0.87, 1.27, 1.40-1.59, 2.26, 2.82-3.01, 3.35, 5.40, 5.72, 6.19, 6.41, 6.89, 7.21, 7.30, 7.84, 7.94.

### Example 21(86)

### N⁵-(4-ethyl-2-isopropylphenyl)-6-methyl-N⁷,N⁷-dipropylpyrazolo[1,5-a]pyrimidine-5,7-diamine:

TLC: Rf 0.49 (hexane : ethyl acetate = 5 : 1);
¹H-NMR (300 MHz, CDCl₃): δ 0.87, 1.26, 1.28, 1.42-1.57, 2.27, 2.67, 2.81- 2.99, 3.35, 6.17, 6.30, 707-7.16, 7.83, 7.88.

### Example 21 (87)

### N⁵-(4-chloro-2-(methylsulfanyl)phenyl)-6-methyl-N⁷,N⁷-dipropylpyrazolo[1,5-a]pyrimidine-5,7-diamine:

TLC: Rf 0.73 (toluene : ethyl acetate = 9 : 1);
¹H-NMR (300 MHz, CDCl₃): δ 8.75, 7.88, 7.86, 7.47, 7.31, 6.27, 3.36, 2.44, 2.37, 1.49, 0.87.

### Example 21(88)

### N⁵-(2-methyl-4-(2-dimethylaminoethoxy)phenyl)-6-methyl-N⁷,N⁷-dipropylpyrazolo[1,5-a]pyrimidine-5,7-diamine:

TLC: Rf 0.28 (ethyl acetate : methanol: triethylamine = 9 : 1 : 0.3);
¹H-NMR (300 MHz, CDCl₃): δ 7.81, 7.69, 6.86-6.78, 6.13, 6.05, 4.08, 3.34, 2.75, 2.36, 2.29, 2.27, 1.49, 0.87.

### Example 21(89)

### N⁵-(2-methyl-4-(3-dimethylaminopropoxy)phenyl)-6-methyl-N⁷,N⁷-dipropylpyrazolo[1,5-a]pyrimidine-5,7-diamine:

TLC: Rf 0.25 (ethyl acetate : methanol : triethylamine = 9 : 1 : 0.3);
¹H-NMR (300 MHz, CDCl₃): δ 7.81, 7.66, 6.84-6.75, 6.13, 6.04, 4.02, 3.34, 2.48, 2.29, 2.28, 2.27, 1.97, 1.49, 0.87.

### Example 22(1)-Example 22(7)

The following compounds were obtained by the same procedure as a series of reactions of Example 3 → Example 4, using a corresponding compound prepared by the same procedure as a series of reactions of Example 19 → Example 20.

### Example 22(1)

### N⁵-(2-chlaro-4-methoxyphenyl)-6-ethyl-N⁷,N⁷-dipropylpyrazolo [1,5-a]pyrimidine-5,7-diamine:

The title compound having the following physical data was obtained by the same procedure as a series of reactions of Example 3 → Example 4, using 5,7-dichloro-6-ethylpyrazolo[1,5-a]pyrimidine.
TLC: Rf0.34 (hexane : ethyl acetate = 9 : 1);
¹H-NMR (300MHz, CDCl₃): δ 8.64, 7.86, 7.04, 6.98, 6.92, 6.22, 3.81, 3.24-3.39, 2.92, 1.41-1.58, 1.35, 0.88.

### Example 22(2)

### N⁵-(2-chloro-4-methoxyphenyl)-N⁷,N⁷, 6-tripropylpyrazolo[1,5-a]pyrimidine-5,7-diamine: TLC: Rf 0.32 (hexane ; ethyl acetate =10:1);

¹H-NMR (300MHz, CDCl₃): δ 0.88, 1.13, 1.50, 1.72, 2.82, 3.29, 3.81, 6.22, 6.91, 6.98, 7.05, 7.86, 8.68.

### Example 22(3)

### N⁵-(2-chloro-4-methoxyphenyl)-6-isopropyl-N⁷,N⁷-dipropylpyrazolo[1,5-a]pyrimidine-5,7-diamine:

TLC: Rf0.64 (hexane : ethyl acetate = 10 : 1);
¹H-NMR (300MHz, CDCl₃). δ 0.88, 1.50, 3.20, 3.38, 3.81, 4.24, 6.20, 6.91, 6.98, 7.09, 7.85, 8.63.

### Example 22(4)

### N⁵-(2-chloro-4-methoxyphenyl)-6-methoxy-N⁷,N⁷-dipropylpyrazolo[1,5-a]pyrimidine-5,7-diamine:

TLC: Rf 0.5 (hexane : ethyl acetate = 10 : 1);
¹H-NMR (300MHz, CDCl₃): δ 0.88, 1.60, 3.60, 3.81, 3.85, 6.20, 6.91, 6.98, 7.67, 7.86, 8.70.

### Example 22(5)

### 6-butyl-N⁵-(2-chloro-4-methoxyphenyl)-N⁷,N⁷-dipropylpyrazolo [1,5-a]pyrimidine-5,7-diamine:

TLC: Rf0.3 (hexane : ethyl acetate = 10 : 1);
¹H-NMR (300MHz, CDCl₃): δ 0.88, 1.03, 1.58, 2.84, 3.30, 3.81, 6.22, 6.92, 6.98, 7.05, 7.86, 8.67.

### Example 22(6)

### N5-(2-chloro-4-methoxyphenyl)-6-phenyl-N⁷,N⁷-dipropylpyrazolo[1,5-a]pyrimidine-5,7-diamine:

TLC: Rf 0.24 (hexane : ethyl acetate = 9 : 1);
¹H-NMR (300MHz, CDCl₃): δ 0-75, 1.41, 3.08, 3.76, 6.24, 6.74, 6,86,7.45, 7.57, 7.91, 8.61.

### Example 22(7)

### N⁵-(2-chloro-4-methoxyphenyl)-6-cyclopentyl-N⁷,N⁷-dipropylpyrazola[1,5-a]pyrimidine-5,7-diamine:

TLC: Rf 0.28 (hexane : ethyl acetate =9:1);
¹H-NMR (300MHz, CDCl₃) δ 0.88, 1.46, 1.81, 1.98, 3.31, 3.81, 4.22, 6.18, 6.69, 6.90, 6.97, 7.85, 8.37.

### Example 23(1)-Example 23(38)

The following compounds were obtained by the same procedure as a series of reactions of Example 3 → Example 4, using a corresponding amine.

### Example 23(1)

### N⁵-(2-chloro-4-methoxyphenyl)-N⁷-(2-methoxyethyl)-6-methyl-N⁷-propylpyrazolo[1,5-a]pyrimidine-5,7-diamine:

TLC: Rf 0.13 (hexane : ethyl acetate = 6 : 1);
¹H-NMR (300MHz, CDCl₃): δ 0.87, 1.40-1.58,2.36,3.26,3.29-3.50,3.52-3.72,3.81,6.23, 6.87-6.97, 6.98, 7.86, 8.63.

### Example 23(2)

### N⁵-(2-chloro-4-methoxyphenyl)-N⁷,N⁷-bis(2-methoxyethyl)-6-methylpyrazolo[1,5-a]pyrimidine-5,7-diamine:

TLC: Rf 0.32 (hexane : ethyl acetate = 1 : 1);
¹H-NMR (300MHz, CDCl₃): δ 2.38, 3.26, 3.38-3.48, 3.53-3.72, 3.81, 6.24, 6.87-6.96, 6.99, 7.85, 8.63.

### Example 23(3)

### ^{N}-(2-chloro-4-methoxyphenyl)-N⁷-(cyclopropylmethyl)-6-methyl-N⁷-(4-methylbenzyl)pyrazolo[1,5-a]pyrimidine-5,7-diamine:

TLC: Rf 0.36 (hexane : ethyl acetate = 8 : 1);
¹H-NMR (300MHz, CDCl₃): δ -0.13 - -0.02, 0.25-0.41, 0.78-0.95, 2.31, 2.34, 3.08-3.35, 3.81, 4.57, 6.25, 6-86-6.95, 6.98, 7.07, 7.19, 7.90, 8.64.

### Example 23(4)

### N⁵-(2-chloro-4-methoxyphenyl)-N⁷-(cyclopropylmethyl)-N⁷-(2-methoxyethyl)-6-methylpyrazolo[1,5-a]pyrimidine-5,7-diamine:

TLC: Rf 0.38 (hexane : ethyl acetate = 2 : 1);
¹H-NMR (300MHz, CDCl₃): δ 0.09-0.10, 0.29-0.46, 0.81-0.98, 2.41, 3.21-3.36, 3.37-3.49, 3.55-3.76, 3.81, 6.24, 6.92, 6.96, 6.99, 7.85, 8.66.

### Example 23 (5)

### N⁵-(2-chloro-4-methoxyphenyl)-N⁷-ethyl-N⁷-(2-methoxyethyl)-6-dipropylpyrazolo[1,5-a]pyrimidine-5,7-diamine:

TLC: Rf0.32 (hexane : ethyl acetate = 2 : 1);
¹H-NMR (300MHz, CDCl₃): δ 8.64, 7.85, 6.99, 6.88-6.96, 6.23, 3.81, 3.56-3.70, 3.35-3.55, 327, 2.37, 1.06.

### Example 23(6)

### N⁵-(2-chloro-4-methoxyphenyl)-N⁷-(cyclopropylmethyl)-6-methyl-N⁷-propylpyrazolo[1,5-a]pyrimidine-5,7-diamine:

TLC: Rf 0.30 (hexane : ethyl acetate = 8 : 1);
¹H-NMR (300MHz, CDCl₃): δ -0.11-0.09, 0.28-0.44, 0.78-0.97, 1.39-1.56, 2.41, 3.15-3.50, 3.81, 6.23, 6.87-6.97, 6.99, 7.85, 8.66.

### Example 23(7)

### N⁵-(2-chloro-4-methoxyphenyl)-N⁷-[2-methoxy-1-(methoxymethyl)ethyl]-6-methylpyrazolo[1,5-a]pyrimidine-5,7-diamine;

TLC: Rf0.30 (hexane: ethyl acetate = 2 : 1);
¹H-NMR (300MHz, CDCl₃): δ 8.50, 7.82, 6.97, 6.89, 6.75, 6.16, 6.08, 4.13-4.26, 3.80, 3.53-3.66, 3.39, 2.30.

### Example 23(8)

### 2-[{5-[(2-chloro-4-methoxyphenyl)amino]-6-methylpyrazolo[1,5-a]pyrimidin-7-yl} (propyl)amino]ethanol:

TLC: Rf 0.30 (hexane : ethyl acetate = 1 : 1);
¹H-NMR (300MHz, CDCl₃): δ 0.87, 1.33-1.54, 2.32, 3.30-3.53, 3.53-3.76, 3.82, 4.98-5.15, 6.26, 6.87-6.97, 6.99, 7.86, 8.58.

### Example 23(9)

### N⁷-butyl-N⁵-(2-chloro-4-methoxyphenyl)-6-methyl-N⁷-propylpyrazolo[1,5-a]pyrimidine-5,7-diamine:

TLC: Rf0.35 (hexane : ethyl acetate = 9 : 1);
¹H-NMR (300MHz, CDCl₃): δ 0.81-0.91, 1.20-1.37, 1.37-1.56, 2.34, 3.28-3-46, 3.81, 6.23, 6.87-6.95, 6.98, 7.86, 8.64.

### Example 23(10)

### N⁵-(2-chloro-4-methoxyphenyl)-N⁷-(2-methoxy-1-methylethyl)-6-methylpyrazolo[1,5-a]pyrimidine-5,7-diamine:

TLC: Rf 0.1 (hexane : ethyl acetate = 4: 1);
¹H-NMR (300MHz, CDCl₃): δ 1.34, 2.30, 3.38, 3.48, 3.80, 4.07-4.25, 5.88, 6.16, 6.75, 6.90, 6.97, 7.81, 8.50.

### Example 23(11)

### N⁵-(2-chloro-4-methoxyphenyl)-N⁷,N⁷-diethyl-6-methylpyrazolo[1,5-a]pyrimidine-5,7-diamine:

TLC: Rf 0.28 (hexane : ethyl acetate = 12 : 1);
¹H-NMR (300MHz, CDCl₃): δ 8.64, 7.85, 6.99, 6.87-6.96, 6.23, 3.81, 3.46, 2.36, 1.05.

### Example 23(12)

### N⁵-(2-chloro-4-methoxyphenyl)-N⁷-ethyl-6-methyl-N⁷-propylpyrazolo[1,5-a]pyrimidine-5,7-diamine:

TLC: Rf 0.32 (hexane : acetone = 9 : 1);
¹H-NMR (300MHz, CDCl₃) δ: 0.87, 1.05, 1.39-1.56, 2.35, 3.29-3.40, 3.40-3.52, 3.81, 6.23, 6.87-6.96, 6.98, 7.86, 8.63.

### Example 23(13)

### N⁵-(2-chloro-4-methoxyphenyl)-N⁷-[(1R)-1-(methoxymethyl)propyl]-6-methylpyrazolo[1,5-a]pyrimidine-5,7-diamine:

TLC: Rf 0.20 (hexane : ethyl acetate = 4 : 1);
¹H-NMR (300MHz, CDCl₃): δ 1.00, 1.52-1.89, 2.30, 3.35, 3.43-3.57, 3.80, 3.86-4.03, 5.87, 6.16, 6.76, 6.89, 6.97, 7.81, 8.50.

### Example 23(14)

### N⁷-(sec-butyl)-N⁵-(2-chloro-4-methoxyphenyl)-6-methylpyrazolo[1, 5-a] pyrimidine-5,7-diamine:

TLC : Rf 0.18 (hexane : ethyl acetate =9:1);
¹H-NMR (300MHz, CDCl₃): δ 8.50, 7.80, 6.97, 6.89, 6.75, 6.16, 5.76, 3.82-3.94, 3.80, 2.30, 1.53-1.79, 1.30, 0.99.

### Example 23(15)

### N⁵-(2-chloro-4-methoxyphenyl)-6-methoxy-N⁷-(2-methoxyethyl)-N⁷-propylpyrazolo[1,5-a]pyrimidine-5,7-diamine:

TLC: Rf 0.45 (toluene : ethyl acetate = 4 : 1);
¹H-NMR (300MHz, CDCl₃): δ 8.70, 7.85, 7.71, 6.98, 6.91, 6.20, 4.00, 3.89, 3.81, 3.57-3.66, 3.51, 3.24, 1.51-1.68, 0.87.

### Example 23(16)

### N⁵-(2-chloro-4-methoxyphenyl)-N⁷-[1-(methoxymethyl)butyl]-6-methylpyrazolo[1,5-a]pyrimidine-5,7-diamine:

TLC: Rf 0.26 (hexane : ethyl acetate = 4 : 1);
¹H-NMR (300MHz, CDCl₃): δ 0.93, 1.32-1.83, 2.30, 3.34, 3.42-3.55, 3.80, 3.96-4.10, 5.85, 6.16, 6.76, 6.90, 6.97, 7.81, 8.51.

### Example 23(17)

### N⁵-(2-chloro-4-methoxyphenyl)-N⁷-(3-methoxypropyl)-6-methyl-N⁷-propylpyrazolo[1,5-a]pyrimidine-5,7-diamine:

TLC: Rf 0.23 (hexane : ethyl acetate = 4 : 1);
¹H-NMR (300MHz, CDCl₃): δ 0.86, 1,38-1,55, 1.64-1.80, 2.34, 3.23, 3.28-3.43, 3.42-3.55, 3.81, 6.21, 6.83-6.95, 6.97, 7.84, 8.60.

### Example 23(18)

### N⁷-butyl-N⁵-(2-chloro-4-methoxyphenyl)-N⁷-ethyl-6-methylpyrazalo[1,5-a]pyrimidine-5,7-diamine:

TLC: Rf 0.34 (hexane : ethyl acetate = 8 : 1);
¹H-NMR (300MHz, CDCl₃): δ 0.87, 1.05, 1.20-1.52, 2.35, 3.28-3.53, 3.81, 6.23, 6.86-6.96, 6.98, 7.85, 8.63.

### Example 23(19)

### N-(2-chloro-4-methoxyphenyl)-7-[(2S, 4R)-4-methoxy-2-(methoxymethyl)-1-pyrrolidinyl]-6-methylpyrazolo[1,5-a]pyrimidine-5-amine:

TLC: Rf 0.40 (hexane : ethyl acetate = 1 : 1);
¹H-NMR (300MHz, CDCl₃); δ 8.59, 7.85, 6.98, 6.91, 6.86, 6.22, 4.74-4.86, 4.10-4.20, 4.00-4.10, 3.81, 3.38-3.45, 3.36, 3.17-3.31, 3.15, 2.30-2.40, 2.29, 2.03-2.13.

### Example 23 (20)

### N⁵-(2-chloro-4-methoxyphenyl)-6-methyl-N⁷-propyl-N⁷-(2-pyridinylmethyl)pyrazolo[1,5-a]pyrimidine-5,7-diamine:

TLC: Rf 0.30 (hexane : ethyl acetate = 2 : 1);
¹H-NMR (300MHz, CDCl₃) : δ 0.85, 1.47-1.65, 2.27, 3.31-3.54, 3.81, 4.74, 6.25, 6.84, 6.91, 6.97, 7.09-7.20, 7.34-7.45, 7.54-7.65, 7.91, 8.50-8.56, 8.59.

### Example 23(21)

### 7-(1-azepanyl)-N-(2-chloro-4-methoxyphenyl)-6-methylpyrazolo[1,5-a]pyrimidine-5-amine:

TLC: Rf 0.57 (hexane : ethyl acetate = 5 : 1);
¹H-NMR (300MHz, CDCl₃): δ 8.58, 7.86, 6.98, 6.91, 6.88, 6.23, 3.81, 3.39-3.46, 2.37, 1.74-1.88.

### Example 23(22)

### N-(2-chloro-4-methoxyphenyl)-6-methyl-7-(1,4-oxazepan-4-yl)pyrazolo[1,5-a]pyrimidine-5-amine:

TLC: Rf 0.49 (hexane ; ethyl acetate = 2 : 1);
¹H-NMR (300MHz, CDCl₃): δ 8.56, 7.85, 6.98, 6.91, 6.87, 6.24, 3.97-4.03, 3.91-3.96, 3.81, 3.55-3.62, 2.38, 2.03-2.12.

### Example 23(23)

### N-(2-chloro-4-methoxyphenyl)-6-methyl-7-(4-methyl-1,4-diazepan-1-yl)pyrazolo[1,5-a]pyrimidine-5-amine:

TLC: Rf 0.31 (chloroform : methanol = 10 : 1);
¹H-NMR (300MHz, CDCl₃): δ 8.56, 7.85, 6.98, 6.90, 6.86, 6.23, 3.81, 3.49-3.62, 2.80-2.86, 2.75-2.80, 2.46, 2.37, 2.02-2.11.

### Example 23(24)

### N⁵-(2-chloro-4-methoxyphenyl)-6-methyl-N⁷-propyl-N⁷-(1,3-thiazol-4-ylmethyl)pyrazolo[1,5-a]pyrimidine-5,7-diamine:

TLC: Rf 0.49 (hexane : ethyl acetate =1:1);
¹H-NMR (300MHz, CDCl₃): δ 8.74, 8.59, 7.90, 7.20, 6.97, 6.90, 6.85, 6.24, 4.79, 3.80, 3.37-3.50, 2.24, 1.47-1.62, 0.87.

### Example 23(25)

### Methyl [{5-[(2-chloro-4-methoxyphenyl)amino]-6-methylpyrazolo[1,5-a]pyrimidin-7-yl}(ethyl)amino]acetate:

TLC: Rf 0.30 (hexane : ethyl acetate =2:1);
¹H-NMR (300MHz, CDCl₃): δ 1.14, 2.20, 3.58, 3.79, 4.03, 4.08, 6.34, 6.81, 6.96, 7.82, 7.86, 10.62.

### Example 23(26)

### N⁵-(2-chloro-4-methoxyphenyl)-N⁷-(cyclopropylmethyl)-6-methoxy-N⁷⁻propylpyrazolo[1,5-a]pyrimidine-5,7-diamine:

TLC: Rf0.56 (hexane : ethyl acetate = 4 : 1);
¹H-NNM (300MHz, CDCl₃): δ 8.71, 7.85, 7.70, 6.98, 6.91, 6.20, 3.91, 3.81, 3.62-3.68, 3.61, 1.53-1.67, 0.98-1.15, 0.88, 0.40-0.49, 0.07-0.17.

### Example 23 (27)

### N-(2-chloro-4-methoxyphenyl)-7-[(2S)-2-(methoxymethyl)- I -pyrrolidinyl]-6-methylpyrazolo[1,5-a]pyrimidine-5-amine:

TLC: Rf 0.40 (hexane : ethyl acetate = 3 : 1);
¹H-NMR (300MHz, CDCl₃): δ 8.61, 7.83, 6.98, 6.91, 6.88, 6.23, 4.67-4.76, 3.81, 3.58-3.67, 3.35-3.44, 3-24, 3.18, 2.32, 2.26-2.37, 2.01-2.13, 1.83-1.96.

### Example 23 (28)

### N-(2-chloro-4-methoxyphenyl)-7-[(2R)-2-(methoxymethyl)-1-pyrrolidinyl]-6-methylpyrazolo[1,5-a]pyrimidine-5-amine:

TLC: Rf 0.40 (hexane : ethyl acetate =3:1);
¹H-NMR (300MHz, CDCl₃): δ 8.61, 7.83, 6.98, 6.84-6.94, 6.23, 4.66-4.77, 3.81, 3.57-3.67, 3.34-3.45, 3.20-3.27, 3.18, 2.32, 2.26-2.41, 2.01-2.14, 1.83-1.95.

### Example 23 (29)

### N-(2-chloro-4-methoxyphenyl)-7-[(2R,6S)-2,6-dimethyl-4-morpholinyl]-6-methylpyrazolo[1,5-a]pyrimidine-5-amine:

TLC: Rf0.44 (hexane : ethyl acetate =3:1);
¹H-NMR (300MHz, CDCl₃): δ 8.55, 7.84, 6.98, 6.91, 6.84, 6.21, 3.89-4.02, 3.81, 3.31-3.46, 3.10-3.26, 2.32, 1.24.

### Example 23(30)

### N-(2-chloro-4-methoxyphenyl)-6-methyl-7-(4-methyl-1-piperazinyl)pyrazolo [1,5-a]pyrimidine-5-amine:

TLC Rf0.24 (chloroform : methanol = 19:1);
¹H-NMR (300MHz, CDCl₃): δ 2.31, 2.39, 2.52-2.74, 3.43-3.69, 3.80, 6.20, 6.81, 6.90, 6.97, 7.83, 8.54.

### Example 23(31)

### 7-(4-acetyl-1-piperazinyl)-N-(2-chloro-4-methoxyphenyl)-6-methylpyrazolo[1,5 - a]pyrimidine-5-amine:

TLC: Rf 0.21 (hexane : ethyl acetate = 1 : 3);
¹H-NMR (300:MHz, CDCl₃): δ 2.17, 2.35, 3.32-3.99, 3.81, 6.22, 6.86, 6.91, 6.99, 7.83, 8.54.

### Example 23(32)

### N-(2-chloro-4-methoxyphenyl)-7-[(2S,4R)-2-(mothoxymethyl)-4-methyl-1-pyrrolidinyl]-6-methylpyrazolo[1,5-a]pyrimidine-5-amine:

TLC: Rf0.18 (hexane : ethyl acetate = 8 : 1);
¹H-NMR (300MHz, CDCl₃): δ 1.14, 1.89-2.11, 2.31, 2.42-2.63, 2.97-3.10, 3.18, 3.22, 3.69, 3.81, 4.67-4.87, 6.23, 6.87, 6.91, 6.98, 7.85, 8.60.

### Example 23(33)

### N-(2-chloro-4-methoxyphenyl)-7-[(2S,4R)-4-ethoxy-2-(ethoxymethyl)-1-pyrrolidinyl]-6-methylpyrazolo[1,5-a]pyrimidine-5-amine:

TLC: Rf 0.20 (hexane : ethyl acetate =3:1);
¹H-NMR (300MHz, CDCl₃): δ 0.94, 1.21, 1.99-2.14, 2.26-2.41, 2.29, 3.16-3.34, 3.34-3.43, 3.43-3.62, 3.81, 4.03-4.14, 4.20-4.32, 4.66-4.81, 6.22, 6.84, 6.91, 6.98, 7.84, 8.57.

### Example 23(34)

### N-(2-chloro-4-methoxyphenyl)-7-[(2S,4S)-2-(methoxymethyl)-4-fluoro-1-pyrrolidinyl]-6-methylpyrazolo[1,5-a]pyrimidine-5-amine:

TLC: Rf0.26 (hexane : ethyl acetate = 12 : 1);
¹H-NNM (300MHz, CDCl₃): δ 2.09-2.30, 2.40, 2.57-2.88, 3.23, 3.33-3.48, 3.49-3.68, 3.71-3.94, 3.81, 4.43-4.60, 5.25-5.53, 6.25, 6.92, 6.95, 6.99, 7.80, 8.62.

### Example 23(35)

### N-(2-chloro-4-methoxyphenyl)-7-[(2S,4S)-2-(methoxymethyl)-4-methyl-1-pyrrolidinyl]-6-methylpyrazolo[1, 5-a]pyrimidine-5-amine:

TLC: Rf 0.20 (toluene : ethyl acetate = 19 : 1);
¹H-NMR (300MHz, CDCl₃): δ 1.15, 1.44-1.59, 2.27-2.42, 2.31, 2.46-2.67, 3.15, 3.17-3.25, 3.25-3.47, 3.81, 4.93-5.06, 6.22, 6.86, 6.91, 6.98, 7.84, 8.59.

### Example 23 (3 6)

### N-(2-chloro-4-methoxyphenyl)-7-[(2S,4R)-4-ethyl-2-(methoxymethyl)-1-pyrrolidinyl]-6-methylpyrazolo[1,5-a]pyrimidine-5-amine

TLC: Rf 0.25 (toluene : ethyl acetate = 19 : 1);
¹H-NMR (300MHz, CDCl₃): δ 0.96, 1.44-1.65, 1.95-2.09, 2.23-2.43, 2.31, 3.03-3.14, 3.19, 3.20-3.29, 3.63-3.76, 3.81, 4.64-4.77, 6.23, 6.87, 6.91, 6.98, 7.84, 8.60.

### Example 23(37)

### N-(2-chloro-4-methoxyphenyl)-7-[(2R,4S)-2-(methoxymethyl)-4-methyl-1-pyrrolidinyl]-6-methylpyrazolo[1, 5-a]pyrimidine-5-amine:

TLC: Rf 0.19 (toluene : ethyl acetate = 19 : 1);
¹H-NMR (300MHz, CDCl₃): δ 1.14, 1.93-2.08, 2.30, 2.41-2.63, 2.98-3.08, 3.18, 3.23, 3.64-3.75, 3.81, 4.71-4.87, 6.23, 6.84-6.96, 6.98, 7.55, 8.51-8.66.

### Example 23 (38)

### N-(2-chloro-4-methoxyphenyl)-7-[(2S)-2-(methoxymethyl)-4-phenyl-2,5-dihydro-1H-pyrrol-1-yl]-6-methylpyrazolo[1,5-a]pyrimidine-5-amine:

TLC: Rf 0.19 (hexane : ethyl acetate =6:1);
¹H-NMR (300 MHz, CDCl₃): δ 2.42, 3.24, 3.43, 3.82, 4.57-4.70, 4.74-4.89, 546-5.63, 6.28, 6.33-6.39, 6.93, 6.98, 7.00, 7.22-7.49, 7.86, 8.58-8.70.

### Example 24(1)-Example 24(4)

The following compounds were obtained by the same procedure as a series of reactions of Example 3 → Example 4, using a corresponding compound prepared by the same procedure as a series of reactions of Example 19 → Example 20.

### Example 24(1)

### N⁵-(2-chloro-4-methoxyphenyl)-2,6-dimethyl-N⁷,N⁷-dipropylpyrazolo[1,5-a]pyrimidine-5,7-diamine:

The title compound having the following physical data was obtained by the same procedure as a series of reactions of Example 3 → Example 4, using 2,6-dimethyl-5,7-dichloropyrazolo[1,5-a]pyrimidine.
TLC: Rf 0.78 (hexane : ethyl acetate = 6 : 1);
¹H-NMR (300MHz, CDCl₃): δ 0.85, 1.45, 2.30, 2.40, 3.33, 3.79, 5.99, 6.83, 6.89, 6.96, 8.60.

### Example 24(2)

### N⁵-(2-chloro-4-methoxyphenyl)-N⁷-(1-ethylpropyl)-2,6-dipropylpyrazolo[1,5-a]pyrimidine-5,7-diamine:

The title compound having the following physical data was obtained by the same procedure as a series of reactions of corresponding Example 3 → Example 4, using 2,6-dimethyl-5,7-dichloropyrazolo[1,5-a]pyrimidine.
TLC: Rf 0.42 (hexane : ethyl acetate =6:1);
¹H-NMR (300MHz, CDCl₃): δ 0.96, 1.65, 2.25, 2.38, 3.68, 3.78, 5.72, 5.92, 6.67, 6.87, 6.95,8.45.

### Example 24(3)

### N⁵-(2-chloro-4-methoxyphenyl)-2-ethyl-6-methyl-N⁷,N⁷-dipropylpyrazolo[1,5-a]pyrimidine-5,7-diamine:

The title compound having the following physical data was obtained by the same procedure as a series of reactions of corresponding Example 3 → Example 4, using 2-ethyl-5,7-dichloro-6-methylpyrazolo[1,5-a]pyrimidine,
TLC: Rf 0.64 (hexane : ethyl acetate =5:1);
¹H-NMR (300MHz, CDCl₃): δ 0.86, 1.31, 1.46, 2.32, 2.77, 3.34, 3.80, 6.03, 6.84, 6.90, 6.97, 8.62.

### Example 24(4)

### 5-[(2-chloro-4-methoxyphenyl)amino]-7-(dipropylamino)-6-methylpyrazolo[1,5-a]pyrimidine-3-carbonitrile:

The title compound having the following physical data was obtained by the same procedure as a series of reactions of Example 3 → Example 4, using 3-cyano-5,7-dichloro-6-methylpyrazolo[1,5-a]pyrimidine.
TLC: Rf0.48 (hexane: ethyl acetate = 3 : 1);
¹H-NMR (300MHz, CDCl₃): δ 0.86, 1.41-1.55, 2.34, 3.27-3.43, 3.83, 6.91-7.04, 7.18, 8.05, 8.77.

### Example 25:

### N²-(2-chloro-4-methoxyphenyl)-N²-ethyl-N⁴,N⁴-dipropyl-5,7-dihydrofuro[3,4-d]pyrimidine-2,4-diamine:

The title compound having the following physical data was obtained by the same procedure as a series of reactions of Example 5, using a corresponding compound prepared in Example 4 and a corresponding halide.
TLC: Rf 0.42 (hexane : ethyl acetate =2:1);
¹H-NMR (300MHz, CDCl₃): δ 0.72, 1.18, 1.43, 3.01, 3.68, 3.81, 4.10, 4.77, 5.12, 6.82, 7.00, 7.14.

### Example 26:

### N²-ethyl-N²-mesityl-N⁴,N⁴-dipropyl-5,7-dihydrofuro[3,4-d]pyrimidine-2,4-diamine:

The title compound having the following physical data was obtained by the same procedure as a series of reactions of Example 4 → Example 5, using a corresponding compound prepared in Example 3 and corresponding aniline.
TLC: Rf 0.16 (hexane : ethyl acetate = 8 : 1);
¹H-NMR (300MHz, DMSO-d₆): δ 0.70, 1.13, 1.39, 2.01, 2.23, 3.09, 3.74, 4.57, 5.03, 6.87.

### Example 27:

### 2-[(2-chloro-4-methoxyphenyl)(methyl)amino]-4-(dipropylamino)furo[3,4-d]pyrimidin-7(5H)-one:

The title compound having the following physical data was obtained by the same procedure as a series of reactions of Example 3 → Example 4 → Example 5, using 2,4-dichlorofuro[3,4-d]pyrimidin-7(5H)-one.
TLC: Rf0,27 (hexane : ethyl acetate = 2 : 1);
¹H-NMR (300MHz, CDCl₃): δ 0.34-1.09, 1.14-1.61, 2.84-3.20, 3.47, 3.81, 5.19-5.38, 6.83, 7.00,7.17.

### Example 28(1)-Example 28(11)

The following compounds were obtained by the same procedure as a series of reactions of Example 5, using the compound prepared in Example 4(1) and a corresponding halide.

### Example 28(1)

### N²-(2-chloro-4-methoxyphenyl)-N²-ethyl-N⁴,N⁴-dipropyl-6,7-dihydro-5H-cyclopenta[d]pyrimidine-2,4-diamine:

TLC: Rf 0.18 (hexane : ethyl acetate = 4 : 1);
¹H-NMR (300MHz, CDCl₃): δ 0.72, 1.17, 1.42, 1.93, 2.70, 2.89, 3.17, 3.80, 4.10, 6.81, 6.99, 7.14.

### Example 28(2)

### N²-(2-chloro-4-methoxyphenyl)-N²,N⁴,N⁴-tripropyl-6,7-dihydro-5H-cyclopenta[d]pyrimidine-2,4-diamine:

TLC: Rf0.25 (hexane : ethyl acetate = 4 : 1);
¹H-NMR (300MHz, CDCl₃): δ 0.73, 0.90, 1.44, 1.60, 1.93, 2.69, 2.89, 3.17, 3.58, 3.80, 4.01, 6.80, 6.98, 7.15.

### Example 28(3)

### N²-(2-chloro-4-methoxyphenyl)-N²-isopropyl-N⁴,N⁴-dipropyl-6,7-dihydro-5H-cyclopenta[d]pyrimidine-2,4-diamine:

TLC: Rf 0.65 (hexane : tetrahydrofuran = 2 : 1);
¹H-NMR (300MHz, CDCl₃): δ 0.70, 1.06, 1.34, 1.94, 2.72, 2.89, 3.12, 3.81, 5.11, 6.80, 7.00, 7.10.

### Example 28(4)

### N²-aryl-N²-(2-chloro-4-methoxyphenyl)-N⁴,N⁴-dipropyl-6,7-dihydro-5H-cyclopenta[d]pyrimidine-2,4-diamine:

TLC: Rf 0.24 (hexane : ethyl acetate = 4 :1);
¹H-NMR (300MHz, CDCl₃): δ 0.72, 1.43, 1.94, 2.70, 2.89, 3.17, 3.79, 4.16, 4.82, 5.05, 6.02, 6.78, 6.98, 7.13.

### Example 28(5)

### N²-(2-chloro-4-methoxyphenyl)-N²-(2-methyl-2-propenyl)-N⁴,N⁴-dipropyl-6,7-dihydro-5H-cyclopenta[d]pyrimidine-2,4-diamine:

TLC: Rf 0.32 (hexane : ethyl acetate = 4:1);
¹H-NMR (300MHz, CDCl₃): δ 0.72, 1.42, 1.79, 1.94, 2.69, 2.89, 3.18, 3.79, 3.98, 4.76, 4.80, 4.94, 6.77, 6.97, 7.17.

### Example 28(6)

### N²-(2-chloro-4-methoxyphenyl)-N²-isobutyl-N⁴,N⁴-dipropyl-6,7-dihydro-5H-cyclopenta[d]pyrimidine-2,4-diamine:

TLC: Rf0.23 (hexane : ethyl acetate =4:1);
¹H-NMR (300MHz, CDCl₃): δ 0.71, 0.94, 1.43, 1.90, 2.66, 2.89, 3.15, 3.41, 3.80, 4.02, 6.80, 6.98, 7.18.

### Example 28(7)

### N-(2-chloro-4-methoxyphenyl)-N-[4-(dipropylamino)-6,7-dihydro-5H-cyclopenta[d]pyrimidin-2-yl]acetamide:

TLC: Rf 0.48 (ethyl acetate);
¹H-NMR (300MHz, cDCl₃): δ 0.77, 1.38, 2.01, 2.46, 2.81, 2.96, 3.21, 3.80, 6.81, 7.00, 7.19.

### Example 28(8)

### N-(2-chloro-4-methoxyphenyl)-N-[4-(dipropylamino)-6,7-dihydro-5H-cydopenta[d]pyrimidin-2-yl]methanesulfonamide:

TLC: Rf 0.35 (hexane : ethyl acetate = 2 : 1);
¹H-NMR (300MHz, CDCl₃): δ 0.73, 1.39, 1.99, 2.78, 2.93, 3.16, 3.71, 3.81, 6.85, 6.98, 7.36.

### Example 28(9)

### N²-(2-chloro-4-methoxyphenyl)-N²-(cyclopropylmethyl)-N⁴,N⁴-dipropyl-6,7-dihydro-5H-cyclopenta[d]pyrimidine-2,4-diamine:

TLC: Rf 0.18 (hexane : ethyl acetate = 4 : 1);
¹H-NMR (300MHz, CDCl₃): δ 0.02-0.19, 0.29-0.47, 0.57-4.84, 0.99-1.19, 1.30-1.54, 1.85-2.04, 2.60-2.78, 2.83-2.96, 2.99-3.30, 3.30-3.51, 3.80, 3.96-4.14, 6.80, 6.97, 7.23.

### Example 28(10)

### N²-(2-chloro-4-methoxyphenyl)-N²-(2-methoxyethyl)-N⁴,N⁴-dipropyl-6,7-dihydro-5H-cyclopenta[d]pyrimidine-2,4-diamine:

TLC: Rf0.41 (hexane : ethyl acetate =2:1);
¹H-NMR (300MHz, CDCl₃): δ 7.21, 6.97, 6.80, 4.23-4.39, 3.80, 3.51-3.78, 3.31, 3.05-3.26, 2.85-2.93, 2.65-2.75, 1.87-2.01, 1.33-1.50, 0.66-0.79.

### Example 28(11)

### N²-ethyl-N²-mesityl-N⁴, N⁴-dipropyl-6,7-dihydro-5H-cyclopenta[d]pyrimidine-2,4-diamine:

The title compound having the following physical data was obtained by the same procedure as a series of reactions of Example 3 (using 2,4-dimethyl-6,7-dihydro-5H-cyclopenta[d]pyrimidine) → Example 4 (using a corresponding aniline) → Example 5 (using a corresponding halide).
TLC: Rf0.52 (hexane : ethyl acetate = 3 : 1);
¹H-NMR (300MHz, DMSO-d₆): δ 0.70, 1.11, 1.39, 1.89, 2.00, 2.23, 2.53, 2.87, 3.18, 3.72, 6.86.

### Example 29(1)-Example 29(10)

The following compounds were obtained by the same procedure as a series of reactions of Example 5, using the compound prepared in Example 4(12) and a corresponding halide.

### Example 29(1)

### N⁵-(2-chloro-4-methoxyphenyl)-N⁵-ethyl-N⁷,N⁷-dipropylpyrazolo[1,5-a]pyrimidine-5,7-diamine:

TLC: Rf 0.20 (hexane : ethyl acetate = 8 : 1);
¹H-NMR (300MHz, CDCl₃): δ 0.76, 1.20, 1.51, 3.39, 3.78, 3.85, 4.19, 4.72, 6.12, 6.90, 7.08, 7.20, 7.81.

### Example 29(2)

### N⁵-(2-chloro-4-methoxyphenyl)-N⁵-(2-methyl-2-propenyl)-N⁷,N⁷-dipropylpyrazolo[1,5-a]pyrimidine-5,7-diamine:

TLC: Rf 0.25 (hexane : ethyl acetate =8: 1);
¹H-NMR (300MHz, CDCl₃): δ 0.77, 1.55, 1.82, 3.42, 3.84, 3.99, 4.80, 5.07, 6.10, 6.86,7.06, 722, 7.80.

### Example 29(3)

### N⁵-aryl-N⁵-(2-chloro-4-methoxyphenyl)-N⁷,N⁷-dipropylpyrazolo[1,5-a]pyrimidine-5,7-diamine:

TLC: Rf 0.21 (hexane : ethyl acetate = 8 : 1);
¹H-NMR (300MHz, DMSO-d₆): δ 0.69, 1.46, 3.43, 3.80, 4.16, 4.73, 5.09, 5.92, 6.00, 7.02, 7.22, 7.34, 7.81.

### Example 29(4)

### N⁵-(2-chloro-4-anethoxyphenyl)-N⁵-(cyelopropylmethyl)-N⁷,N⁷-dipropylpyrazolo[1,5-a]pyrimidine-5,7-diamine:

TLC: Rf0.50 (hexane : ethyl acetate =6:1); )
¹H-NMR (300MHz, CDCl₃): δ 0.06-0.20, 0.34-0.46, 0.76, 1.00-1.18, 1.42-1.60, 3.33-3.56, 3.85, 4.03-4.23, 4.73, 6.11, 6.89, 7.06, 7.29, 7.80.

### Example 29(5)

### N⁵-(2-chloro-4-methoxyphenyl)-N⁵-[2-(dimethylamino)ethyl]-N⁷,N⁷-dipropylpyrazolo[1,5-a]pyrimidine-5,7-diamine trihydrochloride:

TLC: Rf0.42 (chloroform : methanol = 9 : 1);
¹H-NMR (300MHz, CDCl₃): δ 0.80, 1.53-1.72, 2.96, 3.05, 3.42-3.76, 3.86, 3.88-3.95, 4.39-4.40, 4.53-4.66, 4.80-4.93, 6.90, 7.06, 7.10, 7.55, 7.87.

### Example 29(6)

### N⁵-(2-chloro-4-methoxyphenyl)-N⁵-[3-(dimethylamino)propyl]-N⁷,N⁷-propylpyrazolo[1,5-a]pyrimidine-5,7-diamine methanesulfonate:

TLC: Rf 0.38 (chloroform : methanol = 9 : 1);
¹H-NMR (300MHz, CDCl₃): δ 0.78, 1.47-1.66, 2.16-2.40, 2.80, 2.93, 3.21-3.35, 3.37-3.63, 3.73-3.93, 4.11-4.31, 4.51, 6.24-6.60, 7.00, 7.09, 7.39, 7.83.

### Example 29(7)

### N5-(2-chloro-4-methoxyphenyl)-N⁵,N⁷,N⁷-tripropylpyrazolo [1,5-a]pyramidine-5,7-diamine:

TLC: Rf 0.56 (hexane : ethyl acetate = 3 : 1);
¹H-NMR (300MHz, CDCl₃) : δ 7.80, 7.20, 7.07, 6.89, 6.11, 4.66-4.75, 3.95-4.23, 3.85, 3.51-3.72, 3.34-3.45, 1.42-1.70, 0.94, 0.76.

### Example 29(8)

### N⁵-(2-chloro-4-methoxyphenyl)-N⁵-(2-methoxyethyl)-N⁷,N⁷-dipropylpyrazolo [1,5-a]pyrimidine-5,7-diamine:

TLC: Rf0.57 (hexane : ethyl acetate = 2 : 1);
¹H-NMR (300MHz, CDCl₃): δ 7.81, 7.31, 7.05, 6.89, 6.11, 4.73, 4.35-4.55, 3.84, 3.55-3.81, 3.40, 3.32, 1.43-1.59, 0.76.

### Example 29(9)

### ethyl {(2-chloro-4-methoxyphenyl)[7-(dipropylamino)pyrazolo[1,5-a]pyrimidin-5-yl]aminol acetic acid:

TLC: Rf 0.61 (hexane: ethyl acetate =2:1);
¹H-NMR (300MHz, CDCl₃): δ 7.80, 7.62, 7.06, 6.88, 6.08, 5.11-5.33, 4.83, 4.15-4.28, 3.75-3.98, 3.38-3.48, 1.46-1.60, 1.28, 0.77.

### Example 29(10)

### N⁵-(2-chloro-4-methoxyphenyl)-N⁵-(methoxymethyl)-N',N'-dipropylpYrazolo [1,5-a]pyrimidine-5,7-diamine:

TLC: Rf 0.57 (hexane : ethyl acetate = 2 : 1);
¹H-NMR (300MHz, DMSO-d₆): δ 7.80, 7.38, 7.18, 7.04, 6.00, 5.19, 4.92, 3.84, 3.48-3.57, 3.37, 1.44-1.60, 0.76.

### Example 30

### 6-methyl-5-(5-methyl-2,3-dihydro-1H-indol-1-yl)-N,N-dipropylpyrazolo[1,5-a]pyrimidine-7-amine:

A compound prepared by the same procedure as a series of reactions of Example 3 using 5,7-dichloro-6-methylpyrazolo[1,5-a]pyrimidine was dissolved into N,N-dimethyimidazolidinone (2.5 mL). To the mixture of the solution and indoline (149 mg), tris(dibenzylideneacetone)dipalladium (34 mg), N,N-dimesitylimidazolium hydrochloride (25 mg) and sodium hexadimethyldisilazide (275 mg) were added under argon gas atmosphere at room temperature. The mixture was stirred for 1 hour at 100°C under argon gas atmosphere. After the reaction mixture was cooled, a saturated aqueous solution of ammonium chloride (2.0 mL) was added to stop the reaction to the mixture. Water (3.0 mL), hexane (4.0 mL) and ethyl acetate (1.0 mL) were added to the mixture, and then it was stirred for 10 minutes. The organic layer was separated, and the water layer was extracted by a mixture of hexane and ethyl acetate (4:1). The combined organic layer was dried over anhydrous magnesium sulfate and concentrated under reduced pressure. Tetrahydrofuran (2.0 mL) and pyridine (0.3 mL) were added to the residue. And then a solution of phthalic acid anhydride of tetrahydrofuran (1.3 mL) was added to the mixture. The mixture was stirred for 30 minutes at room temperature. The reaction mixture was passed through trisamine resin supported by polystyrene, and after it was washed three times with tetrahydrofuran, the solution was concentrated under reduced pressure. The residue was purified by column chromatography (hexane : acetone = 5 : 1) to give the title compound (136 mg) having the following physical data.
TLC: Rf 0.48 (hexane : ethyl acetate = 5 : 1);
¹H-NMR (300MHz, CDCl₃): δ 7.93, 7.04, 6.90, 6.48, 6.31, 4.11, 3.38-3.59, 3.08, 2.29, 2.14, 1.47-1.66,0.88.

### Example 31

### 5-(5-methyl-2,3-dihydro-1H-indol-1-yl)-N,N-dipropylpyrazolo[1,5-a]pyrimidine-7-amine:

The title compound having the following physical data was obtained by the same procedure as a series of reactions of Example 30, using a compound prepared by the same procedure as a series of reactions of Example 3 using 5,7-dichloropyrazolo[1,5-a]pyrimidine.
TLC: Rf 0.61 (toluene: ethyl acetate = 9:1);
¹H-NMR (300MHz, CDCl₃): δ 7.98, 7.87, 6.92-7.07, 6.19, 5.66, 4.13, 3.59-3.74, 3.15, 2.31, 1.61-1.79, 0.93.

### Example 31(1)

### 5-(5-chloro-1H-indol-1-yl)-N,N-dipropylpyrazolo[1,5-a]pyrimidine-7-amine:

The title compound having the following physical data was obtained by the same procedure as a series of reactions of Example 31, using a corresponding compound.
TLC: Rf 0.64 (toluene : ethyl acetate = 9 : 1);
¹H-NMR (300MHz, CDCl₃): δ 8.23, 7.98, 7.69, 7.60, 7.25, 6.63, 6.43, 6.02, 3.65-3,94, 1.67-1.90, 0.98.

### Example 32

### imidazo[1,2-a]pyrimidin-5,7-diol:

Under argon gas atmosphere, sodium (1.39 g) was added to ethanol (36 mL) by little and little, and the ethanol solution was stirred until sodium was solved completely. A solution of 2-aminoimidazole 1/2 hydrochloride (4.00 g) and diethyl malonate (4.6 mL) were dropped, successively, to the above solution. The mixture was refluxed for 6 hours at 90°C. After the reaction mixture was cooled to room temperature, it was concentrated under reduced pressure. Water (50 mL) was added to the residue. The solution was acidified (pH = 1) by adding 5N hydrochloric acid (10 mL) with stirring under an ice-bath. The precipitate was collected by filtration under vacuum and dried under vacuum to give the title compound having the following physical data.
¹H-NMR (300MHz, CDCl₃): δ 4.97, 7.33, 7.41, 10.39-12.49.

### Example 33

### 5,7-dichloroimidazo[1,2-a]pyrimidine:

Under argon gas atmosphere, phosphoryl chloride (12.4 g) was dropped to the compound prepared in Example 32 (680 mg). The mixture was stirred for 4 hours at 100°C. After the reaction mixture was cooled, it was concentrated under reduced pressure. The obtained residue was dissolved to an ice-water slowly. After the solution was nitrified by adding sodium bicarbonate, it was extracted twice with ethyl acetate. The organic layer was washed with water and normal saline solution, dried over magnesium sulfate, filtered and concentrated under reduced pressure to give the title compound (680 mg) having the following physical data.
TLC: Rf 0.63 (ethyl acetate);
¹H-NMR (300MHz, CDCl₃): δ 7.05, 7.71, 7.86.

### Example 34

### N⁷-(2-chloro-4-methoxyphenyl)-N⁷-ethyl-N⁵,N⁵-dipropylimidazo[1,2-a]pyrimidine-5,7-diamine:

The title compound having the following physical data was obtained by the same procedure as a series of reactions of Example 3 using the compound prepared in Example 33 → Example 4 → Example 5 using a corresponding halide.
TLC: Rf 0.23 (ethyl acetate : methanol = 9 : 1);
¹H-NMR (300MHz, CDCl₃): δ 0.80, 1.22, 1.36-1.59, 2.84-3.06, 3.75-3.86, 3.86, 4.14-4.39, 5.02, 6.90, 7.08, 7.12, 7.19, 7.37.

### Example 34(1)-Example 34(13)

The following compounds were obtained by the same procedure as a series of reactions of Example 3 using the compound prepared in Example 33 or a corresponding compound → Example 4, or by the same procedure as a series of reactions of Example 3 using the compound prepared in Example 33 or a corresponding compound → Example 4 → Example 5 using a corresponding halide.

### Example 34(1)

### N⁷-aryl-N⁷-(2-chloro-4-methoxyphenyl)-N⁵,N⁵-dipropylimidazo[1,2-a]pyrimidine-5,7-diamine:

TLC: Rf0.45 (chloroform : methanol = 10 : 1);
¹H-NMR (300MHz, CDCl₃): δ 7.37, 7.17, 7.13, 7.06, 6.87, 5.98-6.14, 4.91-5,15, 4.19, 3.85, 2.94-3.02, 1.40-1.55, 0.80.

### Example 34(2)

### N⁷-(2-chloro-4-methoxyphenyl)-N⁵,N⁵,N⁷-tripropylimidazo[1,2-a]pyrimidine-5,7-diamine:

TLC: Rf 0.44 (chloroform : methanol = 10 : 1);
¹H-NMR (300MHz, CDCl₃): δ 7.37, 7.20, 7.12, 7.07, 6.90, 5.02, 4.08-4.23, 3.86, 3.61-3.74, 2.92-3.02, 1.62-1.75, 1.40-1.56, 0.93, 0.80.

### Example 34(3)

### N⁷-(2-chloro-4-methoxyphenyl)-N⁷-(2-methoxyethyl)-N⁵,N⁵-dipropylimidazo[1,2-a]pyrimidine-5,7-diamine:

TLC: Rf 0.36 (chloroform : methanol = 10 : 1);
¹H-NMR (300MHz, CDCl₃): δ 7.38, 7.32, 7.12, 7.04, 6.90, 5.05, 4.44-4.57, 3.70-3.93, 3.59-3.69, 3.31, 2.92-3.03, 1.40-1.56, 0.80.

### Example 34(4)

### N⁷-(2-chloro-4-methoxyphenyl)-6-methoxy-N⁵-(2-methoxyethyl)-N⁵-propylimidazo[1,2-a]pyrimidine-5,7-diamine:

TLC: Rf 0.49 (chloroform : methanol = 10 : 1);
¹H-NMR (300MHz, CDCl₃): δ 9.01, 7.79, 7.40, 7.35, 6.97, 6.90, 3.89, 3.81, 3.48-3.60, 3.32-3.42, 3.29, 1.51-1.66,0.89.

### Example 34(5)

### 6-methoxy-N⁵,N⁵-dipropyl-N⁷-(2,4,5-trimethylphenyl)imidazo[1,2-a]pyrimidine-5,7-diamine:

TLC: Rf 0.52 (chloroform : methanol = 10 : 1);
¹H-NMR (300MHz, CDCl₃): δ 8.28, 7.38, 7.21, 7.04, 6.95, 3.82, 3.22-3.33, 2.28, 2.21, 1.54-1.70,0.90.

### Example 34(6)

### N⁷-(2-chloro-4-methoxyphenyl)-6-methoxy-N⁵,N⁵-dipropylimidazo[1,2-a]pyrimidine-5,7-diamine:

TLC: Rf 0.50 (chloroform : methanol = 10: 1);
¹H-NMR (300MHz, CDCl₃): δ 9.00, 7.78, 7.40, 7.24, 6.97, 6.90, 3.86, 3.81, 3.23-3.33, 1.54-1.69, 0.90.

### Example 34(7)

### N⁷-(2,4-dichlorophenyl)-6-methoxy-N⁵,N⁵-dipropylimidazo[1,2-a]pyrimidine-5,7-diamine:

TLC: Rf 0.5 (ethyl acetate : methanol = 10 : 1);
¹H-NMR (300MHz, CDCl₃): δ 9.19, 8.00, 7.45, 7.39, 7.30, 7.27, 3.86, 3.24-3.39, 1.52-1.71, 0.90.

### Example 34(8)

### N⁷-(4-chloro-2-methylphenyl)-6-methoxy-N⁵,N⁵-dipropylimidazo[1,2-a]pyrimidine-5,7-diamine:

TLC: Rf 0.51 (chloroform : methanol = 10 : 1);
¹H-NMR (300MHz, CDCl₃): δ 8.70, 7.40, 7.21-7.26, 7.15-7.18, 3.84, 3.25-3.33, 2.33, 1.55-1.69,0.90.

### Example 34(9)

### 4-{[5-(dipropylamino)-6-methoxyimidazo[1,2-a]pyrimidin-7-yl]amino}-3-ethylbenzonitrile:

TLC: Rf 0.71 (chloroform : methanol = 10 : 1);
¹H-NMR (300MHz, CDCl₃): δ 9.16, 7.55-7.62, 7.45-7.49, 7.28, 3.85, 3.28-3.36, 2.72, 1.56-1.70, 1.36,0.91.

### Example 34(10)

### 6-methoxy-N⁷-(4-methoxy-2-methylphenyl)-N⁵,N⁵-dipropylimidazo[1,2-a]pyrimidine-5,7-diamine:

TLC: Rf 0.42 (chloroform : methanol = 10 : 1);
¹H-NMR (300MHz, CDCl₃): δ 8.28, 7.36, 7.20, 6.95, 6.74-6.85, 3.83, 3.80, 3.22-3.33, 2.32, 1.54-1.70,0.90.

### Example 34(11)

### N⁷-(2-ethyl-4-methylphenyl)-6-methoxy-N⁵,N⁵-dipropylimidazo[1,2-a]pyrimidine-5,7-diamine:

TLC: Rf 0.32 (chloroform : methanol = 10 : 1);
¹H-NMR (300MHz, CDCl₃): δ 8.45, 7.37, 7.21, 7.17, 7.08, 7.02, 3.83, 3.21-3.34, 2.67, 2.33, 1,53-1,70, 1.29, 0.90.

### Example 34(12)

### N⁷-(4-chloro-2-ethylphenyl)-6-methoxy-N⁵,N⁵-dipropylimidazo[1,2-a]pyrimidine-5,7-diamine:

TLC: Rf 0.41 (chloroform : methanol =10:1);
¹H-NMR (300MHz, CDCl₃): δ 8.68, 7.40, 7.20-7.26, 7.17, 3.84, 3.25-3.33, 2.67, 1.54-1.70, 1.31,0.90.

### Example 34(13)

### 6-methoxy-N⁷-(4-methyl-2-vinylphenyl)-N⁵,N⁵-dipropylimidazo[1,2-a]Pyrimidine-5,7-diamine:

TLC: Rf 0.33 (chloroform : methanol = 10 : 1);
¹H-NMR (300MHz, CDCl₃): δ 8.50, 7.38, 7.29, 7.21, 7.18-7.21, 7.15, 6.88, 5-69, 5.43, 3.81, 3.23-3.33, 2.34, 1.54-1-69, 0.90.

### Example 35

### N⁷-mesityl-6-methyl-N⁵,N⁵-dipropylimidazo[1,2-a]pyrimidine-5,7-diamine:

The title compound having the following physical data was obtained by the same procedure as a series of reactions of Example 4, using a compound by the same procedure as a series of reactions of Example 32 → Example 33, and corresponding aniline.
TLC: Rf 0.21 (methylene chloride : methanol = 9 : 1);
¹H-NMR (300MHz, CDCl₃): δ 0.89, 1.54, 2.19, 2.24, 2.29, 3.17, 5.84, 6.90, 7.19, 7.28.

### Example 36

### N-(2-chloro-4-methoxyphenyl)-N-methylguanidine:

2-chloro-4-methoxyphenylmethylaniline (2.0 g) and cyanamide (490 mg) were suspended in water (12 mL). A concentrated hydrochloric acid (0.97 mL) was added to the suspension at room temperature, and then the mixture was stirred with heating for 9 hours at 90°C. After the reaction mixture was cooled, a solid was removed by filtration. The filtrate was concentrated. Ethyl acetate and methanol were added to the residue. The produced solid was collected by filtration and dried under vacuum to give the title compound (1.4 g) having the following physical data.
TLC: Rf 0.03 (chloroform : methanol = 10 : 1);
¹H-NMR (300MHz, CDCl₃): δ 7.46, 7.24, 7.04, 3.81, 3.17.

### Example 37

### N-(2-chloro-4-methoxyphenyl)-N-methyl-6-(methylsulfanyl)-1,3,5-triazine-2,4-diamine

To a suspension of the compound prepared in Example 36 (400 mg) and iminonitrile (233 mg) in water (1.6 mL), an aqueous solution of potassium hydroxide (KOH 180 mg, H₂O 1.6 mL) was added at 40°C. The mixture was stirred for 2 hours. After the reaction mixture was cooled to room temperature, a produced solid was collected by filtration, and dried under vacuum. The solid was dissolved in hexane / ethyl acetate (1 / 1), and it was purified by column chromatography (hexane : ethyl acetate = 1 : 1) to give the title compound (360 mg) having the following physical data.
TLC: Rf 0.47 (hexane : ethyl acetate =1:1);
¹H-NMR (300MHz, CDCl₃):δ 7.27, 7.08, 6.94, 6.50, 3.81, 3.28, 2.09-2.41.

### Example 38

### N-(2-chloro-4-methoxyphenyl)-N-methyl-4-(methylsulfanyl)imidazo[1,2-a][1,3,5]triazine-2-amine:

Water (0.07 mL) and hydrobromic acid (0.07 mL) were added to bromoacetaldehyde dimethyl acetal (291 mg) at room temperature, and the mixture was refluxed with heating for 30 minutes. After the reaction mixture was cooled, dimethoxyethane (1.0 mL) was added to the mixture. And it was neutralized by sodium bicarbonate. The solution was washed with dimethoxyethane and filtrated. To the filtrate, the compound prepared in Example 37 (360 mg) was added at room temperature. The mixture was refluxed with heating for 3.5 hours. After the reaction mixture was cooled, it was concentrated under reduced pressure. The obtained residue was purified by column chromatography (hexane : ethyl acetate = 50 : 50, hexane : ethyl acetate = 0 : 100, dichloromethane : methanol = 20 : 1) to give the title compound (50 mg) having the following physical data.
TLC: Rf 0.21 (hexane : ethyl acetate = 1 : 2);
¹H-NMR (300MHz, CDCl₃): δ 7.3S, 7.20, 7.09, 7.01, 6.85, 3.84, 3.48, 2.22.

### Example 39

### N²-(2-chloro-4-methoxyphenyl)-N²-methyl-N⁴,N⁴-dipropylimidazo[1,2-a][1,3,5]triazine-2,4-diamine:

3-Aminopentane (1.0 mL) was added to the compound prepared in Example 38 (49 mg). The mixture was refluxed with heating for 10 hours. After the reaction mixture was cooled, it was concentrated under reduced pressure. The obtained residue was purified by column chromatography by 100% ethyl acetate to give the title compound (22 mg) having the following physical data.
TLC: Rf 0.25 (ethyl acetate);
¹H-NNM (300MHz, CDCl₃): δ 0.74, 1.53, 3.21, 3.42, 3.80, 6.81, 6.97, 7.07, 7.15, 7.24.

### Example 40

### N²-(2-chloro-4-methoxyphenyl)-N⁴-(1-ethylpropyl)-N²-methylthieno[3,2-d]pyrimidine-2,4-diamine:

The title compound having the following physical data was obtained by the same procedure as a series of reactions of Example 4 using 2-ethylthiothieno[3,2-d]pyrimidin-4-one (it was described in JP 3-17083) → Example 5 → Example 3 using a corresponding amine.
TLC: Rf 0.18 (hexane : ethyl acetate = 4 : 1);
¹H-NMR (300MHz, CDCl₃): δ 0.80, 1.45, 3.44, 3.67, 3.83, 4.26, 6.84, 7.01, 7.22, 7.51.

### Example 40(1)

### N⁷-(2-chloro-4-methoxyphenyl)-N²-methyl-N⁴,N⁴-dipropylthieno[3,2-d]pyrimidine-2,4-diamine:

TLC: Rf 0.36 (hexane : ethyl acetate = 4 : 1);
¹H-NMR (300MHz, CDCl₃) : δ 0.77, 1,50, 3.30, 3,44, 3,81, 6.83, 7.00, 7.22, 7.54.

### Example 41

### N2-(2-chloro-4-methoxyphenyl)-N4-(1-ethylpropyl)-N²-methylthieno[2,3-d]pyrimidine-2,4-diamine:

The title compound having the following physical data was obtained by the same procedure as a series of reactions of Example 4 using 2-ethylthiothieno[2,3-d]pyrimidin-4-one (it was described in US 4,146,716) → Example 5 → Example 3 using a corresponding amine.
TLC: Rf 0.24 (hexane : ethyl acetate = 6 : 1);
¹H-NMR (300MHz, CDCl₃): δ 0.78, 1.43, 3.43, 3.63, 3.83, 4.54, 6.83, 6.91, 7.00, 7.21.

### Example 42

### Ethyl 4-(dipropylamino)-6,7-dihydro-5H-cydopenta[d]pyrimidine-2-carboxylate:

Under argon gas atmosphere, to a solution of the compound prepared by he same procedure as a series of reactions of Example 3 using 2,4-dichloro-6,7-dihydro-5H-cyclopenta[d]pyrimidine, in anhydrous ethanol (1.4 mL) and dimethylformamide (0.7 mL), palladium acetate (8.8 mg), diphenylphosphinoferrocene (22 g) and potassium carbonate (89 mg) were added at room temperature. After carbon dioxide gas displacement, the mixture was refluxed with heating for 1 hour. After the reaction mixture was cooled, a saturated aqueous solution of ammonium chloride was added to stop a reaction. The mixture was extracted with ethyl acetate. The organic layer was washed with water and a saturated aqueous solution of sodium chloride, dried over anhydrous magnesium sulfate and concentrated under reduced pressure. The residue was purified by column chromatography (hexane : ethyl acetate = 1 : 1) to give the title compound (80 mg) having the following physical data.
TLC: Rf 0.26 (hexane : ethyl acetate = 1:1);
¹H-NMR (300MHz, CDC1₃): δ 4,34-4-57, 3.36-3.69, 3.06, 2.94, 1-93-2,22, 1.51-1.78, 1.35-1.52,0.74-1.08.

### Example 43

### (2-chloro-4-methoxypnehyl)[4-(dipropylamino)-6,7-dihydro-5H-cyclopenta[d]pyrimidin-2-yl]methanone:

To a solution of the 3-chloro-4-bromoanisol (1.0 mg) in tetrahydrofuran (2.3 mL), isopropyl magnesium bromide (2.3 mL, 2.0M tetrahydrofuran solution) was dropped at room temperature. The mixture was stirred for 2 hours. The solution was added to a solution of the compound (190 mg) prepared in Example 42 in tetrahydrofuran (3.0 mL) at -30°C. The mixture was stirred for 30 minutes at -10°C. A saturated aqueous solution of ammonium chloride was added to the reaction mixture to stop the reaction. The mixture was extracted with ethyl acetate. The organic layer was washed with a saturated aqueous solution of sodium chloride, dried over anhydrous magnesium sulfate and concentrated under reduced pressure. The obtained residue was purified by column chromatography (hexane : acetone = 2 : 1) to give the title compound (84.2 mg) having the following physical data.
TLC: Rf 0.27 (hexane : acetone =3:1);
¹H-NMR (300MHz, CDCl₃): δ 0.76, 1.42-1.58, 1.95-2.18, 2.9G, 3.06, 3.22-3.40, 3.84, 6.85, 6.90, 7.55.

### Example 44

### 1-(2-chloro-4-methoxyphenyl)cyclopropanecarbonitrile:

To a suspension of sodium hydride (1.0 g) in dimethylformamide (16.0 mL), a solution of 2-chloro-4-methoxyphenylacetonitrile (2.0 g) in dimethylformamide (10.0 mL) was dropped at 0°C. The mixture was stirred for 1 hour at room temperature. To the reaction mixture, 1,2-dibromoethane (1.1 mL) was dropped at 0°C. The mixture was stirred for 20 hours at room temperature. The reaction mixture was cooled to 0°C, and water was added to stop a reaction to the reaction mixture. The mixture was extracted three times with a mixture of hexane and ethyl acetate (4 : 1). The combined organic layer was washed with water and a saturated aqueous solution of sodium chloride, dried over anhydrous magnesium sulfate and concentrated under reduced pressure. The obtained residue was purified by column chromatography (hexane : ethyl acetate = 8 : 1, hexane : ethyl acetate = 4 : 1) to give the title compound having the following physical data.
TLC: Rf 0.35 (hexane : ethyl acetate =4:1);
¹H-NMR (300MHz, CHCl₃-D): δ 7.25, 6.97, 6.78, 3.80, 1,60-1,80, 1,17-1.40.

### Example 45

### 1-(2-chloro-4-methoxyphenyl)cyclopropanimidocarboxylic acid amide:

To a suspension of ammonium chloride (433 mL) in anhydrous toluene (4.0 mL), trimethyl aluminum (3.9 mL, 2.0M toluene solution) was added under ice-bath. The mixture was stirred for 2 hours at room temperature. To the mixture, a solution of the compound (800 mg) prepared in Example 44 in toluene (3.8 mL) was added. The mixture was stirred for 2 days at 80°C. After the reaction mixture was cooled, it was poured into a suspension of silica gel (3.0 g) in chloroform (10 mL) slowly, and stirred for 10 minutes. The reaction mixture was filtered through celite and the filtrate was concentrated under reduced pressure. To the residue, 2N hydrochloric acid (5.0 mL) was added, and it was decanted with diethyl ether. The water layer was neutralized by adding 5N aqueous solution of sodium hydroxide (3.0 mL), and then sodium chloride was added. The solution was extracted with three times with tetrahydrofuran. The organic layer was dried over anhydrous magnesium sulfate and concentrated under reduce pressure to give the title compound (740 mg) having the following physical data.
TLC: Rf 0.17 (methylene chloride : methanol = 9 : 1);
¹H-NMR (300MHz, CHCl₃-D): δ 7.38, 7.04, 6.90, 3.76, 1.39-1.74, 0.91-1.24.

### Example 46

### 2-[1-(2-chloro-4-methoxyphenyl)cyclopropyl-6,7-dihydro-5H-cydopenta[d]pyrimidin-4-ol:

Sodium hydroxide (163 mg) was dissolved in ethanol (3.7 mL) at room temperature, and the compound (640 mg) prepared in Example 45 and ethyl 2-oxycyclopentanecarbonate (0.78 mL) were added to the solution. The mixture was refluxed with heating for 5 hours. After the reaction mixture was cooled, water was added to the mixture and the then the mixture was neutralized by adding 1N hydrochloric acid. The mixture was extracted with ethyl acetate. The organic layer was washed with a saturated aqueous solution of sodium chloride, dried over anhydrous magnesium sulfate and concentrated under reduced pressure. Hexane was added to the obtained residue, and the obtained solid was collected by filtration and dried to give the title compound (630 mg) having the following physical data.
TLC: Rf0.43 (hexane : ethyl acetate = 1 : 2);
¹H-NMR (300MHz, CHCl₃-D): δ 7.34, 7.02, 6.87, 3.85, 2.64-2.96, 1.98-2.17, 1.81-1.96, 1.30-1.43.

### Example 47

### 4-chloro-2-[1-(2-chloro-4-methoxyphenyl)cyclopropyl-6,7-dihydro-5H-cyclopenta[d]pyrimidine:

To the compound prepared in Example 46 (600 mg), phosphoryl chloride (2.0 mL) was added. The mixture was refluxed with heating for 20 minutes. After the reaction mixture was cooled, it was poured into an ice-bath. The mixture was extracted with ethyl acetate. The organic layer was washed with a saturated aqueous solution of sodium bicarbonate and a saturated aqueous solution of sodium chloride, dried over anhydrous magnesium sulfate and concentrated under reduced pressure to give the title compound (660 mg) having the following physical data.
TLC: Rf 0.57 (hexane : ethyl acetate =5:1);
¹H-NMR (300MHz, CHCl₃-D): δ 7.30, 6.95, 6.80, 3.81, 2.77-3.01, 1.98-2.19, 1.74-1.88, 1.27-1.43.

### Example 48

### 2-[1-(2-chloro-4-methoxyphenyl)cyclopropyl]-N,N-dipropyl-6,7-dihydro-5H-cyclopenta[d]pyrimidine-4-amine:

A solution of the compound prepared in Example 47 (300 mg) in 3-aminopentane (1.0 mL) was refluxed with heating for 24 hours. After the reaction mixture was cooled, it was poured into water. The mixture was extracted with ethyl acetate. The organic layer was washed with a saturated aqueous solution of sodium chloride, dried over anhydrous magnesium sulfate and concentrated under reduced pressure. The obtained residue was purified by column chromatography (hexane : ethyl acetate = 5 : 1) to give the title compound (152 mg) having the following physical data.
TLC: Rf 0.35 (hexane : ethyl acetate =5:1);
¹H-NMR (300MHz, CHCl₃-D): δ 0,71, 1.21, 1.39, 1.74, 1.94, 2.74, 2.92, 3.15, 3.78, 6.75, 6.91, 7.27.

### Example 49

### methyl 4-(2-chloro-4-methoxyphenyl)-2-methyl-3-oxobutanoate:

Under argon gas atmosphere, a solution of zinc powder (1.81 g) in tetrahydrofuran (16 mL) was refluxed. To the mixture, methyl 2-bromopropionate (4 drops) was dropped, and 2-chloro-4-methoxyphenylacetnitrile (1.0 g) was added and then methyl 2-bromopropionate (2.46 mL) was dropped. The mixture was refluxed for 10 minutes. After the reaction mixture was cooled, it was diluted with tetrahydrofuran. A 50% aqueous solution of potassium carbonate was added to the diluted solution, and the mixture was stirred for 30 minutes. The reaction mixture was filtered, and washed with tetraliydrofuran. 2N hydrochloric acid (6 mL) was added to the filtrate. The mixture was stirred for 30 minutes and concentrated. The residue was purified by column chromatography on silica gel (hexane : ethyl acetate = 92 : 8 → 71 : 29) to give the title compound (1.22 g) having the following physical data.
TLC: Rf 0.55 (hexane : ethyl acetate = 2: 1).

### Example 50

### 5-(2-chloro-4-methoxybenzyl)-6-methylpyrazolo[1,5-a]pyrimidin-7-ol:

A solution of 3-aminopyrazole (272 mg) and the compound of Example 49 (886 mg) in acetic acid (4.0 mL) was refluxed for 2 hours. After the reaction mixture was cooled, it was diluted with ethyl acetate. The product was collected by filtration to give the title compound (421 mg) having the following physical data.
TLC: Rf 0.52 (chloroform : methanol = 10: 1).

### Example 51

### 7-chloro-5-(2-chloro-4-methoxybenzyl)-6-methylpyrazolo[1,5-a]pyrimidine:

Under argon gas atmosphere, to a solution of the compound prepared in Example 50 (511 mg) in toluene (5.0 mL), diethylaniline (270 µL) and phosphoryl chloride (776 mg) were added. The mixture was refluxed for 2.5, hours. After the reaction mixture was cooled, it was poured into an ice-water and a saturated aqueous solution of sodium bicarbonate was added to the solution. The solution was extracted with ethyl acetate. The extracted solution was washed with water and a saturated aqueous solution of sodium chloride, dried over anhydrous magnesium sulfate and concentrated. The residue was purified by column chromatography on silica gel (hexane : ethyl acetate = 92 : 8 → 71 : 29) to give the title compound (528 mg) having the following physical data.
TLC: Rf0.54 (hexane : ethyl acetate = 2 : 1);
¹H-NMR (300MHz, CHCl₃-D): δ 8.12, 6.98, 6.94, 6.72, 6.68, 4.28, 3.79, 2.35.

### Example 52

### 5-(2-chloro-4-methoxybenzyl)-6-methyl-N-(1-ethylpropyl)pyrazolo[1, 5-a]pyrimidine-7-amine:

The title compound (175 mg) having the following physical data was obtained by the same procedure as a series of reactions of Example 3 using the compound prepared in Example 51 (150 mg) and 3-aminopentane (220 µL).
TLC: Rf 0,57 (hexane : ethyl acetate = 2 : 1);
¹H-NMR (300MHz, CDCl₃): δ 7.95, 6.96, 6.89, 6.67, 6.44, 6.02, 4.20, 3.82-3.95, 3.77, 2.16, 1.49-1.72, 0.93.

### Example 52(1)-Example 52(4)

The following compounds were obtained by the same procedure as a series of reactions of Example 49 → Example 50 → Example 51 → Example 52, using a corresponding compound.

### Example 52(1)

### 5-[1-(2-chloro-4-methoxyphenyl)cyclopropyl]-N,N-dipropylpyrazolo[1,5-a]pyrimidine-7-amine:

TLC: Rf 0.59 (hexane : ethyl acetate = 3 : 1);
¹H-NMR (30OMHz, CDCl₃): δ 0.78, 1.25-1.33, 1.46-1.65, 1.85-1.94, 3.42-3.58, 3.83, 5.39, 6.33, 6.85, 6.99, 7.38, 7.91.

### Example 52(2)

### 5-(2-chloro-4-methoxybenzyl)-6-methyl-N,N-dipropylpyrazolo[1,5-a]pyrimidine-7-amine:

TLC: Rf 0.64 (hexane : ethyl acetate = 3 : 1);
¹H-NMR (300MHz, CDCl₃): δ 8.01, 6.97, 6.91, 6.69, 6.54, 4.22, 3.78, 3.32-3.42, 2.17, 1.37-1.54, 0.75-0.87.

### Example 52(3)

### 5-mesitylmethyl-6-methyl-N,N-dipropylpyrazol[1,5-a]pyrimidine-7-amine:

TLC: Rf0.64 (hexane : ethyl acetate = 5 : 1);
¹H-NMR (300MHz, CDCl₃): δ 0.84, 1.40-1.55, 2.18, 2.30, 2.36, 3.32-3.42, 4.09, 6.40, 6.89, 7.92.

### Example 52(4)

### 5-(2,4-dichlorobenzyl)-6-methyl-N,N-dipropylpyrazolo[1,5-a]pyrimidine-7-amine:

TLC: Rf 0.63 (hexane : ethyl acetate = 4: 1);
¹H-NMR (300 MHz, CDCl₃): δ 0.82, 1.38-1.55, 2.18, 3.29-3.48, 4.24, 6.53, 6.97, 7.13, 7.43, 8.01.

### Example 53:

### 5-(4-chloro-2-methylphenoxy)-6-methyl-N,N-dipropylpyrazolo[1,5-a]pyrimidine-7-amine:

To a solution of 5-chloro-6-methyl-N,N-dipropylpyrazolo[1,5-a]pyrimidine-7-amine (150 mg) in dimethylformamide (3.0 mL), 4-chloro-o-cresol (96 mg) and cesium carbonate (276 mg) were added. The mixture was stirred for 5 hours at 80°C. The reaction mixture was diluted with ethyl acetate. The diluted solution was washed with water and a saturated aqueous solution of sodium chloride, dried over anhydrous magnesium sulfate and concentrated. The residue was purified by column chromatography on silica gel (hexane : ethyl acetate = 100 : 0 → 95 : 5) to give the title compound (190 mg) having the following physical data.
TLC: Rf 0.60 (hexane : ethyl acetate = 7:1);
¹H-NMR (300MHz, CDCl₃): δ 7.87, 7.24-7.28, 7.18-7.24, 7.07, 6.21, 3.40-3.48, 2.33, 2.16, 1.46-1.61, 0.88.

### Example 53(1)-Example 53(2)

The following compounds were obtained by the same procedure as a series of reactions of Example 53, using a corresponding compound.

### Example 53(1)

### 5-(4-ethyl-2-methoxyphenoxy)-6-methyl-N,N-dipropylpyrazolo[1,5-a]pyrimidine-7-amine :

The title compound having the following physical data was obtained by the same procedure as a series of reactions of Example 53, using a corresponding compound.
TLC: Rf 0.36 (hexane : ethyl acetate =7:1);
¹H-NMR (300MHz, CDCl₃): δ 0.87, 1.28, 1.45-1.60, 2.35, 2.69, 3.37-3.47, 3.75, 6.20, 6.80-6.87, 7.07, 7.85.

### Example 53(2)

### 5-[(3-chloro-1,1'-biphenyl-4-yl)oxy]-6-methyl-N,N-dipropylpyrazolo[1,5-a]pyrimidine-7-amine:

TLC: Rf 0.40 (hexane : ethyl acetate = 10 : 1);
¹H-NMR (300 MHz, CDCl₃): δ 0.88, 1.48-1.62, 2.39, 3.42-3.50, 6.24, 7.32- 7.41, 7.41-7.50, 7.50-7.64, 7.70, 7.88.

### Pharmacological Activities

The compound of the present invention of formula (I) possesses CRF receptor antagonistic activity, for example, such an effect of the compound of the present invention was confirmed by following tests.

### Experiment 1

### Binding assay:

### <Cell membrane preparation>

After the cell line expressing human CRF receptor 1 (expressed cell line: CHO-K1 cells) was cultured to reached confluence, the cells were harvested with a scraper. Harvested cells were washed twice with PBS before being suspended in binding assay buffer (Tris-HCl (50 mM, pH 7.0), EDTA (2 mM, pH8.0), MgCl₂ (10 mM)) cooled by ice. Suspended cells were homogenized with a Downs-type homogenizer and subjected to centrifugation at 10,000g to collect the membrane fraction. The harvested cell membrane fraction was re-suspended with a small quantity of the binding assay buffer, and further diluted with said buffer to 1 mg/mL. The membrane fraction thus obtained was used for binding assay.

### <Binding assay>

Fifty µL of [¹²⁵I] h/r CRF prepared to 0.5 nM with binding assay buffer was added to siliconized 1.5 mL tubes. 1 µL of compounds diluted in appropriate multiples, DMSO (for total binding use), or h/r CRF solution (100 µM, for the non-specific binding use), respectively, added to the tubes. Samples of 50 µL each of the membrane fraction preparation were added to the tubes to initiate the reaction (final concentration of [¹²⁵I] h/r CRF: 0.25 nM), then the mixtures were incubated for 2 hours at room temperature. After termination of the reaction, tubes were subjected to centrifugation at 20,000g to collect the membrane fraction. The supernatant was discarded, and the pellet was rinsed twice with cooled PBS (-) containing 0.01% Triton X-100. Radioactivity values of the respective tubes were measured with a γ-counter.

The specific binding was derived by subtracting the non-specific binding value from the each binding value.

The results indicated that these compounds of the present invention exhibited potent affinity on CRF receptor (IC₅₀: < 1 µM).

### Experiment 2

### Receptor antagonistic activity (cyclic AMP assay):

The cell line expressing human CRF1 receptor was cultured using 10% bovine embryo serum and 1% F-12 nutrient mixture containing antibiotics and antifungal under 37°C, 5% carbonic anhydride, 95% air. On the day before a measurement of cyclic AMP, the cell seed to 96-well plate to be 1×10⁴ cell/well. On the measurement day, the cell was washed twice with F-12 nutrient mixture, and F-12 nutrient mixture / 1mM 3-isobutyl-1-methylxanthin (assay medium) (178 µL) was added to each well. After they were incubated for 10 minutes at 37°C, various concentrated solution of the test compound (2 µL) was added, or DMSO (2 µL) to CRF group and blank group was added. After they were incubated for 15 minutes at 37°C, 10 nM assay medium containing human / rat CRF (20 µL) was added to the test compound group and CRF group. To blank group, assay medium containing 0.00001% acetic acid (20 µL) was added. Furthermore, they were incubated for 15 minutes at 37°C. A supernatant was removed, and the reaction was stopped by cooling using ice. Also, all reaction was carried out by 3 wells. The cumulative dosage of intracellular cyclic AMP was measured by Biotrak enzyme immunoassay system (Amersham Biosciences). The cumulative dosage of cyclic AMP was derived by subtracting the average value of 3 wells of blank group from the average value of 3 wells. The IC₅₀ values calculated by nonlinear regression analysis with logarithm concentrate of the compound as the autonomous variable and cyclic AMP cumulative dosage as an induced variable

The results indicated that compound (1) exhibited potent antagonist activity on CRF receptor (IC₅₀: < 1 µM).

### Formulation example 1

The following components were admixed in conventional method and punched out to obtain 10,000 tablets each containing 10 mg of active ingredient.

| | |
|---|---|
| N²-(2-chloro-4-methoxyphenyl)-N⁴,N⁴-dipropyl-5,7- dihydrofuro[3,4-d]pyrimidine-2,4-diamine | 100 g |
| Carboxymethylcellulose calcium (disintegrating agent) | 20 g |
| Magnesium stearate (lubricating agent) | 10 g |
| Microcrystalline cellulose | 870 g |

### Formulation example 2

The following components were admixed in conventional method. The solution was sterilized in conventional manner, filtered through dust removal equipment, placed 5 ml portions into ampoules and sterilized by autoclave to obtain 10,000 ampoules each containing 20 mg of the active ingredient.

| | |
|---|---|
| N²-(2-chloro-4-methoxyphenyl)-N⁴,N⁴-dipropyl-5,7- dihydrofuro[3,4-d]pynmidine-2,4-diamine | 200 g |
| mannitol ¥ | 20 g |
| distilled water | 50 L |

### Industrial Applicability

The compound of the present invention is useful, in order to bind a CRF receptor and show a CRF receptor antagonistic activity, for the prevention and/or treatment of CRF mediated diseases, for example, neuropsychiatric disorders, digestive diseases.

## Claims

1. A CRF antagonist comprising, as an active ingredient, a compound represented by formula (I): 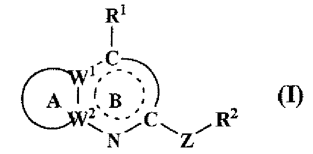 wherein ring A represents a 5- or 6-membered monocyclic ring which may be substituted with 1 to 3 substituents selected from a halogen atom, CF₃, OCF₃, hydroxyl, mercapto, carboxyl, (C1-6 alkoxy)carbonyl, carbamoyl, nitro, cyano, oxo, and C1-6 alkyl, C2-6 alkenyl, C2-6 alkynyl, C1-6 alkoxy or C1-6 alkylthio which each may be substituted with 1 to 3 substituents selected from a halogen atom, CF₃ and hydroxyl;
ring B represents a 5- to 7-membered monocyclic unsaturated heterocyclic ring which may contain 1 or 2 hetero atoms selected from a nitrogen atom, an oxygen atom and/or a sulfur atom which may be oxidized, other than the nitrogen atom, W¹ and W² and which may be further substituted;
W¹ and W² each independently represents a carbon atom or a nitrogen atom;
Z represents -NR³-, in which R³ represents a hydrogen atom, C1-6 alkyl, C2-6 alkenyl or C2-6 alkynyl which each may be substituted, -CO-(C1-6 alkyl which may be substituted), -SO₂-(C1-6 alkyl which may be substituted), an oxygen atom, a sulfur atom which may be oxidized, or -CR⁴R⁵-, in which R⁴ and R⁵ each independently represents a hydrogen atom, C1-5 alkyl, C2-6 alkenyl or C2-6 alkynyl which each may be substituted, or R⁴ and R⁵ may be taken together to represent (i) oxo, (ii) C2-5 alkylene in which one carbon atom may be substituted with one oxygen atom, nitrogen atom or sulfur atom which may be oxidized, wherein the C2-5 alkylene may be substituted with a substituent(s), or (iii) C1-6 alkylidene which may be substituted;
R¹ represents:
(i) C1-15 alkyl, C2-15 alkenyl or C2-15 alkynyl which each may be substituted,
(ii) amino which may be protected,
(iii) hydroxyl which may be protected,
(iv) mercapto which may be protected,
(v) -S(O)ₙR⁶, in which n represents 1 or 2, and R⁶ represents (a) C1-15 alkyl, C2-15 alkenyl or C2-15 alkynyl which each may be substituted or (b) a cyclic group which may be substituted,
(vi) -COR⁷, in which R⁷ represents (a) a hydrogen atom, (b) C1-15 alkyl, C2-15 alkenyl or C2-15 alkynyl which each may be substituted, (c) hydroxyl which may be protected, (d) amino which may be protected, or (e) a cyclic group which may be substituted, or
(vii) a cyclic group which may be substituted;
R² represents an unsaturated cyclic group which may be substituted, in which the substituent may be taken together with R³ to form C2-5 alkylene which may be substituted,
a salt thereof, an N-oxide thereof, a solvate thereof or a prodrug thereof.

2. A compound represented by formula (I-A): 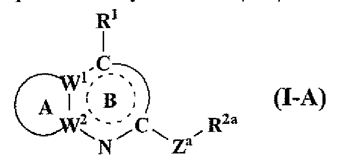 wherein 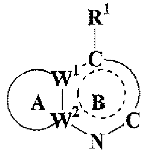 represents a ring selected from
(1) cyclic group 1: 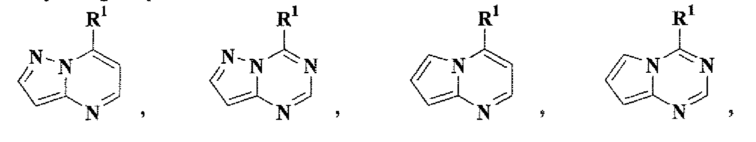 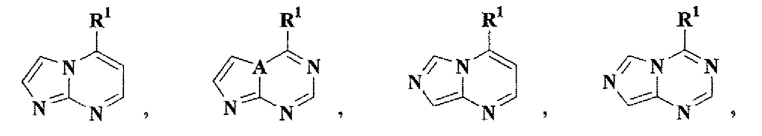 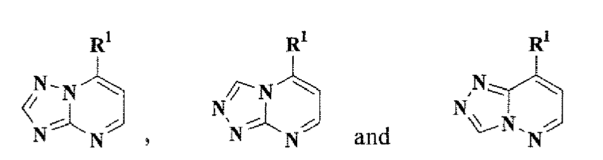 and
(2) cyclic group 2: 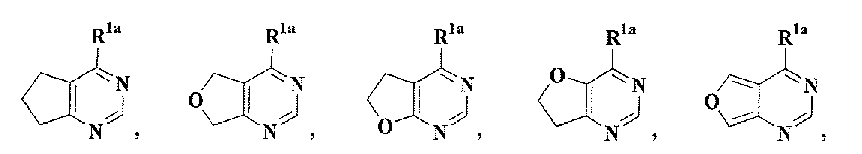 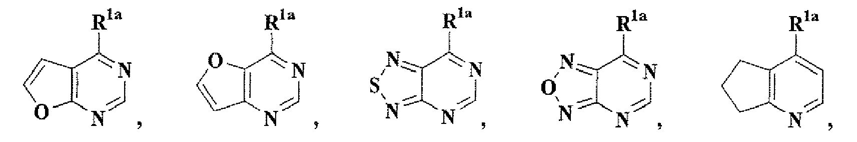 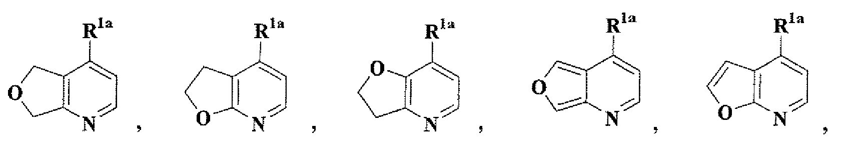 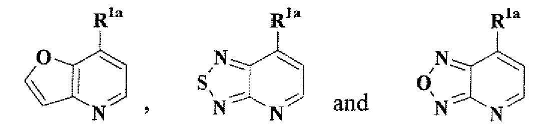 and ring A may be substituted with 1 to 3 substituents selected from a halogen atom, CF₃, OCF₃, hydroxyl, mercapto, carboxyl, (C1-6 alkoxy)carbonyl, carbamoyl, nitro, cyano, oxo, and C1-6 alkyl, C2-6 alkenyl, C2-6 alkynyl, C1-6 alkoxy or C1-6 alkylthio which each may be substituted with 1 to 3 substituents selected from a halogen atom, CF₃ and hydroxyl, and ring B may be further substituted;
R¹ represents:
(i) C1-15 alkyl, C2-15 alkenyl or C2-15 alkynyl which each may be substituted,
(ii) amino which may be protected,
(iii) hydroxyl which may be protected,
(iv) mercapto which may be protected,
(v) -S(O)ₙR⁶, in which n represents 1 or 2, and R⁶ represents (a) C1-15 alkyl, C2-15 alkenyl or C2-15 alkynyl which each may be substituted, or (b) a cyclic ring which may be substituted,
(vi) -COR⁷, in which R⁷ represents (a) a hydrogen atom, (b) C1-15 alkyl, C2-15 alkenyl or C2-15 alkynyl which each may be substituted, (c) hydroxyl which may be protected, (d) amino which may be protected, or (e) a cyclic group which may be substitute, or
(vii) a cyclic group which may be substituted;
R^{1a} represents:
(i) C1-15 alkyl or C2-15 alkenyl which may be substituted with substituent group 1,
(ii) NR⁸R⁹, in which R⁸ represents (a) a hydrogen atom or (b) C1-15 alkyl or C2-15 alkenyl which each may be substituted with substituent group 1, and R⁹ represents (a) a hydrogen atom, (b) C1-15 alkyl or C2-15 alkenyl substituted with substituent group 1, (c) -COR¹⁰, in which R¹⁰ represents (aa) a hydrogen atom or (bb) C1-15 alkyl or C2-15 alkenyl which each may be substituted with substituent group 1, (d) -COOR¹⁰, in which R¹⁰ has the same meaning as described above, or (e) -CON(R⁸)₂, in which R⁸s each independently has the same meaning as described above,
(iii) OR¹⁰, in which R¹⁰ has the same meaning described above,
(iv) SR¹⁰, in which R¹⁰ has the same meaning described above,
(v) S(O)ₙR¹¹, in which n represents 1 or 2, and R¹¹ represents C1-15 alkyl or C2-15 alkenyl which each may be substituted with substituent group 1, or
(vi) COR¹², in which R¹² represents (a) a hydrogen atom, (b) C1-15 alkyl or C2-15 alkenyl which each may be substituted with substituent group 1, (c) -OR¹⁰, in which R¹⁰ has the same meaning as described above, or (d) -NR⁸R⁹, in which R⁸ and R⁹ have the same meanings as described above;
the substituent group 1 represents (1) a halogen atom, (2) CF₃, (3) OCF₃, (4) cyano, (5) nitro, (6) hydroxyl, (7) C1-6 alkoxy, (8) carboxyl, (9) (C1-6 alkoxy)carbonyl, (10) C1-5 acyl, (11) carbamoyl in which a nitrogen atom may be protected with 1 or 2 of C1-6 alkyl, (12) C1-6 alkylthio, (13) C1-6 alkylsulfonyl, or (14) NR¹³R¹⁴, in which R¹³ represents (a) a hydrogen atom, (b) C1-6 alkyl, or (c) C2-6 alkenyl, and R¹⁴ represents (a) a hydrogen atom, (b) C1-6 alkyl, (c) C2-6 alkenyl, (d) -COR¹⁵, in which R¹⁵ represents (aa) a hydrogen atom, (bb) C1-6 alkyl or (cc) C2-6 alkenyl, (e) -COOR¹⁵, in which R¹⁵ has the same meaning as described above, or (f) -CON(R¹⁶)₂, in which R¹⁶s each independently represents a hydrogen atom or C1-6 alkyl;
Z^{a} represents -NR³-, in which R³ represents a hydrogen atom, C1-6 alkyl, C2-6 alkenyl or C2-6 alkynyl which each may be substituted, -CO-(C1-6 alkyl which may be substituted), -SO₂-(C1-6 alkyl which may be substituted), an oxygen atom, a sulfur atom which may be oxidized, or -CR⁴R⁵-, in which R⁴ and R⁵ each independently represents a hydrogen atom, or C1-6 alkyl, C2-6 alkenyl or C2-6 alkynyl which each may be substituted, or R⁴ and R⁵ may be taken together to represent (i) oxo, (ii) C2-5 alkylene in which one carbon atom may be substituted with one oxygen atom, nitrogen atom or sulfur atom which may be oxidized, wherein the C2-5 alkylene may be substituted with a substituent(s), or (iii) C1-6 alkylidene which may be substituted;
R^{2a} represents (1) a C5-12 monocyclic or bicyclic unsaturated carbocyclic ring which may be substituted, (2) pyridine which may be substituted, (3) a bicyclic heterocyclic ring which may be substituted, in which benzene and a 5- or 6-membered monocyclic heterocyclic ring are fused, (4) a bicyclic heterocyclic ring which may be substituted, in which a pyridine ring and a C5-6 monocyclic carbocyclic ring are fused, or (5) a bicyclic heterocyclic ring which may be substituted, in which a pyridine ring and a 5-or 6-membered monocyclic heterocyclic ring are fused,
a salt thereof, an N-oxide thereof, a solvate thereof or a prodrug thereof

3. The compound according to claim 2,
wherein the ring 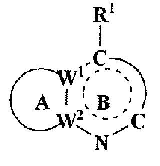 is 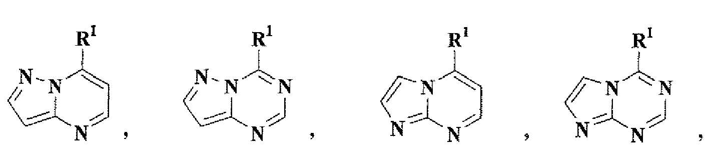 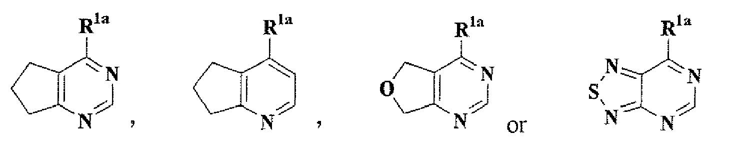 wherein all symbols have the same meanings as described in claim 2,
a salt thereof, an N-oxide thereof, a solvate thereof or a prodrug thereof.

4. The compound according to claim 2, wherein R¹ is amino which may be protected, or R^{1a} is NR⁸R⁹, in which R⁸ and R⁹ have the same meanings as described in the claim 2, a salt thereof an N-oxide thereof, a solvate thereof or a prodrug thereof

5. The compound according to claim 2, wherein Z^{a} is -NR³-, in which R³ has the same meaning as described in claim 2, a salt thereof an N-oxide thereof, a solvate thereof or a prodrug thereof.

6. The compound according to claim 2, wherein Z^{a} is -CR^{4b}R^{5b}-, in which R^{4b} and R^{5b} are taken together to represent C2-5 alkylene in which one carbon atom may be substituted with one oxygen atom, nitrogen atom or sulfur atom which may be oxidized, wherein the C2-5 alkylene may be substituted with a substituent(s), a salt thereof, an N-oxide thereof, a solvate thereof or a prodrug thereof

7. The compound according to claim 2, which is represented by formula (I-A-3): 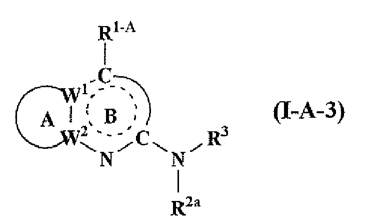 wherein 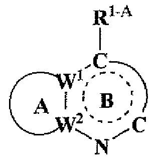 is 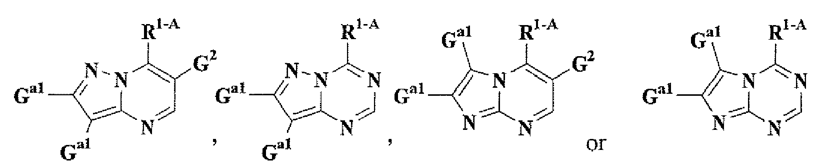 R^{1-A} represents amino which may be protected with 1 or 2 of C1-15 alkyl which may be substituted;
G^{al}s each independently represents a hydrogen atom, a halogen atom, CF₃, OCF₃, hydroxyl, mercapto, carboxyl, (C1-6 alkoxy)carbonyl, carbamoyl, nitro, cyano, or C1-6 alkyl, C2-6 alkenyl, C2-6 alkynyl, C1-6 alkoxy or C1-6 alkylthio which each may be substituted with 1 or 2 substituents selected from a halogen atom, CF₃ and hydroxyl;
G² represents a hydrogen atom, C1-15 alkyl, C2-15 alkenyl or C2-15 alkynyl which may be substituted, hydroxyl which may be protected, cyclopropane, cyclobutane, cyclopentane, cyclohexane, phenyl, a halogen atom, CF₃, or cyano; and other symbols have the same meanings as in claim 2,
a salt thereof, an N-oxide thereof, a solvate thereof or a prodrug thereof

8. The compound according to claim 2, which is represented by formula (I-A-4): 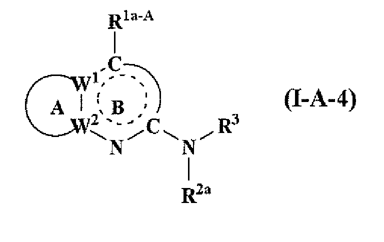 wherein 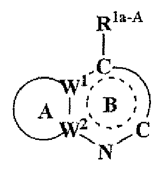 is 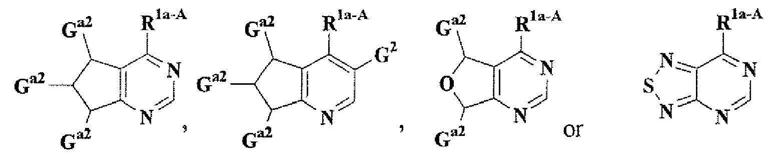 R^{1a-A} represents NR^{8A}R^{9A}, in which one of R^{8A} and R^{9A} represents C1-15 alkyl which may be substituted with the substituent group 1 and another represents a hydrogen atom or C1-15 alkyl which may be substituted with the substituent group 1, wherein the substituent group 1 has the same meaning as in claim 2;
G^{a2}s each independently represents a hydrogen atom, a halogen atom, CF₃, OCF₃, hydroxyl, mercapto, carboxyl, (C1-6 alkoxy)carbonyl, carbamoyl, nitro, cyano, oxy, oxo, or C1-6 alkyl, C2-6 alkenyl, C2-6 alkynyl, C1-6 alkoxy or C1-6 alkylthio which each may be substituted with 1 or 2 substituents selected from a halogen atom, CF₃ and hydroxyl; and other symbols have the same meanings as described in claim 2 or 7, a salt thereof, an N-oxide thereof, a solvate thereof or a prodrug thereof.

9. The compound according to claim 2, which is:
(1) N⁵-(2-chloro-4-methoxyphenyl)-6-methyl-N⁷,N⁷-dipropylpyrazolo[1,5-a]pyrimidine-5,7-diamine,
(2) N⁵-(2-chloro-4-methoxyphenyl)-N⁷-(1-ethylpropyl)-6-methylpyrazolo[1,5-a]pyrimidine-5,7-diamine,
(3) N⁵-(2-chloro-4-methoxyphenyl)-6-ethyl-N⁷,N⁷-dipropylpyrazolo[1,5-a]pyrimidine-5,7-diamine,
(4) N²-(2-chloro-4-methoxyphenyl)-N²-ethyl-N⁴,N⁴-dipropyl-6,7-dihydro-5H-cyclopenta[d]pyrimidine-2,4-diamine,
(5) N⁵-(2-chloro-4-methoxyphenyl)-6-methoxy-N⁷,N⁷-dipropylpyrazolo[1,5-a]pyrimidine-5,7-diamine,
(6) N²-allyl-N²-(2-chloro-4-methoxyphenyl)-N⁴,N⁴-dipropyl-6,7-dihydro-5H-cyclopenta[d]pyrimidine-2,4-diamine,
(7) 6-methyl-N⁵-[2-methyl-4-(trifluoromethoxy)phenyl]-N⁷,N⁷⁻dipropylpyrazolo[1,5-a]pyrimidine-5,7-diamine,
(8) N⁷-butyl-N⁵-(2-chloro-4-methoxyphenyl)-N⁷-ethyl-6-methylpyrazolo[1,5-a]pyrimidine-5,7-diamine,
(9) N⁵-(2-ethyl-4-methylphenyl)-6-methyl-N⁷,N⁷-dipropylpyrazolo[1,5-a]pyrimidine-5,7-diamine,
(10) 6-methoxy-N⁵-(4-methyl-2-vinylphenyl)-N⁷,N⁷-dipropylpyrazolo[1,5-a]pyrimidine-5,7-diamine, or
(11) N⁵-(2-ethyl-4-methylphenyl)-6-methoxy-N⁷,N⁷-dipropylpyrazolo[1,5-a]pyrimidine-5,7-diamine.

10. A pharmaceutical composition comprising, as an active ingredient, the compound represented by formula (I-A) according to claim 2, a salt thereof, an N-oxide thereof, a solvate thereof or a prodrug thereof.

11. The pharmaceutical composition according to claim 10, which is a CRF antagonist.

12. The pharmaceutical composition according to claim 10, which is an agent for preventing and/or treating CRF mediated diseases.

13. The pharmaceutical composition according to claim 12, wherein the CRF mediated diseases are psychiatric and neurologic disorders or digestive diseases.

14. The pharmaceutical composition according to claim 13, wherein the psychiatric and neurologic disorders or the digestive diseases are mood disorders, anxiety disorders, stress-related disorders, eating disorders, symptom caused by psychotropic substance or dependency thereon, organic mental disorder, schizophrenic disorder, attention-deficit hyperactivity disorder or irritable bowel syndrome.

15. The pharmaceutical composition according to claim 14, wherein the psychiatric and neurologic disorders or the digestive diseases are depression, mood disorders, eating disorders, drug addiction, drug dependency or irritable bowel syndrome.

16. A medicament comprising a combination of the compound represented by formula (I-A) according to claim 2, a salt thereof, an N-oxide thereof, a solvate thereof or a prodrug thereof with at least one selected from a tricyclic antidepressant, a tetracyclic antidepressant, a monoamine oxidase inhibitor, a serotonin and noradrenaline reuptake inhibitor, a selective serotonin reuptake inhibitor, a serotonin reuptake inhibitor, a psychoanaleptic, an antianxiety agent, an antipsychotic agent, a mitochondrial benzodiazepine receptor ligand, an NK1 antagonist, a gastrointestinal promotility agent, a 5-HT₃ antagonist, a 5-HT₄ agonist, an anticholinergic agent, an antidiarrheal drug, a lapactic and an autonomic modulating agent.

17. A method for antagonizing CRF, which comprises administering to a mammal an effective amount of the compound represented by formula (I), a salt thereof, an N-oxide thereof, a solvate thereof or a prodrug thereof: 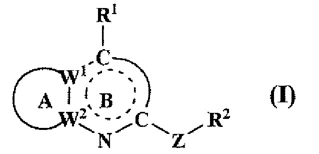 wherein all symbols have the same meanings as described in claim 1.

18. A method for preventing and/or treating a CRF mediated disease, which comprises administering to a mammal an effective amount of the compound represented by formula (I-A), a salt thereof, an N-oxide thereof, a solvate thereof or a prodrug thereof: 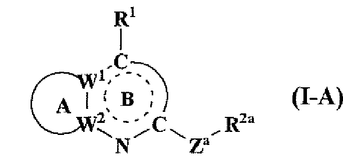 wherein all symbols have the same meanings as described in claim 2.

19. Use of the compound represented by formula (I), a salt thereof, an N-oxide thereof, a solvate thereof or a prodrug thereof for the manufacture of a CRF antagonist: 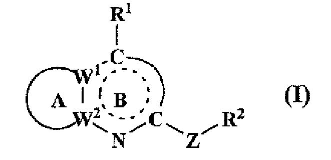 wherein all symbols have the same meanings as described in claim 1.

20. Use of the compound represented by formula (I), a salt thereof, an N-oxide thereof, a solvate thereof or a prodrug thereof for the manufacture of a CRF mediated disease: 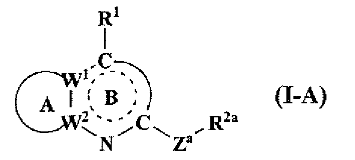 wherein all symbols have the same meanings as described in claim 2.
